# EUROPEAN PATENT SPECIFICATION

(11) **EP 1 180 105 B1**
(45) Date of publication and mention of the grant of the patent: **14.05.2003**
(21) Application number: 00917713.0
(22) Date of filing: 03.03.2000
(51) Int. Cl.: C07D 471/04, A61K 31/437, C07D 513/14, C07D 471/14, A61K 31/4365, A61P 35/00

(54) **SUBSTITUTED AZA-OXINDOLE DERIVATIVES**
SUBSTITUIERTE AZAOXINDOLEDERIVATE
DERIVES D'AZA-OXINDOLE SUBSTITUES

(30) Priority: 04.03.1999 GB 9904995
(43) Date of publication of application: 20.02.2002
(73) Proprietor: GLAXO GROUP LIMITED, Greenford, Middlesex UB6 ONN (GB)
(72) Inventor: HARRIS, Philip, Anthony Glaxo Wellcome Inc., Research Triangle Park, NC 27709 (US); KUYPER, Lee, Frederick Glaxo Wellcome Inc., Research Triangle Park, NC 27709 (US); LACKEY, Karen, Elizabeth Glaxo Wellcome Inc., Research Triangle Park, NC 27709 (US); VEAL, James, Marvin Glaxo Wellcome Inc., Research Triangle Park, NC 27709 (US)
(74) Representative: Dolton, Peter Irving Ernest
(86) International application number: US0005583
(87) International publication number: WO00055159

(56) References cited:
- WO-A-98/50356
- DE-A- 19 816 624

## Description

The present invention provides novel compounds, novel compositions and methods for their use and manufacture. The compounds and compositions of the present invention are generally useful pharmacologically as therapeutic agents in disease states alleviated by the inhibition or antagonism of protein kinase activated signalling pathways in general, and in particular in the pathological processes which involve aberrant cellular proliferation, such disease states including tumor growth, restenosis, atherosclerosis, and thrombosis. In particular, the present invention relates to a series of substituted aza-oxindole compounds, which exhibit protein tyrosine kinase and protein serine/threonine kinase inhibition, and which are useful for the prevention of chemotherapy-induced alopecia.

### BACKGROUND OF THE INVENTION

Protein kinases play a critical role in the control of cell growth and differentiation and are key mediators of cellular signals leading to the production of growth factors and cytokines. See, for example, Schlessinger and Ullrich, *Neuron* **1992,** 9, 383. A partial, non-limiting, list of such kinases includes abl, ARaf, ATK, ATM, bcr-abl, Blk, BRaf, Brk, Btk, CDK1, CDK2, CDK3, CDK4, CDK5, CDK6, CDK7, CDK8, CDK9, c-fms, c-kit, c-met, cRaf1, CSF1R, CSK, c-src, EGFR, ErbB2, ErbB3, ErbB4, ERK, ERK1, ERK2, Fak, fes, FGFR1, FGFR2, FGFR3, FGFR4, FGFR5, Fgr, FLK-4, Fps, Frk, Fyn, GSK, gsk3a, gsk3b, Hck, IGF-1R, IKK, IKK1, IKK2, IKK3, INS-R, Integrin-linked kinase, JAK, JAK1, JAK2, JAK3, JNK, Lck, Lyn, MEK, MEK1, MEK2, p38, PDGFR, PIK, PKB1, PKB2, PKB3, PKC, PKCa, PKCb, PKCd, PKCe, PKCg, PKCI, PKCm, PKCz, PLK1, Polo-like kinase, PYK2, tie₁, tie₂, TrkA, TrkB, TrkC, UL13, UL97, VEGF-R1, VEGF-R2, Yes and Zap70. Protein kinases have been implicated as targets in central nervous system disorders such as Alzheimer's (Mandelkow, E. M. et al. *FEBS Lett.* **1992**, *314*, 315; Sengupta, A. et al. *Mol. Cell. Biochem*. **1997**, *167*,99), pain sensation (Yashpal, K. *J. Neurosci.* **1995**, *15*, 3263-72), inflammatory disorders such as arthritis (Badger, *J. Pharm. Exp. Ther.* **1996**, 279, 1453), psoriasis (Dvir, et al, *J. Cell Biol.* **1991**, *113*, 857), bone diseases such as osteoporosis (Tanaka et al, *Nature,* **1996**, *383,* 528), cancer (Hunter and Pines, *Cell* **1994**, *79*, 573), atherosclerosis (Hajjar and Pomerantz, *FASEB J.* **1992**, *6*, 2933), thrombosis (Salari, *FEBS* **1990**, *263*, 104), metabolic disorders such as diabetes (Borthwick, A.C. et al. *Biochem. Biophys. Res. Commun.* **1995,** *210*, 738), blood vessel proliferative disorders such as angiogenesis (Strawn et al *Cancer Res.* **1996,** *56,* 3540; Jackson et al *J. Pharm. Exp. Ther.* **1998**, *284*, 687), restenosis (Buchdunger et al, *Proc, Nat. Acad. Sci USA* **1991,** *92,* 2258), autoimmune diseases and transplant rejection (Bolen and Brugge, *Ann. Rev. Immunol.* **1997**, *15*, 371) and infectious diseases such as viral (Littler, E.*Nature* **1992,** *358*, 160), and fungal infections (Lum, R. T. PCT Int. Appl., WO 9805335 A1 980212).

The signals mediated by kinases have also been shown to control growth, death and differentiation in the cell by regulating the processes of the cell cycle (Massague and Roberts, Current Opinion in Cell Biology **1995**, 7, 769-72). Progression through the eukaryotic cell cycle is controlled by a family of kinases called cydin dependent kinases (CDKs) (Myerson, et al., EMBO Journal **1992,** *11,* 2909). The coordinate activation and inactivation of different cyclin/CDK complexes is necessary for normal progression through the cell cycle (Pines, Trends in Biochemical Sciences **1993,** *18*, 195; Sherr, Cell **1993**, *73*, 1059). Both the critical G1-S and G2-M transitions are controlled by the activation of different cyclin/CDK activities. In G1, both cyclin D/CDK4 and cyclin E/CDK2 are thought to mediate the onset of S-phase (Matsushime, et al., Molecular & Cellular Biology **1994,** *14*, 2066; Ohtsubo and Roberts, Science **1993,** *259,* 1908; Quelle, et al., Genes & Development **1993,** *7,* 1559; Resnitzky, et al., Molecular & Cellular Biology **1994,** *14*, 1669). Progression through S-phase requires the activity of cyclin A/CDK2 (Girard, et al., Cell **1991,** *67,* 1169; Pagano, et al., EMBO Journal **1992,** *11,* 961; Rosenblatt, et al., Proceedings of the National Academy of Science USA **1992,** *89,* 2824; Walker and Maller, Nature **1991,** *354,* 314; Zindy, et al., Biochemical & Biophysical Research Communications **1992,** *182,* 1144) whereas the activation of cyclin A/*cdc2* (CDK1) and cydin B/*cdc2* are required for the onset of metaphase (Draetta, Trends in Cell Biology **1993,** *3,* 287; Murray and Kirschner, Nature **1989,** 339, 275; Solomon, et al., Molecular Biology of the Cell. **1992,** *3,* 13; Girard, et al., Cell **1991,** 67, 1169; Pagano, et al., EMBO Journal **1992,** *11,* 961; Rosenblatt, et al., Proceedings of the National Academy of Science USA **1992,** *89,* 2824; Walker and Maller, Nature **1991,** *354,* 314; Zindy, et al., Biochemical & Biophysical Research Communications **1992,** *182,* 1144). It is not surprising, therefore, that the loss of control of CDK regulation is a frequent event in hyperproliferative diseases and cancer (Pines, Current Opinion in Cell Biology **1992**, *4*, 144; Lees, Current Opinion in Cell Biology **1995**, *7*, 773; Hunter and Pines, Cell **1994**, 79, 573). The selective inhibition of CDKs is therefore an object of the present invention.

### SUMMARY OF THE INVENTION

In brief summary, the invention comprises compounds of the formula (I): 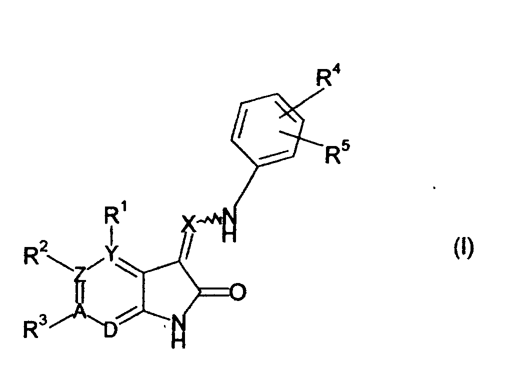 wherein
X is selected from the group consisting of: N, CH, CCF₃, and C(C₁₋₁₂ aliphatic);
Y is C or N, with the proviso that when Y is N, R¹ is absent, and Z, A and D are each C;
Z is C or N, with the proviso that when Z is N, R² is absent, and Y, A and D are each C;
A is C or N, with the proviso that when A is N, R³ is absent, and Y, Z and D are each C;
D is CH or N, with the proviso that when D is N, then Y, Z and A are each C;
with the further proviso that Y, Z, A and D do not all simultaneously represent C; and CH respectively.
R¹ is selected from the group consisting of: hydrogen, C₁₋₁₂ aliphatic, thiol, hydroxy, hydroxy-C₁₋₁₂ aliphatic, Aryl, Aryl-C₁₋₁₂ aliphatic, R⁶-Aryl-C₁₋₁₂ aliphatic, Cyc, Cyc-C₁₋₆ aliphatic, Het, Het-C₁₋₁₂ aliphatic, C₁₋₁₂ alkoxy, Aryloxy, amino, C₁₋₁₂ aliphatic amino, di-C₁₋₁₂ aliphatic amino, di-C₁₋₁₂ aliphatic aminocarbonyl, di-C₁₋₁₂ aliphatic aminosulfonyl, C₁₋₁₂ alkoxycarbonyl, halogen, cyano, sulfonamide and nitro, where
R⁶, Aryl, Cyc and Het are as defined below;
R² is selected from the group consisting of: hydrogen, C₁₋₁₂ aliphatic, N-hydroxyimino-C₁₋₁₂ aliphatic, C₁₋₁₂ alkoxy, hydroxy-C₁₋₁₂ aliphatic, C₁₋₁₂ alkoxycarbonyl, carboxyl C₁₋₁₂ aliphatic, Aryl, R⁶-Aryl-oxycarbonyl, R⁶-oxycarbonyl-Aryl, Het, aminocarbonyl, C₁₋₁₂ aliphatic-aminocarbonyl, Aryl-C₁₋₁₂ aliphatic-aminocarbonyl, R⁶-Aryl-C₁₋₁₂ aliphatic-aminocarbonyl, Het-C₁₋₁₂ aliphatic-aminocarbonyl, hydroxy-C₁₋₁₂ aliphatic-aminocarbonyl, C₁₋₁₂-alkoxy-C₁₋₁₂ aliphatic-aminocarbonyl, C₁₋₁₂ alkoxy-C₁₋₁₂ aliphatic-amino, di-C₁₋₁₂ aliphatic amino, di-C₁₋₁₂ aliphatic aminocarbonyl, di-C₁₋₁₂ aliphatic aminosulfonyl, halogen, hydroxy, nitro, C₁₋₁₂ aliphatic-sulfonyl, aminosulfonyl and C₁₋₁₂ aliphatic-aminosulfonyl, where R⁶ Aryl and Het are as defined below;
R¹ and R² are optionally joined to form a fused ring selected from the group as defined for Het below, and said fused ring is optionally substituted by one or more substituents selected from the group consisting of: C₁₋₁₂ aliphatic, halogen, nitro, cyano, C₁₋₁₂ alkoxy, carbonyl-C₁₋₁₂ alkoxy and oxo;
R³ is selected from the group consisting of: hydrogen, C₁₋₁₂ aliphatic, hydroxy, hydroxy C₁₋₁₂ aliphatic, di-C₁₋₁₂ aliphatic amino, di-C₁₋₁₂ aliphatic aminocarbonyl, di-C₁₋₁₂ aliphatic aminosulfonyl, C₁₋₁₂ alkoxy, Aryl, Aryloxy, hydroxy-Aryl, Het, hydroxy-Het, Het-oxy and halogen, where Aryl and Het are as defined below;
R² arid R³ are optionally joined to form a fused ring selected from the group as defined for Het below, and said fused ring is optionally substituted by C₁₋₆ aliphatic and/or C₁₋₆ aliphatic-carbonyl;
R⁴ is selected from the group consisting of: sulfonic acid, C₁₋₁₂ aliphatic-sulfonyl, sulfonyl-C₁₋₁₂ aliphatic, C₁₋₁₂ aliphatic-sulfonyl-C₁₋₆ aliphatic, C₁₋₆ aliphatic-amino, R⁷-sulfonyl, R⁷-sulfonyl-C₁₋₁₂ aliphatic, R⁷-aminosulfonyl, R⁷-aminosulfonyl-C₁₋₁₂ aliphatic, R⁷-sulfonylamino, R⁷-sulfonylamino-C₁₋₁₂ aliphatic, aminosulfonylamino, di-C₁₋₁₂ aliphatic amino, di-C₁₋₁₂ aliphatic aminocarbonyl, di-C₁₋₁₂ aliphatic aminosulfonyl, di-C₁₋₁₂ aliphatic aminosulfonyl-C₁₋₁₂ aliphatic, (R⁸)₁₋₃-Arylamino, (R⁸)₁₋₃-Arylsulfonyl, (R⁸)₁₋₃-Aryl-aminosulfonyl, (R⁸)₁₋₃-Aryl-sulfonylamino, Het-amino, Het-sulfonyl, Het-aminosulfonyl,
aminoiminoamino and aminoiminoaminosulfonyl, where R⁷, R⁸, Aryl and Het are as defined below;
R⁵ is hydrogen or R⁴ and R⁵ are optionally joined to form a fused ring selected from the group as defined for Het below, and said fused ring is optionally substituted by one or more substituents selected from the group consisting of: C ₁₋₁₂ aliphatic, oxo and dioxo;
R⁶ is selected from the group consisting of: C₁₋₁₂ aliphatic, hydroxy, C₁₋₁₂ alkoxy and halogen;
R⁷ is selected from the group consisting of: hydrogen, C₁₋₁₂ aliphatic, C₁₋₁₂ alkoxy, hydroxy-C₁₋₁₂ alkoxy, hydroxy-C₁₋₁₂ aliphatic, carboxylic acid, C₁₋₁₂ aliphatic-carbonyl, Het, Het-C₁₋₁₂-aliphatic, Het-C₁₋₁₂-alkoxy, di-Het-C₁₋₁₂-alkoxy Aryl, Aryl-C₁₋₁₂-aliphatic, Aryl-C₁₋₁₂-alkoxy, Aryl-carbonyl, C₁₋₁₈ alkoxyalkoxyalkoxyalkoxyaliphatic and hydroxyl, where Het and Aryl are as defined below;
R⁸ is selected from the group consisting of: hydrogen, nitro, cyano, C₁₋₁₂ alkoxy, halo, carbonyl-C₁₋₁₂ alkoxy and halo-C₁₋₁₂aliphatic;
Aryl is selected from the group consisting of: phenyl, naphthyl, phenanthryl and anthracenyl;
Cyc is selected from the group consisting of: cyclopropyl, cyclobutyl, cyclopentyl, cyclohexyl, cycloheptyl and cyclooctyl, and optionally has one or more degrees of unsaturation;
Het is a saturated or unsaturated heteroatom ring system selected from the group consisting of: benzimidazole, dihydrothiophene, dioxin, dioxane, dioxolane, dithiane, dithiazine, dithiazole, dithiolane, furan, imidazole, isoquinoline, morpholine, oxazole, oxadiazole, oxathiazole, oxathiazolidine, oxazine, oxadiazine, piperazine, piperadine, pyran, pyrazine, pyrazole, pyridine, pyrimidine, pyrrole, pyrrolidine, quinoline, tetrahydrofuran, tetrazine, thidiazine, thiadiazole, thiatriazole, thiazine, thiazole, thiomorpholine, thiophene, thiopyran, triazine and triazole;
and the salts and solvates thereof, in either crystalline or amorphous form.

The esters, amides and and carbamates are preferably hydrolyzable and are more preferably biohydrolyzable. The salts are preferably pharmaceutically acceptable salts.

A more preferred genus of compounds of the present invention includes compounds of formula (I), defined as follows: 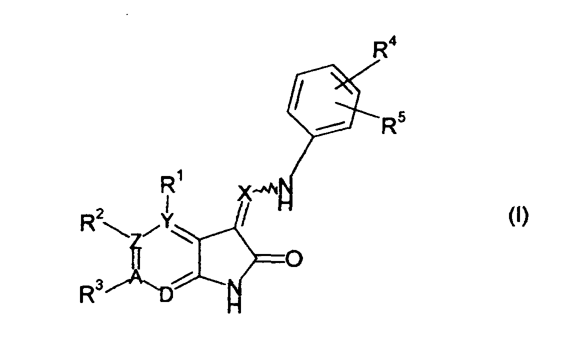 wherein
X is selected from the group consisting of: N, CH and C(C₁₋₆ aliphatic);
Y is C or N, with the proviso that when Y is N, R¹ is absent, and Z, A and D are each C;
Z is C or N, with the proviso that when Z is N, R² is absent, and Y, A and D are each C;
A is C or N, with the proviso that when A is N, R³ is absent, and Y, Z and D are each C;
D is C or N, with the proviso that when D is N, then Y, Z and A are C;
with the further proviso that Y, Z, A and D do not simultaneously all represent C;
R¹ is selected from the group consisting of: hydrogen, C₁₋₆ aliphatic, hydroxy-C₁₋₆ aliphatic, Aryl-C₁₋₆ aliphatic, R⁶-Aryl-C₁₋₆ aliphatic, Cyc-C₁₋₆ aliphatic, Het-C₁₋₆ aliphatic, C₁₋₆alkoxy, Aryloxy, aminocarbonyl, di-C₁₋₆ aliphatic amino, di-C₁₋₆ aliphatic aminocarbonyl, di-C₁₋₆ aliphatic aminosulfonyl, C₁₋₆ alkoxycarbonyl, halogen and nitro, where R⁶, Aryl, Cyc and Het are as defined below;
R² is selected from the group consisting of: hydrogen, C₁₋₆ aliphatic, R⁷-C₁₋₆ aliphatic, C₁₋₆alkoxy, hydroxy-C₁₋₆ aliphatic, C₁₋₆ alkoxycarbonyl, carboxyl C₁₋₆ aliphatic, Aryl, R⁶-Aryl-oxycarbonyl, R⁶-oxycarbonyl-Aryl, Het, aminocarbonyl, C₁₋₆ aliphatic-aminocarbonyl, Aryl-C₁₋₆ aliphatic-aminocarbonyl, R⁶-Aryl-C₁₋₆ aliphatic-aminocarbonyl, Het-C₁₋₆ aliphatic-aminocarbonyl, hydroxy-C₁₋₆ aliphatic-aminocarbonyl, C₁₋₆alkoxy-C₁₋₆ aliphatic-aminocarbonyl, C₁₋₆ alkoxy-C₁₋₆ aliphatic-amino, di-C₁₋₆ aliphatic amino, di-C₁₋₆ aliphatic aminocarbonyl, di-C₁₋₆ aliphatic aminosulfonyl, halogen, hydroxy, nitro, sulfo, C₁₋₆ aliphatic-sulfonyl, aminosulfonyl, C₁₋₆ aliphatic-aminosulfonyl and quatemary ammonium, where R⁶, R⁷, Aryl and Het are as defined below;
R¹ and R² are optionally joined to form a fused ring selected from the group as defined for Het below, and said fused ring is optionally substituted by halogen and/or oxo;
R³ is selected from the group consisting of: hydrogen, C₁₋₆ aliphatic, hydroxy, hydroxy C₁₋₆ aliphatic, di-C₁₋₆ aliphatic amino, di-C₁₋₆ aliphatic aminocarbonyl, di-C₁₋₆ aliphatic aminosulfonyl, C₁₋₆ alkoxy, Aryl, Aryloxy, hydroxy-Aryl, Het, hydroxy-Het, Het-oxy and halogen, where Aryl and Het are as defined below;
R² and R³ are optionally joined to form a fused ring selected from the group as defined for Het below, and said fused ring is optionally substituted by C₁₋₆ aliphatic or C₁₋₆ aliphatic-carbonyl;
R⁴ is selected from the group consisting of: sulfonic acid, C₁₋₁₂ aliphatic-sulfonyl, sulfonyl-C₁₋₁₂ aliphatic, C₁₋₁₂ aliphatic-sulfonyl-C₁₋₆ aliphatic, C₁₋₆ aliphatic-amino, R⁷-sulfonyl, R⁷-sulfonyl-C₁₋₁₂ aliphatic, R⁷-aminosulfonyl, R⁷-aminosulfonyl-C₁₋₁₂ aliphatic, R⁷-sulfonylamino, R⁷-sulfonylamino-C₁₋₁₂ aliphatic, aminosulfonylamino, di-C₁₋₁₂ aliphatic amino, di-C₁₋₁₂ aliphatic aminocarbonyl, di-C₁₋₁₂ aliphatic aminosulfonyl, di-C₁₋₁₂ aliphatic aminosulfonyl-C₁₋₁₂ aliphatic, (R⁸)₁₋₃-Arylamino, (R⁸)₁₋₃-Arylsulfonyl, (R⁸)₁₋₃-Aryl-aminosulfonyl, (R⁸)₁₋₃-Aryl-sulfonylamino, Het-amino, Het-sulfonyl, Het-aminosulfonyl, aminoiminoamino and aminoiminoaminosulfonyl, where R⁷, R⁸, Aryl and Het are as defined below;
R⁵ is hydrogen;
R⁴ and R⁵ are optionally joined to form a fused ring selected from the group as defined for Het below, and said fused ring is optionally substituted by oxo or dioxo;
R⁶ is selected from the group consisting of: hydrogen, C₁₋₆ aliphatic, hydroxy, C₁₋₆ alkoxy and halogen;
R⁷ is selected from the group consisting of: hydrogen, C₁₋₁₂ aliphatic, C₁₋₁₂ alkoxy, hydroxy-C₁₋₁₂ alkoxy, hydroxy-C₁₋₁₂ aliphatic, carboxylic acid, C₁₋₁₂ aliphatic-carbonyl, Het, Het-C₁₋₁₂-aliphatic, Het-C₁₋₁₂-alkoxy, di-Het-C₁₋₁₂-alkoxy Aryl, Aryl-C₁₋₁₂-aliphatic, Aryl-C₁₋₁₂-alkoxy, Aryl-carbonyl, C₁₋₁₈ alkoxyalkoxyalkoxyalkoxyaliphatic and hydroxyl, where Het and Aryl are as defined below;
R⁸ is hydrogen and/or halo-C₁₋₆ aliphatic;
Aryl is phenyl or naphthyl;
Cyc is selected from the group consisting of: cyclopropyl, cyclobutyl, cyclopentyl, cyclohexyl, cycloheptyl and cyclooctyl, and optionally has one or more degrees of unsaturation;
Het is a saturated or unsaturated heteroatom ring system selected from the group consisting of: benzimidazole, dihydrothiophene, dioxin, dioxane, dioxolane, dithiane, dithiazine, dithiazole, dithiolane, furan, imidazole, morpholine, oxazole, oxadiazole, oxathiazole, oxathiazolidine, oxazine, oxadiazine, piperazine, piperadine, pyran, pyrazine, pyrazole, pyridine, pyrimidine, pyrrole, pyrrolidine, tetrahydrofuran, tetrazine, thiadiazine, thiadiazole, thiatriazole, thiazine, thiazole, thiomorpholine, thiophene, thiopyran, triazine and triazole; and
the salts, esters, amides, carbamates, solvates, polymorphs, hydrates, affinity reagents and/or prodrugs thereof, in either crystalline or amorphous form. The esters, amides and carbamates are preferably hydrolyzable and are more preferably biohydryzeable. The salts are preferably pharmaceutically acceptable salts.

A highly preferred genus of compounds of the present invention indudes compounds of formula (I), defined as follows: 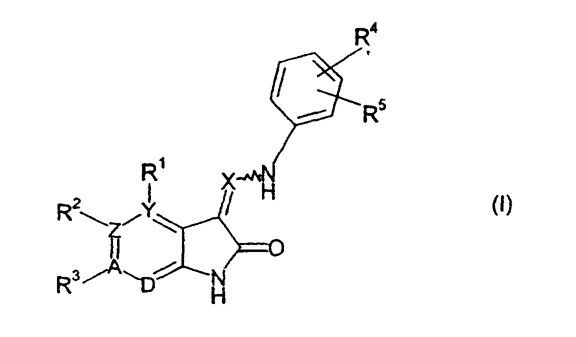 wherein X is selected from the group consisting of: N, CH and CCH_{3;}
Y is C or N, with the proviso that when Y is N, R¹ is absent, and Z, A and D are each C;
Z is C or N, with the proviso that when Z is N, R² is absent, and Y, A and D are each C;
A is C or N, with the proviso that when A is N, R³ is absent, and X, Y and D are each C;
D is C or N, with the proviso that when D is N, then Y, Z and A are each C; with the further proviso that Y, Z, A and D do not simultaneously all represent C;
R¹ is selected from the group consisting of: hydrogen, C₁₋₆ aliphatic, hydroxy-C₁₋₆ aliphatic, di-C₁₋₆ aliphatic amino, di-C₁₋₆ aliphatic aminocarbonyl, di-C₁₋₆ aliphatic aminosulfonyl, Aryl-C₁₋₆ aliphatic, R⁶-Aryl-C₁₋₆ aliphatic, Cyc-C₁₋₆ aliphatic, Het-C₁₋₆ aliphatic, C₁₋₆ alkoxy, Aryloxy, aminocarbonyl, C₁₋₆ alkoxycarbonyl, halogen and nitro, where R⁶, Aryl, Cyc and Het are as defined below;
R² is selected from the group consisting of: hydrogen, C₁₋₆ aliphatic, N-hydroxyimino-C₁₋₆ aliphatic, C₁₋₆alkoxy, C₁₋₆ alkoxycarbonyl, Aryl, R⁶-Aryloxycarbonyl, Het, aminocarbonyl, C₁₋₆ aliphatic aminocarbonyl, Aryl-C₁₋₆ aliphatic aminocarbonyl, R⁶-Aryl-C₁₋₆ aliphatic aminocarbonyl, Het-C₁₋₆ aliphatic aminocarbonyl, di-C₁₋₆ aliphatic amino, di-C₁₋₆ aliphatic aminocarbonyl, di-C₁₋₆ aliphatic aminosulfonyl, hydroxy-C₁₋₆ aliphatic aminocarbonyl, C₁₋₆-alkoxy-C₁₋₆ aliphatic aminocarbonyl, C₁₋₆ alkoxy-C₁₋₆ aliphatic amino, halogen, hydroxy, nitro, C₁₋₆ aliphatic sulfonyl, aminosulfonyl and C₁₋₆ aliphatic aminosulfonyl, where R⁶, Aryl and Het are as defined below;
R¹ and R² are optionally joined to form a fused ring selected from the group as defined for Het below, and said fused ring is optionally substituted by halogen and/or oxo;
R³ is selected from the group consisting of: hydrogen, C₁₋₆ aliphatic, hydroxy, hydroxy C₁₋₆ aliphatic, di-C₁₋₆ aliphatic amino, di-C₁₋₆ aliphatic aminocarbonyl, di-C₁₋₆ aliphatic aminosulfonyl C₁₋₆ alkoxy, Aryloxy, Het and halogen, where Aryl and Het are as defined below;
R² and R³ are optionally joined to form a fused ring selected from the group as defined for Het below, and said fused ring is optionally substituted by C₁₋₆ alkyl and/or C₁₋₆ alkylcarbonyl;
R⁴ is selected from the group consisting of: R⁷-sulfonyl, R⁷-sulfonyl C₁₋₆-aliphatic, C₁₋₆ aliphatic sulfonyl-C₁₋₆ aliphatic, R⁷-aminosulfonyl, di-C₁₋₆ aliphatic amino, di-C₁₋₆ aliphatic aminocarbonyl, di-C₁₋₆ aliphatic aminosulfonyl, di-C₁₋₆ aliphatic aminosulfonyl-C₁₋₆ aliphatic, R⁷-aminosulfonyl C₁₋₆ aliphatic, aminosulfonylamino, R⁷-C₁₋₆ aliphatic aminosulfonyl-C₁₋₆ aliphatic, Aryl, Het, R⁸-Aryl-aminosulfonyl, Het-aminosulfonyl and aminoiminoaminosulfonyl, where R⁷, R⁸, Aryl and Het are as defined below;
R⁵ is hydrogen;
R⁴ and R⁵ are optionally joined to form a fused ring selected from the group as defined for Het below, and said used ring is optionally substituted by oxo or dioxo;
R⁶ is selected from the group consisting of: hydroxy, C₁₋₆ alkoxy and halogen;
R⁷ is selected from the group consisting of: hydrogen, C₁₋₆ aliphatic, hydroxy C₁₋₆-alkoxy, hydroxy-C₁₋₆ aliphatic, C₁₋₆ aliphatic carbonyl, Aryl-carbonyl, C₁₋₁₂ alkoxyalkoxyalkoxyalkoxyalkyl, hydroxyl, Aryl, Aryl-C₁₋₆-alkoxy, Aryl-C₁₋₆-aliphatic, Het, Het-C₁₋₆-alkoxy, di-Het-C₁₋₆-alkoxy, Het-C₁₋₆-aliphatic and di-Het-C₁₋₆-aliphatic;
R⁸ is trifluoromethyl;
Aryl is phenyl;
Cyc is cyclobutyl;
Het is a saturated or unsaturated heteroatom ring system selected from the group consisting of: benzimidazole, dihydrothiophene, dioxolane, furan, imidazole, morpholine, oxazole, pyridine, pyrrole, pyrrolidine, thiadiazole, thiazole, thiophene, and triazole;
and the salts, esters, amides, carbamates, solvates, polymorphs, hydrates, affinity reagents and/or prodrugs thereof, in either crystalline or amorphous form. The esters, amides and carbamates are preferably hydrolyzable and are more preferably biohydrolyzable. The salts are preferably pharmaceutically acceptable salts.

A preferred group of compounds of the present invention with respect to the substitutions at R⁴ are compounds of formula (I): 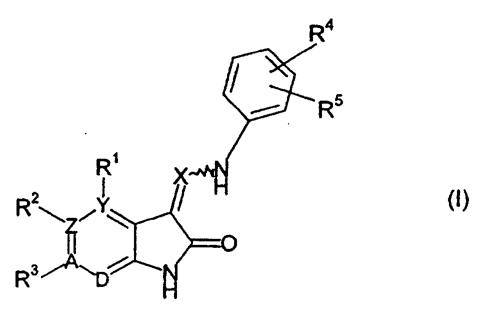 wherein X is N or CH;
Y is C or N, with the proviso that when Y is N, R¹ is absent, and Z, A and D are each C;
Z is C or N, with the proviso that when Z is N, R² is absent, and Y, A and D are each C;
A is C or N, with the proviso that when A is N, R³ is absent, and Y, Z and D are each C;
D is C or N, with the proviso that when D is N, Y, Z and A are each C;
with the further proviso that Y, Z, A and D do not simultaneously all represent C;
R¹ is selected from the group consisting of: hydrogen, C₁₋₁₂ aliphatic, thiol, hydroxy, hydroxy-C₁₋₁₂ aliphatic, Aryl, Aryl-C₁₋₁₂ aliphatic, R⁶-Aryl-C₁₋₁₂ aliphatic, Cyc, Cyc-C₁₋₆ aliphatic, Het, Het-C₁₋₁₂ aliphatic, C₁₋₁₂ alkoxyl, Aryloxy, amino, C₁₋₁₂ aliphatic amino, di-C₁₋₁₂ aliphatic amino, di-C₁₋₁₂aliphatic aminocarbonyl, di-C₁₋₁₂ aliphatic aminosulfonyl, C₁₋₁₂ alkoxycarbonyl, halogen, cyano, sulfonamide and nitro, where R⁶, Aryl, Cyc and Het are as defined below;
R² is selected from the group consisting of: hydrogen, C₁₋₁₂ aliphatic, N-hydroxyimino-C₁₋₁₂ aliphatic, C₁₋₁₂ alkoxy, hydroxy-C₁₋₁₂ aliphatic, C₁₋₁₂ alkoxycarbonyl, carboxyl C₁₋₁₂ aliphatic, Aryl, R⁶-Aryl-oxycarbonyl, R⁶-oxycarbonyl-Aryl, Het, aminocarbonyl, C₁₋₁₂ aliphatic-aminocarbonyl, Aryl-C₁₋₁₂ aliphatic-aminocarbonyl, R⁶-Aryl-C₁₋₁₂ aliphatic-aminocarbonyl, Het-C₁₋₁₂ aliphatic-aminocarbonyl, hydroxy-C₁₋₁₂ aliphatic-aminocarbonyl, C₁₋₁₂-alkoxy-C₁₋₁₂ aliphatic-aminocarbonyl, C₁₋₁₂ alkoxy-C₁₋₁₂ aliphatic-amino, di-C₁₋₁₂ aliphatic amino, di-C₁₋₁₂ aliphatic aminocarbonyl, di-C₁₋₁₂ aliphatic aminosulfonyl, halogen, hydroxy, nitro, C₁₋₁₂ aliphatic-sulfonyl, aminosulfonyl and C₁₋₁₂ aliphatic-aminosulfonyl, where R⁶, Aryl and Het are as defined below;
R¹ and R² are optionally joined to form a fused ring selected from the group as defined for Het below, and said fused ring is optionally substituted by one or more substituents selected from the group consisting of: halogen, nitro, cyano, C₁₋₁₂ alkoxy, carbonyl-C₁₋₁₂ alkoxy and oxo;
R³ is selected from the group consisting of: hydrogen, C₁₋₁₂ aliphatic, hydroxy, hydroxy C₁₋₁₂ aliphatic, di-C₁₋₁₂ aliphatic amino, di-C₁₋₁₂ aliphatic aminocarbonyl, di-C₁₋₁₂ aliphatic aminosulfonyl, C₁₋₁₂ alkoxy, Aryl, Aryloxy, hydroxy-Aryl, Het, hydroxy-Het, Het-oxy, or halogen, where Aryl and Het are as defined below;
R² and R³ are optionally joined to form a fused ring selected from the group as defined for Het below, and said fused ring is optionally substituted by C₁₋₆ aliphatic and/or C₁₋₆ aliphatic-carbonyl;
R⁴ is selected from the group consisting of: R⁷-aminosulfonyl, R⁷-aminosulfonyl-C₁₋₁₂ aliphatic, R⁷-sulfonylamino, R⁷-sulfonylamino-C₁₋₁₂ aliphatic, aminosulfonylamino, di-C₁₋₁₂ aliphatic aminosulfonyl, di-C₁₋₁₂ aliphatic aminosulfonyl-C₁₋₁₂ aliphatic, (R⁸)₁₋₃-Aryl-aminosulfonyl, (R⁸)₁₋₃-Arylsulfonylamino and aminoiminoaminosulfonyl, where R⁷, R⁸, Aryl and Het are as defined below;
R⁵ is hydrogen;
R⁴ and R⁵ are optionally joined to form a fused ring selected from the group as defined for Het below, and said fused ring is optionally substituted by oxo or dioxo;
R⁶ is selected from the group consisting of: C₁₋₁₂ aliphatic, hydroxy, C₁₋₁₂ alkoxy and halogen;
R⁷ is selected from the group consisting of: hydrogen, C₁₋₁₂ aliphatic, C₁₋₁₂ alkoxy, hydroxy-C₁₋₁₂ alkoxy, hydroxy-C₁₋₁₂ aliphatic, carboxylic acid, C₁₋₁₂ aliphatic-carbonyl, Het, Het-C₁₋₁₂-aliphatic, Het-C₁₋₁₂-alkoxy, di-Het-C₁₋₁₂-alkoxy Aryl, Aryl-C₁₋₁₂-aliphatic, Aryl-C₁₋₁₂-alkoxy, Aryl-carbonyl, C₁₋₁₈ alkoxyalkoxyalkoxyalkoxyaliphatic and hydroxyl, where Het and Aryl are as defined below;
R⁸ is selected from the group consisting of: hydrogen, nitro, cyano, C₁₋₁₂ alkoxy, halo, carbonyl-C₁₋₁₂ alkoxy and halo-C₁₋₁₂ aliphatic; and
Aryl is selected from the group consisting of: phenyl, naphthyl, phenanthryl and anthracenyl;
Cyc is selected from the group consisting of: cyclopropyl, cyclobutyl, cyclopentyl, cyclohexyl, cycloheptyl and cyclooctyl, and optionally has one or more degrees of unsaturation;
Het is a saturated or unsaturated heteroatom ring system selected from the group consisting of: benzimidazole, dihydrothiophene, dioxin, dioxane, dioxolane, dithiane, dithiazine, dithiazole, dithiolane, furan, imidazole, morpholine, oxazole, oxadiazole, oxathiazole, oxathiazolidine, oxazine, oxadiazine, piperazine, piperadine, pyran, pyrazine, pyrazole, pyridine, pyrimidine, pyrrole, pyrrolidine, tetrahydrofuran, tetrazine, thiadiazine, thiadiazole, thiatriazole, thiazine, thiazole, thiomorpholine, thiophene, thiopyran, triazine and triazole;
and the salts, esters, amides, carbamates, solvates, polymorphs, hydrates, affinity reagents and/or prodrugs thereof, in either crystalline or amorphous form. The esters, amides and carbamates are preferably hydrolyzable and are more preferably biohydrolyzeable. The salts are preferably pharmacetically acceptable salts.

A preferred group of compounds of the present invention with respect to the substitutions at R⁴ are compounds of formula (I): 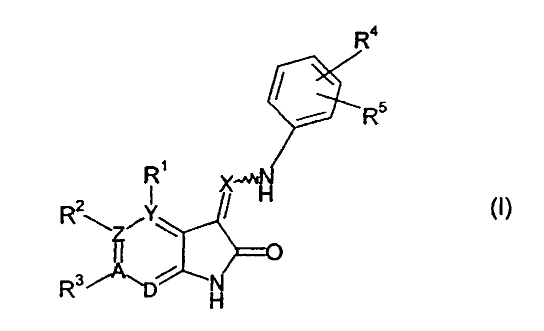 wherein X is CH;
Y is C or N, with the proviso that when Y is N, R¹ is absent, and Z, A and D are each C;
Z is C or N, with the proviso that when Z is N, R² is absent, and Y, A and D are each C;
A is C or N, with the proviso that when A is N, R³ is absent, and Y, Z and D are each C;
D is C or N, with the proviso that when D is N, Y, Z and A are each C;
R¹ is hydrogen;
R² is selected from the group consisting of: hydrogen, C₁₋₁₂ alkoxycarbonyl, Aryl, Het and halogen, where Aryl and Het are as defined below;
R³ is hydrogen or halogen;
R⁴ is selected from the group consisting of: R⁷-aminosulfonyl, R⁷-aminosulfonyl-C₁₋₁₂ aliphatic, R⁷-sulfonylamino, R⁷-sulfonylamino-C₁₋₁₂ aliphatic, aminosulfonylamino, di-C₁₋₁₂ aliphatic aminosulfonyl, di-C₁₋₁₂ aliphatic aminosulfonyl-C₁₋₁₂ aliphatic, (R⁸)₁₋₃-Aryl-aminosulfonyl, (R⁸)₁₋₃-Aryl-sulfonylamino and aminoiminoaminosulfonyl, where
R⁷, R⁸, Aryl and Het are as defined below;
R⁵ is hydrogen;
R⁴ and R⁵ are optionally joined to form a fused ring selected from the group as defined for Het below, and said fused ring is optionally substituted by oxo or dioxo;
R⁷ is selected from the group consisting of: hydrogen, C₁₋₁₂ aliphatic, C₁₋₁₂ alkoxy, hydroxy-C₁₋₁₂ alkoxy, hydroxy-C₁₋₁₂ aliphatic, carboxylic acid, C₁₋₁₂ aliphatic-carbonyl, Het, Het-C₁₋₁₂-aliphatic, Het-C₁₋₁₂-alkoxy, di-Het-C₁₋₁₂-alkoxy Aryl, Aryl-C₁₋₁₂-aliphatic, Aryl-C₁₋₁₂-alkoxy, Aryl-carbonyl, C₁₋₁₈ alkoxyalkoxyalkoxyalkoxyaliphatic and hydroxyl, where Het and Aryl are as defined below;
R⁸ is selected from the group consisting of: hydrogen, nitro, cyano, C₁₋₁₂ alkoxy, halo, carbonyl-C₁₋₁₂ alkoxy and halo-C₁₋₁₂ aliphatic;
Aryl is selected from the group consisting of: phenyl, naphthyl, phenanthryl and anthracenyl;
Cyc is selected from the group consisting of: cyclopropyl, cyclobutyl, cyclopentyl, cyclohexyl, cycloheptyl or cyclooctyl, and optionally has one or more degrees of unsaturation;
Het is a saturated or unsaturated heteroatom ring system selected from the group consisting of: benzimidazole, dihydrothiophene, dioxin, dioxane, dioxolane, dithiane, dithiazine, dithiazole, dithiolane, furan, imidazole, morpholine, oxazole, oxadiazole, oxathiazole, oxathiazolidine, oxazine, oxadiazine, piperazine, piperadine, pyran, pyrazine, pyrazole, pyridine, pyrimidine, pyrrole, pyrrolidine, tetrahydrofuran, tetrazine, thiadiazine, thiadiazole, thiatriazole, thiazine, thiazole, thiomorpholine, thiophene, thiopyran, triazine and triazole;
and the salts, esters, amides, carbamates solvates, polymorphs, hydrates, affinity reagents and/or prodrugs thereof, in either crystalline or amorphous form. The esters, amides and carbamates are preferably hydrolyzable and are more preferably biohydrolyzable. The salts are preferably pharmaceutically acceptable salts.

Due to the presence of an oxindole exocyclic double bond, also included in the compounds of the invention are their respective pure E and Z geometric isomers as well as mixtures of E and Z isomers. The invention as described and claimed does not set any limiting ratios on prevalence of Z to E isomers. Thus, for example, compound number 1 in the tables below is disclosed and claimed as the E geometric isomer thereof, the Z geometric isomer thereof, and a mixture of the E and Z geometric isomers thereof, but not limited by any given ratio(s).

Likewise, it is understood that compounds of formula (I) may exist in tautomeric forms other than that shown in the formula.

Certain of the compounds as described will contain one or more chiral, or asymmetric, centers and will therefore be capable of existing as optical isomers that are either dextrorotatory or levorotatory. Also included in the compounds of the invention are the respective dextrorotatory or levorotatory pure preparations, and mixtures thereof.

Certain compounds of formula (I) above may exist in stereoisomeric forms (e.g. they may contain one or more asymmetric carbon atoms or may exhibit cis-trans isomerism). The individual stereoisomers (enantiomers and diastereoisomers) and mixtures of these are included within the scope of the present invention. Likewise, it is understood that compounds of formula (I) may exist in tautomeric forms other than that shown in the formula, and these are also included within the scope of the present invention.

The present invention also provides compounds of formula (I) and pharmaceutically acceptable salts thereof (hereafter identified as the "active compounds") for use in medical therapy, and particularly in the treatment of disorders mediated by CDK2 activity, such as alopecia induced by cancer chemotherapy.

A further aspect of the invention provides a method of treatment of a human or animal body suffering from a disorder kinase is a mitogen activated protein kinase which comprises administering an effective amount of an active compound of formula (I) to the human or animal patient.

Another aspect of the present invention provides the use of an active compound of formula (I), in the preparation of a medicament for the treatment of malignant tumors, or for the treatment of alopecia induced by cancer chemotherapy or induced by radiation therapy. Alternatively, compounds of formula (I) can be used in the preparation of a medicament for the treatment of a disease mediated by a kinase selected from the group consisting of: abl, ARaf, ATK, ATM, bcr-abl, Blk, BRaf, Brk, Btk, CDK1, CDK2, CDK3, CDK4, CDK5, CDK6, CDK7, CDK8, CDK9, c-fms, c-kit, c-met, cRaf1, CSF1R, CSK, c-src, EGFR, ErbB2, ErbB3, ErbB4, ERK, ERK1, ERK2, Fak, fes, FGFR1, FGFR2, FGFR3, FGFR4, FGFR5, Fgr, FLK-4, Fps, Frk, Fyn, GSK, gsk3a, gsk3b, Hck, IGF-1R, IKK, IKK1, IKK2, IKK3, INS-R, Integrin-linked kinase, JAK, JAK1, JAK2, JAK3, JNK, Lck, Lyn, MEK, MEK1, MEK2, p38, PDGFR, PIK, PKB1, PKB2, PKB3, PKC, PKCa, PKCb, PKCd, PKCe, PKCg, PKCI, PKCm, PKCz, PLK1, Polo-like kinase, PYK2, tie₁, tie₂, TrkA, TrkB, TrkC, UL13, UL97, VEGF-R1, VEGF-R2, Yes and Zap70. Additionally, compounds of formula (I) can be used in the preparation of a medicament for the treatment of a disease or disorder such as organ transplant rejection, tumor growth, chemotherapy-induced alopecia, chemotherapy-induced thrombocytopenia, chemotherapy-induced leukopenia, mucocitis, plantar-palmar syndrome, restenosis, atherosclerosis, rheumatoid arthritis, angiogenesis, hepatic cirrhosis, glomerulonephritis, diabetic nephropathy, malignant nephrosclerosis, thrombotic microangiopathy, glomerulopathy, psoriasis, diabetes mellitus, inflammation, neurodegenerative disease, macular degeneration, actinic keratosis and hyperprotiferative disorders.

Another aspect of the present invention provides the use of an active compound of formula (I), in coadministration with previously known anti-tumor therapies for more effective treatment of such tumors.

Another aspect of the present invention provides the use of an active compound of formula (I) in the preparation of a medicament for the treatment of viral or eukaryotic infection.

Other aspects of the present invention related to the inhibition of mitogen activated protein kinases are discussed in more detail below.

Compounds we have synthesized as part of the present invention which are currently preferred are listed in Tables 1 and 2 below. Compounds are identified by the numbers shown in the first column; variables below in the rest of the columns are with reference to the generic structure (I). Corresponding IUPAC nomenclature are disclosed in Table 2. Since all substituents at each point of substitution are capable of independednt synthesis of each other, the tables are to be read as a matrix in which any combination of substituents is within the scope of the disclosure and claims of the invention.

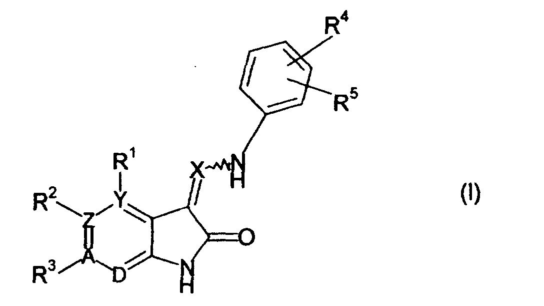

| **Example** | **Y** | **Z** | **A** | **D** | **R**^{**1**} | **R**^{**2**} | **R**^{**3**} | **R**^{**4**} | **R**^{**5**} | **X** |
|---|---|---|---|---|---|---|---|---|---|---|
| 1 | C | N | C | C | H | - | H | 4'-SO₂NH₂ | H | CH |
| 2 | C | C | N | C | H | H | - | 4'-SO₂NH₂ | H | CH |
| 3 | N | C | C | C | - | H | H | 4'-CH=NN(H)-5' | | CH |
| 4 | N | C | C | C | - | H | H | 4'-N=CH-CH=CH-5' | | CH |
| 5 | C | C | C | N | H | H | H | 4'-SO₂NH₂ | H | CH |
| 6 | C | C | C | N | H | H | H | 4'-CH=NN(H)-5' | | CH |
| 7 | C | C | C | N | H | H | H | 4'-N=CH-CH=CH-5' | | CH |
| 8 | C | C | C | N | H | phenyl | H | 4'-SO₂NH₂ | H | CH |
| 9 | C | C | C | N | H | phenyl | H | 4'-CH=NN(H)-5' | | CH |
| 10 | C | C | C | N | H | phenyl | H | 4'-N=CH-CH=CH-5' | | CH |
| 11 | C | C | C | N | H | 2-furanyl | H | 4'-SO₂NH₂ | H | CH |
| 12 | C | C | C | N | H | 2-furanyl | H | 4'-CH=NN(H)-5' | | CH |
| 13 | C | C | C | N | H | 2-furanyl | H | 4'-N=CH-CH=CH-5' | | CH |
| 14 | C | C | C | N | H | 3-thiophenyl | H | 4'-SO₂NH₂ | H | CH |
| 15 | C | C | C | N | H | 3-thiophenyl | H | 4'-CH=NN(H)-5' | | CH |
| 16 | C | C | C | N | H | 3-thiophenyl | H | 4'-N=CH-CH=CH-5' | | CH |
| 17 | C | C | C | N | H | Br | H | 4'-SO₂NH₂ | H | CH |
| 18 | C | C | C | N | H | Br | H | 4'-CH=NN(H)-5' | | CH |
| 19 | C | C | C | N | H | Br | H | 4'-N=CH-CH=CH-5' | | CH |
| 20 | C | C | C | N | H | H | Cl | 4'-SO₂NH₂ | H | CH |
| 21 | C | C | C | N | H | H | Cl | 4'-CH=NN(H)-5' | | CH |
| 22 | C | C | C | N | H | H | Cl | 4'-N=CH-CH=CH-5' | | CH |
| 23 | C | C | C | N | H | carbethoxy | H | 4'-SO₂NH₂ | H | CH |
| 24 | C | C | C | N | H | carbethoxy | H | 4'-CH=NN(H)-5' | | CH |
| 25 | C | C | C | N | H | carbethoxy | H | 4'-N=CH-CH=CH-5' | | CH |

Standard accepted nomenclature corresponding to the Examples set forth in this specification are set forth below. In some cases nomenclature is given for one or more possible isomers.

**Table 2**

| **No.** | **IUPAC NAME** |
|---|---|
| **1** | 4-{[(2-Oxo-1,2-dihydro-3H-pyrrolo[3,2-c]pyridin-3-ylidene)methyl]amino} benzenesulfonamide |
| **2** | 4-{[(2-Oxo-1,2-dihydro-3H-pyrrolo[2,3-c]pyridin-3-ylidene)methyl]amino} benzenesulfonamide |
| **3** | 3-[(1H-Indazol-6-ylamino)methylidene]-1H-pyrrolo[3,2-b]pyridin-2-one |
| **4** | 3-[(6-Quinolinylamino)methylidene]-1,3-dihydro-2H-pyrrolo[3,2-b]pyridin-2-one |
| **5** | 4-{[(2-Oxo-1,2-dihydro-3H-pyrrolo[2,3-b]pyridin-3-ylidene)methyl]amino} benzenesulfonamide |
| **6** | 3-[(1 H-Indazol-6-ylamino)methylidene]-1H-pyrrolo[2,3-b]pyridin-2-one |
| **7** | 3-[(6-Quinolinylamino)methylidene]-1,3-dihydro-2H-pyrrolo[2,3-b]pyridin-2-one |
| **8** | 4-{[(2-Oxo-5-phenyl-1,2-dihydro-3H-pyrrolo[2,3-b]pyridin-3-ylidene)methyl] amino}benzenesulfonamide |
| **9** | 3-[(1H-Indazol-6-ylamino)methylidene]-5-phenyl-1H-pyrrolo[2,3-b]pyridin-2-one |
| **10** | 5-Phenyl-3-[(6-quinolinylamino)methylidene]-1,3-dihydro-2H-pyrrolo[2,3-b]pyridin-2-one |
| **11** | 4-({[5-(2-Furyl)-2-oxo-1,2-dihydro-3H-pyrrolo[2,3-b]pyridin-3-ylidene]methyl} amino)benzenesuffonamide |
| **12** | 5-(2-Furyl)-3-[(1H-indazol-6-ylamino)methylidene]-1H-pyrrolo[2,3-b]pyridin-2-one |
| **13** | 5-(2-Furyl)-3-[(6-quinolinylamino)methylidene]-1,3-dihydro-2H-pyrrolo[2,3-b]pyridin-2-one |
| **14** | 4-({[2-Oxo-5-(3-thienyl)-1,2-dihydro-3H-pyrrolo[2,3-b]pyridin-3-ylidene]methyl} amino)benzenesulfonamide |
| **15** | 3-[(1H-Indazol-6-ylamino)methylidene]-5-(3-thienyl)-1,3-dihydro-2H-pyrrolo[2,3-b]pyridin-2-one |
| **16** | 3-[(6-Quinolinylamino)methylidene]-5-(3-thienyl)-1,3-dihydro-2H-pyrrolo[2,3-b]pyridin-2-one |
| **17** | 4-{[(5-Bromo-2-oxo-1,2-dihydro-3H-pyrrolo[2,3-b]pyridin-3-ylidene)methyl] amino}benzenesulfonamide |
| **18** | 5-Bromo-3-[(1H-indazol-6-ylamino)methylidene]-1,3-dihydro-2H-pyrrolo[2,3-b]pyridin-2-one |
| **19** | 5-Bromo-3-[(6-quinolinylamino)methylidene]-1,3-dihydro-2H-pyrrolo[2,3-b]pyridin-2-one |
| **20** | 4-{[(6-Chloro-2-oxo-1,2-dihydro-3H-pyrrolo[2,3-b]pyridin-3-ylidene)methyl] amino}benzenesulfonamide |
| **21** | 6-Chloro-3-[(1H-indazol-6-ylamino)methylidene]-1,3-dihydro-2H-pyrrolo[2,3-b]pyridin-2-one |
| **22** | 6-Chloro-3-[(6-quinolinylamino)methylidene]-1,3-dihydro-2H-pyrrolo[2,3-b]pyridin-2-one |
| **23** | Ethyl 3-{[4-(aminosulfonyl)anilino]methylidene}-2-oxo-1,2-dihydro-3H-pyrrolo[2,3-b]pyridine-5-carboxylate |
| **24** | Ethyl 3-[(1H-indazol-6-ylamino)methylidene]-2-oxo-1,2-dihydro-3H-pyrrolo[2,3-b]pyridine-5-carboxylate |
| **25** | Ethyl 2-oxo-3-[(6-quinolinylamino)methylidene]-2,3-dihydro-1H-pyrrolo[2,3-b]pyridine-5-carboxylate |

The invention discloses 10 different points of substitution on structural formula (I). Each of these points of substitution bears a substituent whose selection and synthesis as part of this invention was independent of all other points of substitution on formula (I). Thus, each point of substitution is now further described individually.

Preferred substitutions at the R¹ position include hydrogen, halogen, amide, nitro, lower alkyl, hydroxy, hydroxyalkyl, pyrimidineloweralkyl, loweralkoxycarbonyl, cyclic loweralkyl, hydroxyphenylloweralkyl, phenoxy, alkoxy and pyrazole; and R¹ fused with R² to form a fused ring selected from the group consisting of: thiazole, pyrazole, triazole, halogen-substituted diazole, acyl substituted pyrrole and pyridine. Most preferred substituents at R¹ include hydrogen and methyl and R¹ fused with R² for form fused thiazole or fused pyridine. The most highly preferred substitution at the R¹ position is hydrogen.

Preferred substitutions at the R² position include hydrogen, halogen, sulfate, amine, quaternary amine, amide, ester, phenyl, alkoxy, aminosulfonyl, lower alkyl sulfonyl, furanyl lower alkyl amide, pyridinyl lower alkyl amide, alkoxy-substituted phenyl lower alkyl amide, morpholino lower alkyl amide, imidazolyl lower alkyl amide, hydroxy lower alkyl amide, alkoxy lower alkyl amide, lower alkyl amide, lower alkyl sulfonamide, lower alkyl hydroxy substituted amino, nitro, halogen-substituted phenoxycarbonyl and triazole and oxazole rings, or are R² fused with R³ to form a fused ring selected from the group consisting of: oxazole, pyrrole, and dioxolane, which fused ring is optionally substituted by lower alkyl or lower alkyl carbonyl, and which fused ring is optionally a hetero ring having nitrogen as the heteroatom and forming a quaternary ammonium salt ionically bonded with a halogen atom. Most preferred substituents at R² include hydrogen, phenyl, 2-furanyl, 3-thiophenyl, bromo and carbethoxy.

Preferred substitutions at R³ include hydrogen, lower alkyl, hydroxy lower alkyl, halogen, phenoxy and alkoxy. Most preferred include hydrogen and chloro. Most highly preferred is hydrogen.

Preferred substitutions at R⁴ include sulfonylamino, sulfonylaminoamino, lower alkyl sulfonylamino, lower alkylsulfonyl lower alkyl, alkoxysulfonylamino, phenylcarbonylsulfonylamino, phenoxysulfonyl, hydroxy lower alkylsulfonylamino, hydroxy lower alkylsulfonylamino lower alkyl, alkyl, phenylsulfonylamino (optionally substituted by halogen-substituted lower alkyl), aminoiminosulfonylamino, alkylsulfonylaminoalkyl, pyridinyl lower alkyl sulfonylamino, benzamideazolesulfonylamino, pyridylsulfonylamino, pyrimidinylsulfonylamino, thiadiazolylsulfonylamino (optionally substituted by lower alkyl), thiazolesulfonylamino, hydroxyalkoxyalkylsulfonylamino and 4'-SO₂NH[(CH₂)₂O]₄CH₃, or R⁴ fused with R⁵ to form a fused ring selected from the group consisting of imidazole, triazole, cyclic sulfonylamino and thiaphene, where said fused ring is optionally disubstituted on the sulfur heteroatom by oxo. The most preferred substitutions include 2-pyridine sulfonylamino, 4-pyridine sulfonylamino, hydroxy n-butyl sulfonylamino, methylsulfonylaminomethylene, sulfonyldimethylamino, fused 1,2-pyrazole and sulfonylamino. In a most highly preferred embodiment, R⁴ is sulfonylamino or fused 1,2-pyrazole.

The preferred substitution at R⁵ is hydrogen.

Preferred substitutions at X include N, CH and CCH₃. Most preferred is CH.

The preferred substitution at Y is N or C.

The preferred substitution at Z is N or C.

The preferred substitution at A is N or C.

The preferred substitution at D is N or C.

### DETAILED DESCRIPTION OF THE INVENTION

Salts encompassed within the term "pharmaceutically acceptable salts" refer to non-toxic salts of the compounds of this invention which are generally prepared by reacting the free base with a suitable organic or inorganic acid or by reacting the acid with a suitable organic or inorganic base. Representative salts include the following salts: Acetate, Benzenesulfonate, Benzoate, Bicarbonate, Bisulfate, Bitartrate, Borate, Bromide, Calcium Edetate, Camsylate, Carbonate, Chloride, Clavulanate, Citrate, Diethanolamine, Dihydrochloride, Edetate, Edisylate, Estolate, Esylate, Fumarate, Gluceptate, Gluconate, Glutamate, Glycollylarsanilate, Hexylresorcinate, Hydrabamine, Hydrobromide, Hydrocloride, Hydroxynaphthoate, Iodide, Isethionate, Lactate, Lactobionate, Laurate, Malate, Maleate, Mandelate, Mesylate, Metaphosphoric, Methylbromide, Methylnitrate, Methylsulfate, Monopotassium Maleate, Mucate, Napsylate, Nitrate, N-methylglucamine, Oxalate, Pamoate (Embonate), Palmitate, Pantothenate, Phosphate/diphosphate, Polygalacturonate, Potassium, Salicylate, Sodium, Stearate, Subacetate, Succinate, Tannate, Tartrate, Teoclate, Tosylate, Trifluoroacetate, Triethiodide, Trimethylammonium and Valerate.

Other salts which are not pharmaceutically acceptable may be useful in the preparation of compounds of formula (I) and these form a further aspect of the invention.

Also included within the scope of the invention are the individual isomers of the compounds represented by formula (I) above as well as any wholly or partially equilibrated mixtures thereof. The present invention also covers the individual isomers of the compounds represented by formula above as mixtures with isomers thereof in which one or more chiral asymmetric centers are inverted.

As used herein, the term "aliphatic" refers to the terms alkyl, alkylene, alkenyl, alkenylene, alkynyl and alkynylene.

As used herein, the term "lower" refers to a group having between one and six carbons.

As used herein, the term "alkyl" refers to a straight or branched chain hydrocarbon having from one to twelve carbon atoms, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. Examples of "alkyl" as used herein include, but are not limited to, n-butyl, n-pentyl, isobutyl and isopropyl, and the like.

As used herein, the term "alkylene" refers to a straight or branched chain divalent hydrocarbon radical having from one to ten carbon atoms, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. Examples of "alkylene" as used herein include, but are not limited to, methylene, ethylene, and the like.

As used herein, the term "alkenyl" refers to a hydrocarbon radical having from two to ten carbons and at least one carbon - carbon double bond, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed.

As used herein, the term "alkenylene" refers to an straight or branched chain divalent hydrocarbon radical having from two to ten carbon atoms and one or more carbon - carbon double bonds, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. Examples of "alkenylene" as used herein include, but are not limited to, ethene-1,2-diyl, properie-1,3-diyl, methylene-1,1-diyl, and the like.

As used herein, the term "alkynyl" refers to a hydrocarbon radical having from two to ten carbons and at least one carbon - carbon triple bond, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed.

As used herein, the term "alkynylene" refers to a straight or branched chain divalent hydrocarbon radical having from two to ten carbon atoms and one or more carbon - carbon triple bonds, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. Examples of "alkynylene" as used herein include, but are not limited to, ethyne-1,2-diyl, propyne-1,3-diyl, and the like.

As used herein, the term "cycloaliphatic" refers to the terms cycloalkyl, cydoalkylene, cycloalkenyl, cycloalkenylene, cycloalkynyl and cycloalkylnylene.

As used herein, "cycloalkyl" refers to a alicyclic hydrocarbon group with one or more degrees of unsaturation, having from three to twelve carton atoms, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. "Cycloalkyl" indudes, by way of example, cyclopropyl, cyclobutyl, cyclopentyl, cyclohexyl, cycloheptyl, or cyclooctyl, and the like.

As used herein, the term "cycloalkylene" refers to an non-aromatic alicyclic divalent hydrocarbon radical having from three to twelve carbon atoms, optionally substituted with substituents selected from the group consisting of: tower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. Examples of "cycloalkylene" as used herein include, but are not limited to, cyclopropyl-1,1-diyl, cyclopropyl-1,2-diyl, cydobutyl-1,2-diyl, cydopentyl-1,3-diyl, cyclohexyl-1,4-diyl, cycloheptyl-1,4-diyl, or cyclooctyl-1,5-diyl, and the like.

As used herein, the term "cycloalkenyl" refers to a substituted alicyclic hydrocarbon radical having from three to twelve carbon atoms and at least one carbon-carbon double bond in the ring system, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. Examples of "cycloalkenylene" as used herein include, but are not limited to, 1-cyclopentene-3-yl, 1-cyclohexene-3-yl, 1-cycloheptene-4-yl, and the like.

As used herein, the term "cycloalkenylene" refers to a substituted alicyclic divalent hydrocarbon radical having from three to twelve carbon atoms and at least one carbon-carbon double bond in the ring system, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. Examples of "cycloalkenylene" as used herein include, but are not limited to, 4,5-cyclopentene-1,3-diyl, 3,4-cyclohexene-1,1-diyl, and the like.

As used herein, the term "heteroatom ring system" is inclusive of heterocyclic, heterocyclyl, heteroaryl and heteroarylene ring systems. Nonlimiting examples of such heteroatom ring systems are recited in the Summary of the Invention, above.

As used herein, the term "heterocyclic" or the term "heterocyclyl" refers to a three to twelve-membered heterocyclic ring having one or more degrees of unsaturation containing one or more heteroatomic substitutions selected from S, SO, SO₂, O, or N, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. Such a ring may be optionally fused to one or more of another "heterocyclic" ring(s) or cycloalkyl ring(s). Examples of "heterocyclic" include, but are not limited to, tetrahydrofuran, pyran, 1,4-dioxane, 1,3-dioxane, piperidine, pyrrolidine, morpholine, tetrahydrothiopyran, tetrahydrothiophene, and the like.

As used herein, the term "heterocyclylene" refers to a three to twelve-membered heterocyclic ring diradical having one or more degrees of unsaturation containing one or more heteroatoms selected from S, SO, SO₂, O, or N, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or lower perfluoroalkyl, multiple degrees of substitution being allowed. Such a ring may be optionally fused to one or more benzene rings or to one or more other "heterocyclic" rings or cycloalkyl rings. Examples of "heterocyclylene" rings include, but are not limited to, tetrahydrofuran-2,5-diyl, morpholine-2,3-diyl, pyran-2,4-diyl, 1,4-dioxane-2,3-diyl, 1,3-dioxane-2,4-diyl, piperidine-2,4-diyl, piperidine-1,4-diyl, pyrrolidine-1,3-diyl, morpholine-2,4-diyl, and the like.

As used herein, the term "aryl" refers to a benzene ring or to an optionally substituted benzene ring system fused to one or more optionally substituted benzene rings to form anthracene, phenanthrene, or napthalene ring systems, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, tetrazolyl, carbamoyl optionally substituted by alkyl, aminosulfonyl optionally substituted by alkyl, acyl, aroyl, heteroaroyl, acyloxy, aroyloxy, heteroaroyloxy, alkoxycarbonyl, nitro, cyano, halogen, lower perfluoroalkyl, heteroaryl, or aryl, multiple degrees of substitution being allowed. Examples of aryl include, but are not limited to, phenyl, 2-naphthyl, 1-naphthyl, biphenyl, and the like.

As used herein, the term "arylene" refers to a benzene ring diradical or to a benzene ring system diradical fused to one or more optionally substituted benzene rings, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, tetrazolyl, carbamoyl optionally substituted by alkyl, aminosulfonyl optionally substituted by alkyl, acyl, aroyl, heteroaroyl, acyloxy, aroyloxy, heteroaroyloxy, alkoxycarbonyl, nitro, cyano, halogen, lower perfluoroalkyl, heteroaryl, or aryl, multiple degrees of substitution being allowed. Examples of "arylene" include, but are not limited to, benzene-1,4-diyl, naphthalene-1,8-diyl, anthracene-1,4-diyl, and the like.

As used herein, the term "heteroaryl" refers to a five - to seven - membered aromatic ring, or to a polycyclic heterocyclic aromatic ring, containing one or more nitrogen, oxygen, or sulfur heteroatoms at any position, where N-oxides and sulfur monoxides and sulfur dioxides are permissible heteroaromatic substitutions, optionally substituted with substituents selected from the group which indudes lower alkyl, lower alkoxy, lower alkylsulfanyl, lower alkylsulfenyl, lower alkylsulfonyl, oxo, hydroxy, mercapto, amino optionally substituted by alkyl, carboxy, tetrazolyl, carbamoyl optionally substituted by alkyl, aminosulfonyl optionally substituted by alkyl, acyl, aroyl, heteroaroyl, acyloxy, aroyloxy, heteroaroyloxy, alkoxycarbonyl, nitro, cyano, halogen, lower perfluoroalkyl, heteroaryl and aryl, multiple degrees of substitution being allowed. For polycyclic aromatic ring systems, one or more of the rings may contain one or more heteroatoms. Examples of "heteroaryl" used herein are furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, indole and indazole, and the like.

As used herein, the term "heteroarylene" refers to a five - to seven - membered aromatic ring diradical, or to a polycyclic heterocyclic aromatic ring diradical, containing one or more nitrogen, oxygen, or sulfur heteroatoms, where N-oxides and sulfur monoxides and sulfur dioxides are permissible heteroaromatic substitutions, optionally substituted with substituents selected from the group consisting of: lower alkyl, lower alkoxy, lower alkylsulfanyl, lower alkylsulfenyl, lower alkylsulfonyl, oxo, hydroxy, mercapto, amino optionally substituted by alkyl, carboxy, tetrazolyl, carbamoyl optionally substituted by alkyl, aminosulfonyl optionally substituted by alkyl, acyl, aroyl, heteroaroyl, acyloxy, aroyloxy, heteroaroyloxy, alkoxycarbonyl, nitro, cyano, halogen, lower perfluoroalkyl, heteroaryl, or aryl, multiple degrees of substitution being allowed. For polycyclic aromatic ring system diradicals, one or more of the rings may contain one or more heteroatoms. Examples of "heteroarylene" used herein are furan-2,5-diyl, thiophene-2,4-diyl, 1,3,4-oxadiazole-2,5-diyl, 1,3,4-thiadiazole-2,5-diyl, 1,3-thiazole-2,4-diyl, 1,3-thiazole-2,5-diyl, pyridine-2,4-diyl, Pyridine-2,3-diyl, Pyridine-2,5-diyl, pyrimidine-2,4-diyl, quinoline-2,3-diyl, and the like.

As used herein, the term "alkoxy" refers to the group RₐO-, where Rₐ is aliphatic.

As used herein, the term "alkylsulfanyl" refers to the group RₐS-, where Rₐ is aliphatic.

As used herein, the term "alkylsulfenyl" refers to the group RₐS(O)-, where Rₐ is aliphatic.

As used herein, the term "alkylsulfonyl" refers to the group RₐSO₂-, where Rₐ is aliphatic.

As used herein, the term "acyl" refers to the group RₐC(O)-, where Rₐ is aliphatic, cycloaliphatic, or heterocyclyl.

As used herein, the term "aroyl" refers to the group RₐC(O)- , where Rₐ is aryl.

As used herein, the term "heteroaroyl" refers to the group RₐC(O)-, where Rₐ is heteroaryl.

As used herein, the term "alkoxycarbonyl" refers to the group RₐOC(O)-, where Rₐ is aliphatic.

As used herein, the term "acyloxy" refers to the group RₐC(O)O- , where Rₐ is aliphatic, cycloaliphatic, or heterocyclyl.

As used herein, the term "aroyloxy" refers to the group RₐC(O)O- , where Rₐ is aryl.

As used herein, the term "heteroaroyloxy" refers to the group RₐC(O)O- where Rₐ is heteroaryl.

As used herein, the term "optionally" is inclusive of embodiments in which a described condition is present and embodiments in which such described condition is not present, for example, where the term is used with reference to a chemical substituent, it indicates the inclusion of embodiments in which the specified substituent is present as well as embodiments in which the specified substituent is not present.

As used herein, the term "substituted" refers to substitution with the named substituent or substituents, multiple degrees of substitution being allowed.

As used herein, the terms "contain" or "containing" in reference to any of the above-defined alkyl, alkenyl, alkynyl or cycloalkyl substituents, are inclusive of in-line substitutions at any position along such alkyl, alkenyl, alkynyl or cycloalkyl substituents, with one or more of any of O, S, SO, SO₂, N, or N-alkyl, including, for example, -CH₂-O-CH₂-, -CH₂-SO₂-CH₂-, -CH₂-NH-CH₃ and so forth.

As used herein, the term "solvate" is a complex of variable stoichiometry formed by a solute (in this invention, a compound of formula (I)) and a solvent. Such solvents for the purpose of the invention may not interfere with the biological activity of the solute. Solvents may be, by way of example, water, ethanol, or acetic acid.

The compounds of the present invention have the ability to crystallize in more than one form, a characteristic which is known as polymorphism, and such polymorphic forms ("polymorphs") are within the scope of the present invention. Polymorphism generally can occur as a response to changes in temperature or pressure or both and can also result from variations in the crystallization process. Polymorphs can be distinguished by various physical characteristics known in the art such as x-ray diffraction patterns, solubility, and melting point.

As used herein, the terms "biohydrolyzable carbonate", "biohydrolyzable ureide" and "biohydcolyzable carbamate" include carbonates, ureides, and carbamates, respectively, of a compound of the general formula (I) which carbonates, ureides, and carbamates, do not completely diminish the biological activity of the parent substance. Such biohydrolyzable carbonates, ureides, and carbamates may confer on the parent compound of the general formula (I) advantageous properties *in vivo*, such as improved duration of action, onset of action, and the like. Also included are compounds which are relatively biologically inactive but which are converted in vivo by the subject to the biologically active principle. An advantage of such biohydrolyzable forms is that, for example, they facilitate improved oral administration because the carbonates, ureides, and carbamates are more readily absorbed from the gut and are then transformed to a compound of formula (I) in plasma. Many of such biohydrolyzable compounds are known in the art and include, by way of example, lower alkyl carbamates.

As used herein, the term "biohydrolyzable ester" is an ester of a compound of general formula which does not completely diminish the biological activity of the parent substance. Such esters may confer on the parent compound of the general formula (I) advantageous properties *in vivo,* such as improved duration of action, onset of action, and the like. Also included are esters which are relatively biologically inactive but which are converted in vivo by the subject to the biologically active principle. An advantage of such biohydrolyzable esters is that, for example, they facilitate improved oral administration because they are more readily absorbed from the gut and are then transformed to a compound of formula (I) in plasma. Many biohydrolyzable esters are known in the art and include, by way of example, lower alkyl esters, lower acyloxy-alkyl esters, lower alkoxyacyloxyalkyl esters, alkoxyacyloxy esters, alkyl acylamino alkyl esters and choline esters.

As used herein, the term "biohydrolyzable amide" is an amide of a compound of general formula which does not completely diminish the biological activity of the parent substance. Such amides may confer on the parent compound of the general formula (I) advantageous properties *in vivo*, such as improved duration of action, onset of action, and the like. Also included are amides which are relatively biologically inactive but which are converted in vivo by the subject to the biologically active principle. An advantage of such biohydrolyzable amides is that, for example, they facilitate improved oral administration because they are more readily absorbed from the gut and are then transformed to a compound of formula (I) in plasma. Many biohydrolyzable amides are known in the art and include, by way of example, lower alkyl amides, α-amino acid amides, alkoxyacyl amides and alkylaminoalkylcarbonyl amides.

As used herein, the term "prodrug" includes biohydrolyzable amides, biohydrolyzable esters and biohydrolyzable carbamates and also encompasses compounds in which the biohydrolyzable functionality in such prodrug is encompassed in the compound of formula (I): for example, a lactam formed by a carboxylic group in R₁ and an amine in R₂, and compounds which may be oxidized or reduced biologically at a given functional group to yield drug substances of formula (I). Examples of these functional groups are, but are not limited to, 1,4-dihydropyridine, N-alkylcarbonyl-1,4-dihydropyridine, 1,4-cyclohexadiene, tert-butyl, and the like.

As used herein, the term "affinity reagent" is a group attached to the compound of formula (I) which does not affect its in vitro biological activity, allowing the compound to bind to a target, yet such a group binds strongly to a third component allowing (a) characterization of the target as to localization within a cell or other organism component, perhaps by visualization by fluorescence or radiography, or (b) facile separation of the target from an unknown mixture of targets, whether proteinaceous or not proteinaceous. An example of an affinity reagent according to (b) would be biotin either directly attached to (I) or linked with a spacer of one to 50 atoms selected from the group consisting of C, H, O, N, S, or P in any combination. An example of an affinity reagent according to (a) above would be fluorescein, either directly attached to (I) or linked with a spacer of one to 50 atoms selected from the group consisting of C, H, O, N, S, or P in any combination.

The term "effective amount" means that amount of a drug or pharmaceutical agent that will elicit the biological or medical response of a tissue, system, animal or human that is being sought by a researcher or clinician. The term "therapeutically effective amount" means any amount which, as compared to a corresponding subject who has not received such amount, results in improved treatment, healing, prevention, or amelioration of a disease or disorder, or a decrease in the rate of advancement of a disease or disorder, and also includes amounts effective to enhance normal physiological function.

Whenever the terms "aliphatic" or "aryl" or either of their prefixes appear in a name of a substituent (e.g. arylalkoxyaryloxy) they shall be interpreted as including those limitations given above for "aliphatic" and "aryl". Aliphatic or cycloalkyl substituents shall be recognized as being term equivalents to those having one or more degrees of unsaturation. Designated numbers of carbon atoms (e.g. C₁₋₁₀) shall refer independently to the number of carbon atoms in an aliphatic or cyclic aliphatic moiety or to the aliphatic portion of a larger substituent in which the term "aliphatic" appears as a prefix (e.g. "al-").

As used herein, the term "disubstituted amine" or "disubstituted amino-" shall be interpreted to include either one or two substitutions on that particular nitrogen atom.

As used herein, the term "oxo" shall refer to the substituent =O.

As used herein, the term "halogen" or "halo" shall include iodine, bromine, chlorine and fluorine.

As used herein, the term "mercapto" shall refer to the substituent -SH.

As used herein, the term "carboxy" shall refer to the substituent - COOH.

As used herein, the term "cyano" shall refer to the substituent -CN.

As used herein, the term "aminosulfonyl" shall refer to the substituent -SO₂NH₂.

As used herein, the term "carbamoyl" shall refer to the substituent - C(O)NH₂.

As used herein, the term "sulfanyl" shall refer to the substituent -S-.

As used herein, the term "sulfenyl" shall refer to the substituent -S(O)-.

As used herein, the term "sulfonyl" shall refer to the substituent - S(O)₂-.

The compounds of formula (I) are readily synthesized using various synthetic procedures known in the art and are readily prepared according to the following reaction Synthesis Schemes (in which all variables are as defined herein) and examples or modifications thereof using readily available starting materials, reagents and conventional synthesis procedures. In these reactions, it is also possible to make use of variants which are themselves known to those of ordinary skill in this art, but are not mentioned in greater detail.

### Synthesis Schemes

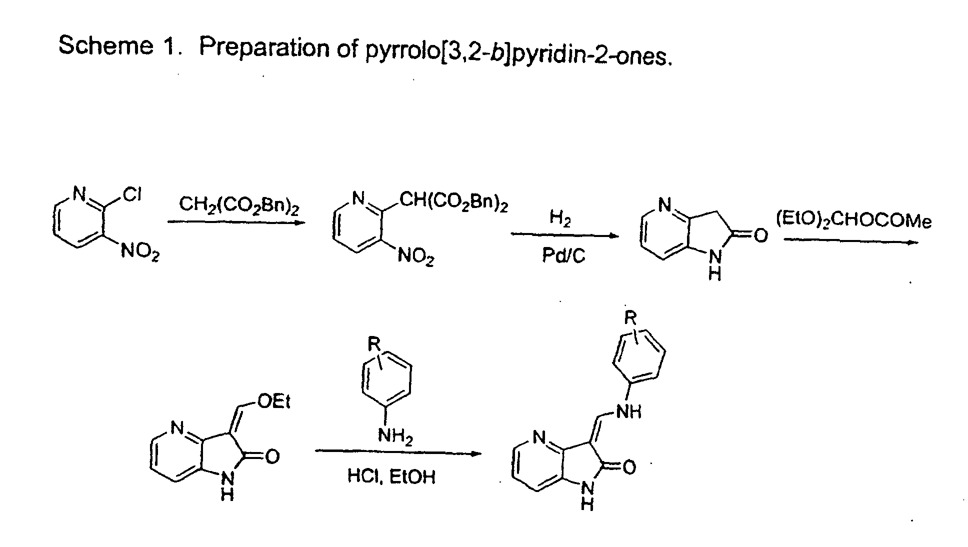

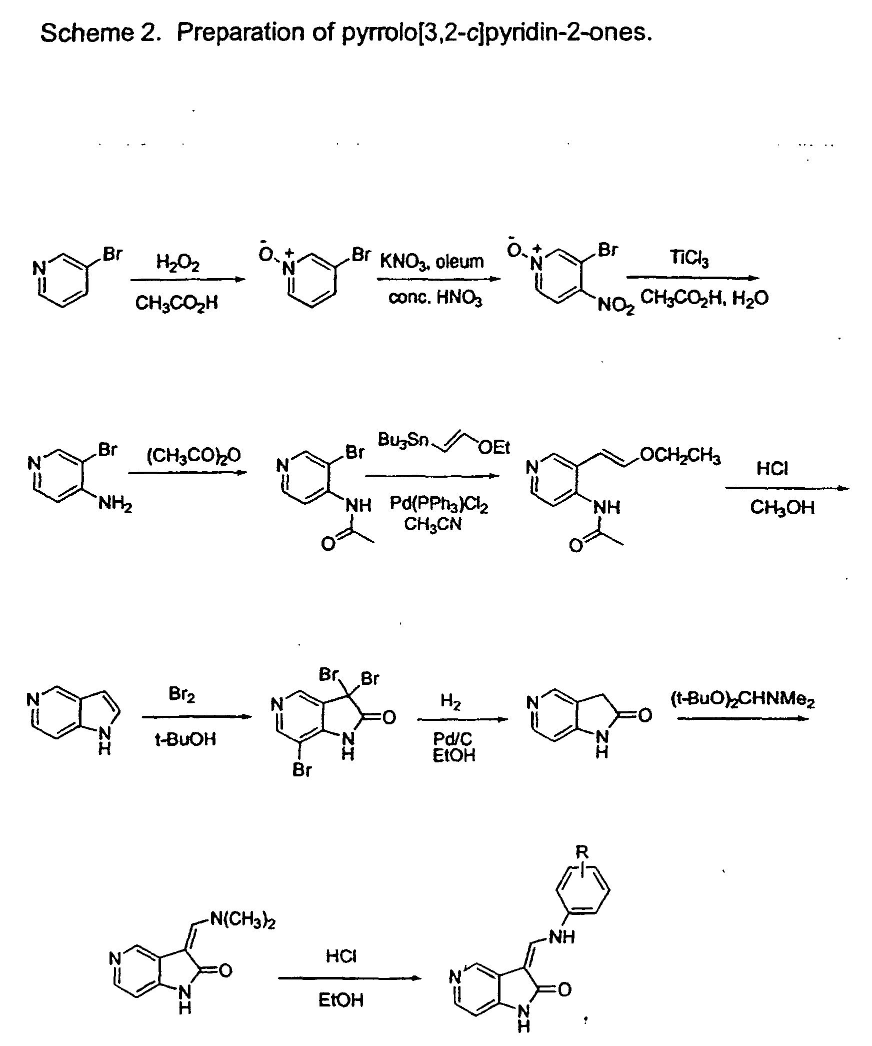

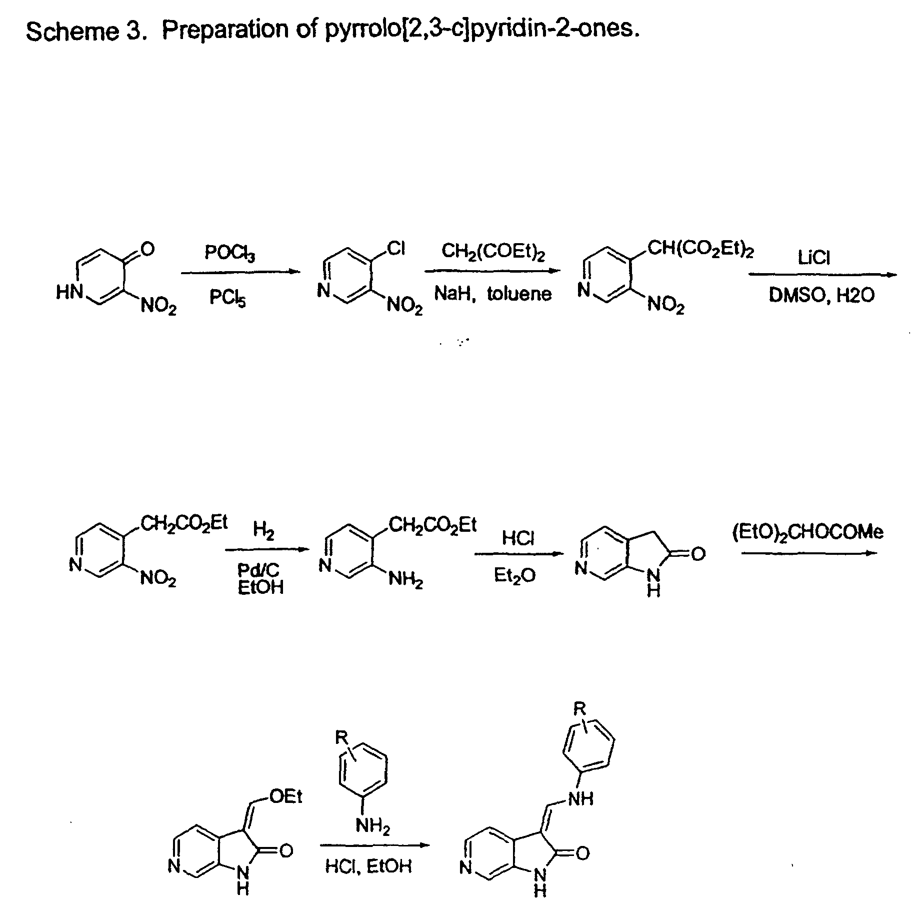

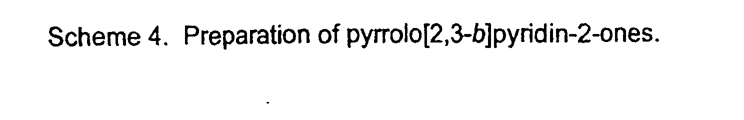

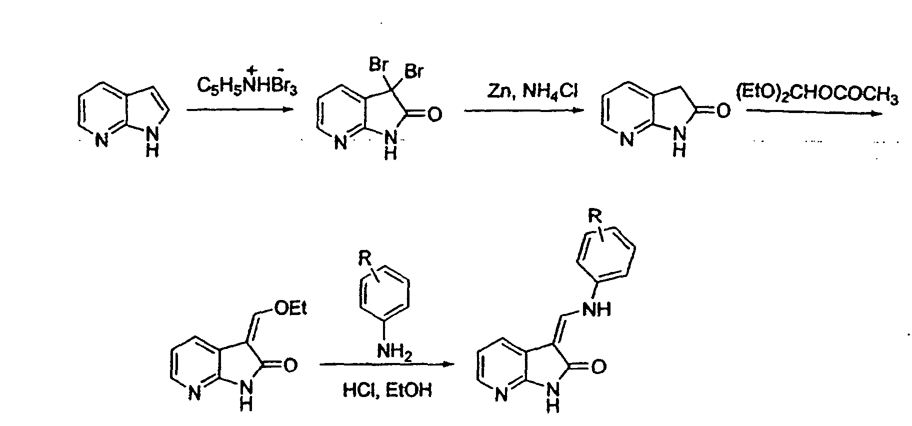

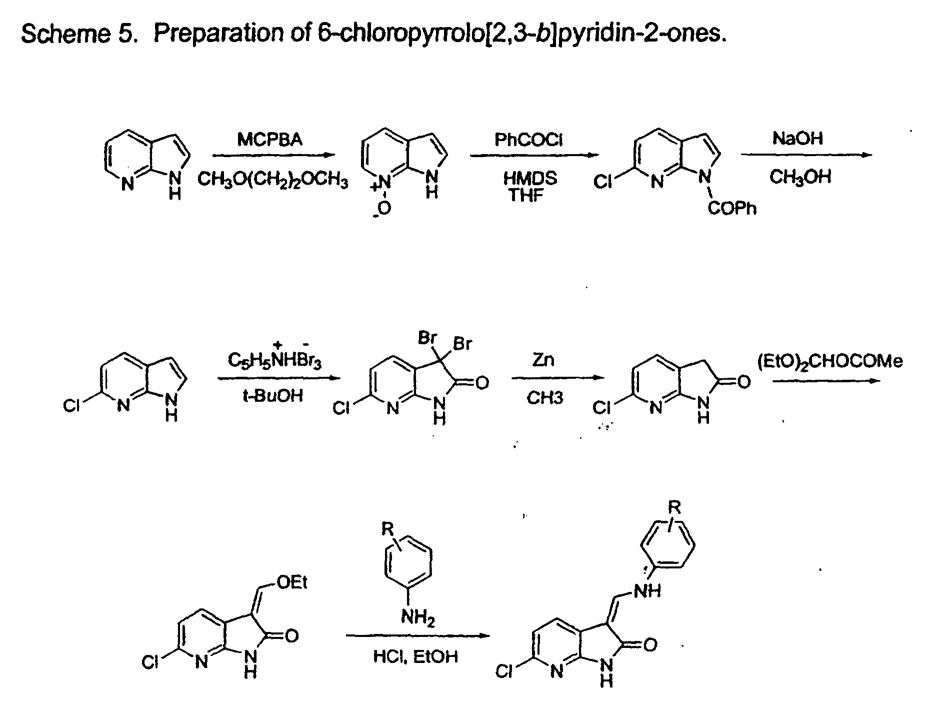

In the foregoing Schemes 1-5, R is R⁴ and/or R⁵ as described herein. 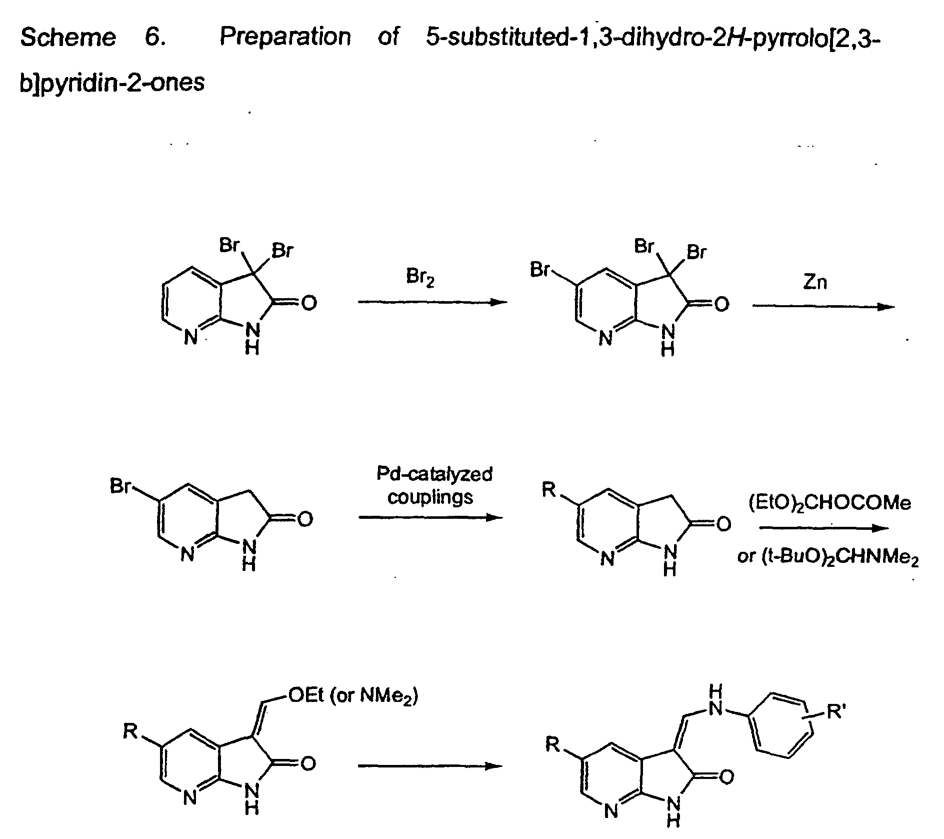 wherein R is Het, Ar or CO₂Et, where Het and Ar are as described herein.

The following are three preferred synthetic schemes according to the present invention:

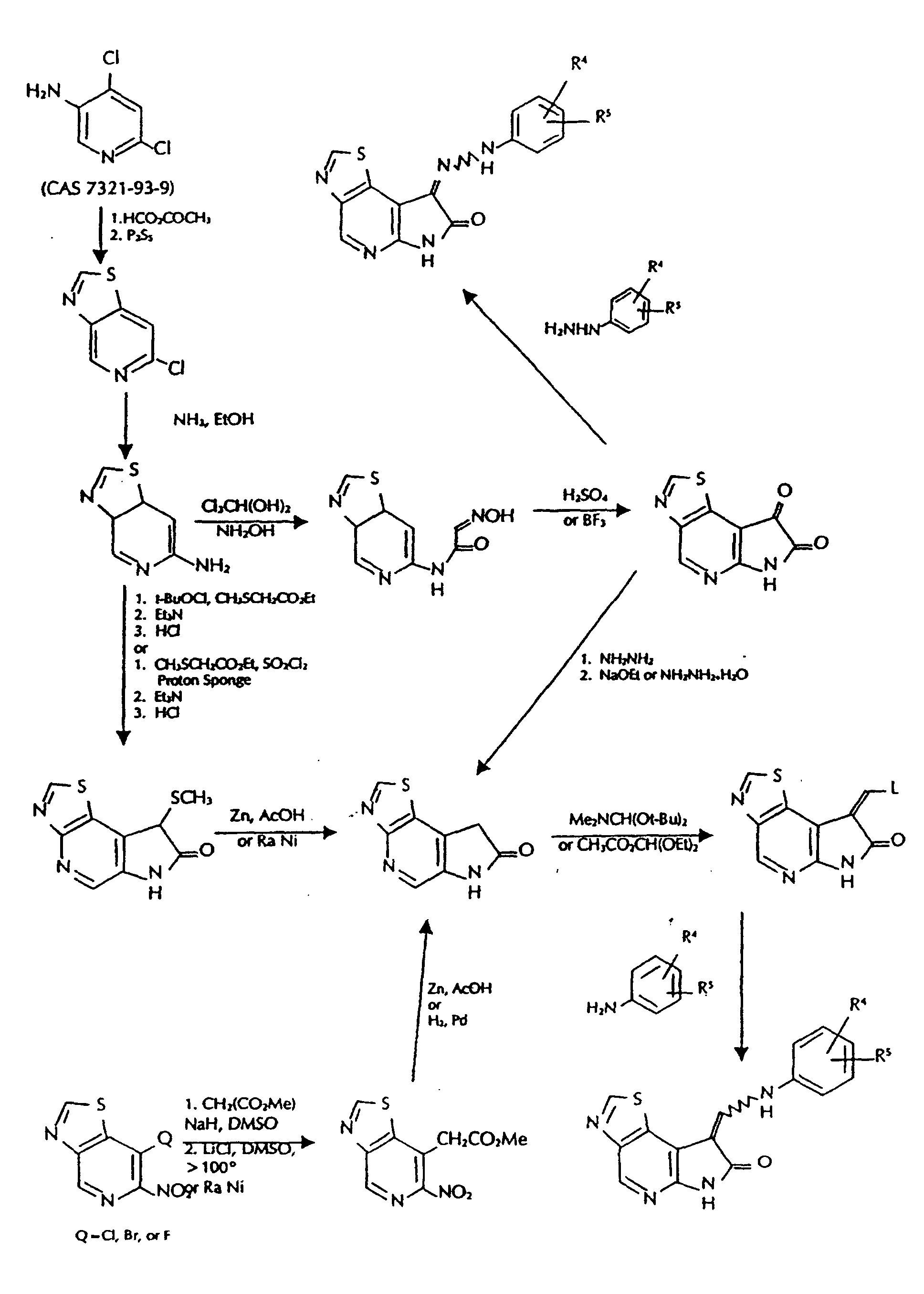

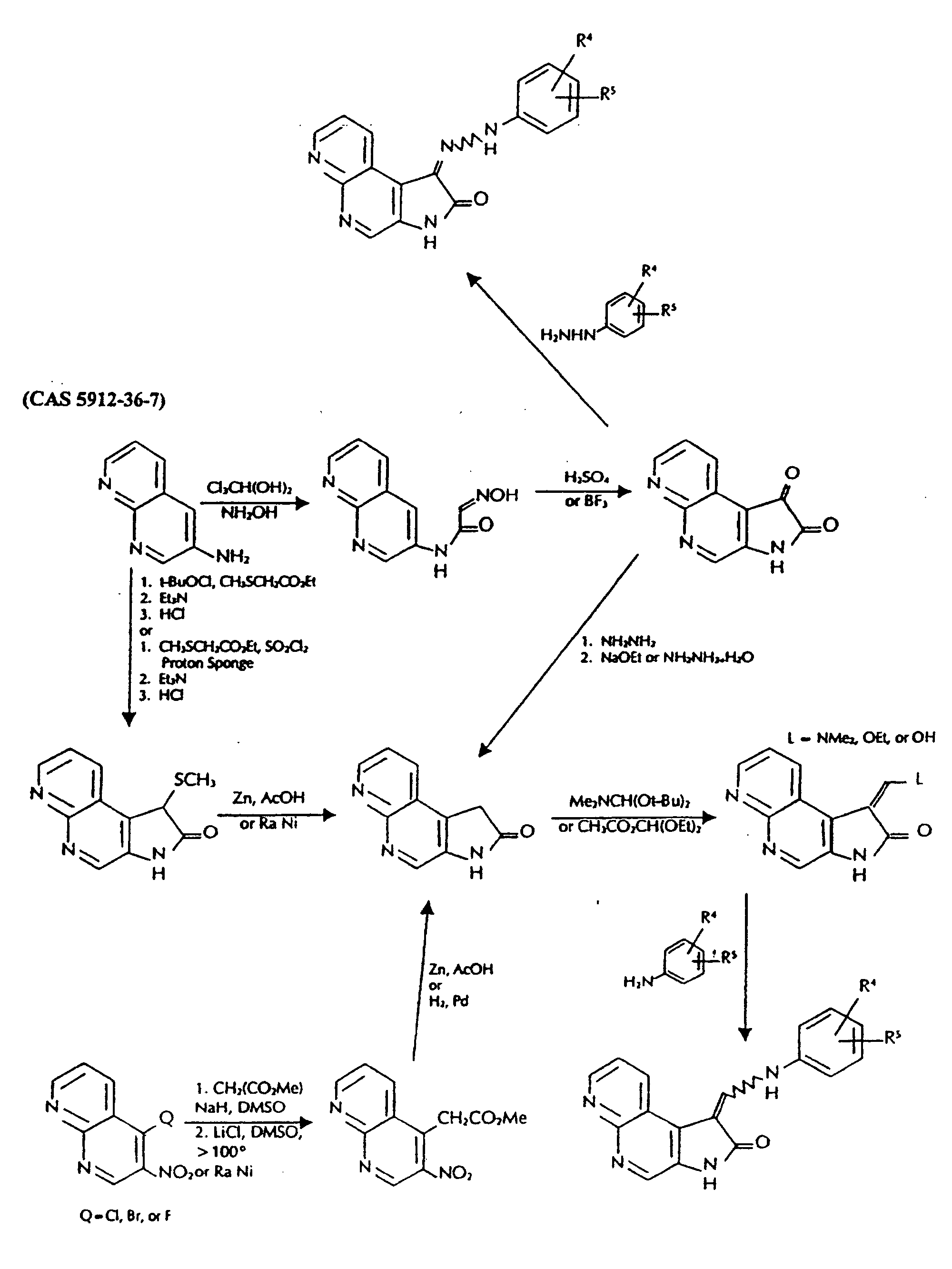

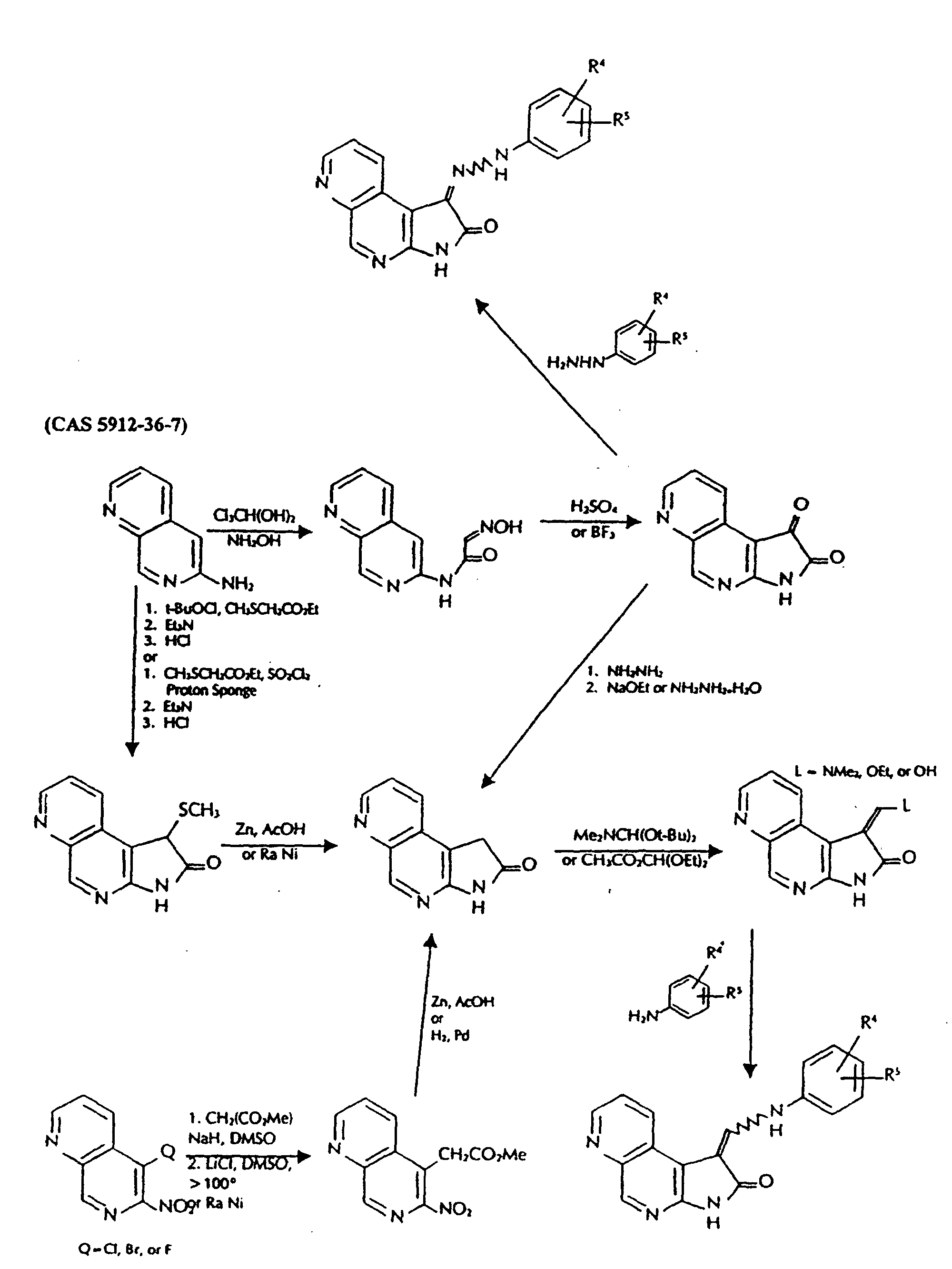

The most preferred compounds of the invention are any or all of those specifically set forth in these examples. These compounds are not, however, to be construed as forming the only genus that is considered as the invention, and any combination of the compounds or their moieties may itself form a genus. The following examples further illustrate details for the preparation of the compounds of the present invention. Those skilled in the art will readily understand that known variations of the conditions and processes of the following preparative procedures can be used to prepare these compounds. All temperatures are degrees Celsius unless noted otherwise.

Abbreviations used in the Examples are as follows:
- g: = grams
- mg: = milligrams
- L: = liters
- mL: = milliliters
- M: = molar
- N: = normal
- mM: = millimolar
- i.v.: = intravenous
- p.o.: = per oral
- s.c.: = subcutaneous
- Hz: = hertz
- mol: = moles
- mmol: = millimoles
- mbar: = millibar
- psi: = pounds per square inch
- rt: = room temperature
- min: = minutes
- h: = hours
- mp: = melting point
- TLC: = thin layer chromatography
- R_{f}: = relative TLC mobility
- MS: = mass spectrometry
- NMR: = nuclear magnetic resonance spectroscopy
- APCI: = atmospheric pressure chemical ionization
- ESI: = electrospray ionization
- m/z: = mass to charge ratio
- tᵣ: = retention time
- Pd/C: = palladium on activated carbon
- ether: = diethyl ether
- MeOH: = methanol
- EtOAc: = ethyl acetate
- TEA: = triethylamine
- DIEA: = diisopropylethylamine
- THF: = tetrahydrofuran
- DMF: = N, N-dimethylformamide
- DMSO: = dimethylsulfoxide
- DDQ: =2,3-dichloro-5,6-dicyano-1,4-benzoquinone
- LAH: = lithium aluminum hydride
- TFA: = trifluoroacetic acid
- LDA: = lithium diisopropylamide
- THP: = tetrahydropyranyl
- NMM: = N-methylmorpholine, 4-methylmorpholine
- HMPA: = hexamethylphosphoric triamide
- DMPU: = 1,3-dimethypropylene urea
- d: = days
- ppm: = parts per million
- kD: = kiloDalton
- LPS: = lipopolysaccharide
- PMA: = phorbol myristate acetate
- SPA: = scintillation proximity assay
- EDTA: = ethylenediamine tetraacetic acid
- FBS: = fetal bovine serum
- PBS: = phosphate buffered saline solution
- BrdU: = bromodeoxyuridine
- BSA: = bovine serum albumin
- FCS: = fetal calf serum
- DMEM: = Dulbecco's modified Eagle's medium
- pfu: = plaque forming units
- MOI: = multiplicity of infection

Reagents are commercially available or are prepared according to procedures in the literature. The physical data given for the compounds exemplified is consistent with the assigned structure of those compounds. ¹H NMR spectra were obtained on VARIAN Unity Plus NMR spectrophotometers at 300 or 400 Mhz. Mass spectra were obtained on Micromass Platform II mass spectrometers from Micromass Ltd. Altrincham, UK, using either Atmospheric Chemical Ionization (APCI) or Electrospray Ionization (ESI). Analytical thin layer chromatography (TLC) was used to verify the purity of some intermediates which could not be isolated or which were too unstable for full characterisation, and to follow the progess of reactions. Unless otherwise stated, this was done using silica gel (Merck Silica Gel 60 F254). Unless otherwise stated, column chromatography for the purification of some compounds, used Merck Silica gel 60 (230-400 mesh), and the stated solvent system under pressure.

A Micromass Platform II mass spectrometer equipped with an electrospray ion source was used to acquire low resolution LC-MS data for the samples that were prepared in library format. The system software runs on a PC computer with the Microsoft operating system, and consists of Masslynx v3.1 and Openlynx v3.1 software packages. The mass spectrometer inlet system was comprised of a Hewlett Packard 1100 HPLC Chromatograph, a Gilson 215 autosampler, and a Hewlett Packard 1100 photo-diode array detector. A Supelco ABZ+ 5cm column was used to provide separations prior to electrospray ionization. The HPLC was programmed as follows:

| **Time Flow Rate** | **%A** | **%B** |
|---|---|---|
| 0.0 min | 85 | 15 |
| 0.6ml/min | | |
| 3.0 min | 25 | 75 |
| 0.6ml/min | | |
| 4.0 min | 0 | 100 |
| 0.6ml/min | | |
| 5.0 min | 0 | 100 |
| 0.6ml/min | | |

The data were processed automatically using standard peak detection parameters provided by the Openlynx software.

Micromass LCT bench-top mass spectrometer, equipped with an electrospray ionization source was used to obtain accurate mass data for the samples that were prepared in library format. The LCT utilizes two hexapole RF lenses to transfer ions from the source to an orthogonal acceleration time-of-flight (TOF) analyser. The ions emerging from the analyser are detected using a dual microchannel plate detector and ion counting system. The system software runs on a PC computer with the Microsoft operating system, and consists of Masslynx v3.2 and Openlynx v3.2 software packages. The mass spectrometer inlet system is comprised of a Waters Alliance 2690 Separations Module, Waters 2700 autosampler, Waters 996 photo-diode array detector and Valco column switching device. A mobile phase flow rate of 1 ml/min exits the Alliance 2690 and is reduced to a mass spectrometer flow rate of 20ul/min using an Acurate flow splitter. A lock mass solution at a flow rate of 4ul/min is added to the spectrometer flow via a Harvard syringe pump and a tee piece placed immediately before the electrospray probe. The instrument resolution was determined by acquiring a spectrum and measuring the full peak width t half peak height (FWHH). The instrument was tuned to provide a resolution of 4600 to 5000 (FWHH). The instrument was calibrated using the ions of polyethylene glycol (PEG) as reference standards. The lock mass used [3,5-Dil-Tyr, Ala,N-Me-Phe, Gly-0I] Enkephalin (MH+ C26H34I2N5O6= 766.0599) at a concentration of 5 ng/ul.

### Example 1: 4-{[(2-Oxo-1,2-dihydro-3H-pyrrolo[3,2-c]pyridin-3-ylidene)-methyl] amino}benzenesulfonamide

### a) 3-Dimethylamino-methylene-1,5-diazainden-2-one:

3-Bromo-4-nitropyridine-1-oxide was prepared according to the procedure of Daisley and Hanbali (Org. Prep. Proced. Int. **1983,** *15,* 280) and converted to 1,5-diazaindene via the method of Sakamoto et. al. (Heterocycles **1992,** *34,* 2379). 1,5-Diazaindene was subsequently converted to 1,5-diazainden-2-one hydrobromide via the procedure outlined by Robinson and Donahue (J. Org. Chem. **1991**, *56,* 4805) and reaction of this with N,N-dimethylformamide-di-t-butyl acetal in DMF gave 3-dimethylamino-methylene-1,5-diazainden-2-one (as described for the preparation of 3-dimethylamino-methylene-1,6-diazainden-2-one): ¹H NMR (DMSO-d₆): δ 3.35 (s, 6H), 7.13 (d, J = 6.1 Hz, 1H), 8.09 (s, 1H), 8.20 (d, J = 6.1 Hz, 1H), 8.57 (s, 1H), 11.50 (s, 1H); C₁₀H₁₁N₃O: APES+ MS: *m*/*z* 190 (M+H).

### b) 4-{[(2-Oxo-1,2-dihydro-3H-pyrrolo[3,2-c]pyridin-3-ylidene)methyl]amino}-benzenesulfonamide (mixture of E and Z isomers):

3-Dimethylamino-methylene-1,5-diazainden-2(3H)-one was reacted with sulfanilamide in ethanol with hydrochloric acid to give the title compound (by the method described for Example 2, section f) as a 15:4 mixture of *Z:E* isomers. ¹H NMR (DMSO-d₆, peak areas normalized using the overlapping peak at δ 8.45 as 1H): δ 7.37 (m, 3H), 7.72 (d, J = 8.8 Hz, 2H), 7.86 (d, J = 8.8 Hz, 2H), 8.18 *E* (d, J = 14.2 Hz, 0.21H), 8.45 (m, 1H), 8.96 *Z* (s, 0.79H), 9.14 *Z* (d, J = 13.5 Hz, 0.79H), 9.48 *E* (s, 0.21H), 10.7 *E* (m, 0.21H), 10.98 *Z* (d, J = 13.5 Hz, 0.79H), 11.88 *E* (s, 0.21H), 12.11 *Z* (s, 0.79H), 14.7 (br s, 1H); C₁₄H₁₂N₄O₃S: APES+MS *m*/*z* 317 (M+H).

### Example 2: 4-{[(2-Oxo-4,2-dihydro-3H-pyrrolo[2,3-c]pyridin-3-ylidene)methyl] amino}benzenesulfonamide

### a) 3-Nitro-4-pyridinyl-propanedioic acid diethyl ester:

To a suspension of 3.05 g of 60% sodium hydride (76.0 mmol) in toluene (50 mL) in a 250 mL RB flask was added 12.2g of diethyl malonate (76.0 mmol) dropwise. The reaction mixture was stirred for 30 min under nitrogen, and a solution of 10.8 g of 4-chloro-3-nitropyridine (prepared according to the procedure of Houston et al. J. Med. Chem. **1985**, *28*, 467) in toluene (50 mL) was added dropwise and the resulting mixture refluxed for 4 h. The reaction mixture was concentrate, and the residue was partitioned between 100 mL each of dilute hydrochloric acid and diethyl ether. The aqueous phase was extracted twice with 100 mL of ether, and the combined ether phases were dried over magnesium sulfate and concentrated to give a dark oil. This was chromatographed on silica gel eluting with a hexane-30% hexane/EtOAc gradient to give 3.5 g of the title compound as a colorless oil which crystallized on standing. ¹H NMR (DMSO-d₆): δ 1.15 (t, J = 7.1 Hz, 6H), 4.16 (q, J = 7.1 Hz, 4H), 5.55 (s, 1H), 7.59 (d, J = 5.1 Hz, 1H), 8.89 (d, J = 5.1 Hz, 1H), 9.24 (s, 1H); C₁₂H₁₄N₂O₆: APES-MS *m*/*z* 281 (M-H).

### b) 3-Nitro-4-pyridineacetic acid ethyl ester

To a 100 ml RB flask was added 1.5 g (5.3 mmol) of 3-nitro-4-pyridinylpropanedioic add diethyl ester, 0.450 g (10.6 mmol) of lithium chloride, 0.095 g (5.3 mmol) of water and 35 mL of DMSO. The solution was heated at 100°C for 4h. The cooled reaction mixture was diluted with ethyl acetate (50 mL) and washed successively with water (50 mL) and brine (50 mL). The organic phase was dried over sodium sulfate, filtered and concentrated, and the residue was chromatographed on silica gel eluting with a hexane-10% EtOAc/hexane gradient to give 0.76 g of the title compound as a yellow solid. ¹H NMR (DMSO-d6): δ 1.14 (t, J = 7.1 Hz, 3H), 4.06 (q, J = 7.1 Hz, 2H), 4.12 (s, 2H), 7.63 (d, J = 4.9 Hz, 1H), 8.82 (d, J = 4.9 Hz, 1H), 9.21 (s, 1H); C₉H₁₀N₂O₄: APES+MS *m*/*z* 211 (M+H).

### c) 3-Amino-4-pyridineacetic acid ethyl ester:

To a 250-ml Parr flask was added 0.30 g (1.4 mmol) of 3-nitro-4-pyridineacetic acid ethyl ester, 50 mg of 10% palladium on charcoal and 100 mL of ethanol . The mixture was subjected to hydrogenation using a Parr hydrogenator at 40 PSI for 15 min. The mixture was filtered through celite, and the filtrate was concentrated to give an oil which crystallised under a high vacuum to afford 0.282 g of the title compound as a yellow solid. ¹H NMR (DMSO-d₆): δ 1.15 (t, J = 7.2 Hz, 3H), 3.52 (s, 2H), 4.05 (q, J = 7.2 Hz, 2H), 5.11 (s, 2H), 6.89 (d, J = 4.8 Hz, 1H), 7.67 (d, J = 4.8 Hz, 1H), 7.92 (s, 1H).

### d) 1,6-diazainden-2(3H)-one hydrochloride:

To a 50-ml round-bottom flask was added 0.36 g (2.0 mmol) of 3-amino-4-pyridineacetic acid ethyl ester, 15 mL of ether and 10 mL of 10% hydrochloric acid . The resulting biphasic solution was stirred for 16 h at room temperature. The two phases were separated, and the ether phase was washed with 5 mL of water. The combined aqueous phases were evaporated to dryness to yield 0.285 g of the title compound as a brown solid. ¹H NMR (DMSO-d₆): δ 5.93 (s, 1H), 7.59 (d, J = 5.6 Hz, 1H), 8.02 (s, 1H), 8.49 (d, J = 5.6 Hz, 1H), 12.54 (s, 1H), 14.0 (bs, 1H); C₈H₆N₂O: APCI+ MS: *m*/*z* 135 (M+H).

### e) 3-Dimethylamino-methylene-1,6-diazainden-2(3H)-one:

To a suspension of 0.080 g (0.47 mmol) of 1,6-diazainden-2(3H)-one in 10 mL of DMF was added 1.2 g (6.0 mmol) of N,N-dimethylformamide-di-t-butyl acetal. The mixture was stirred at ambient temperature for 2 h, and a dark oil deposited. The DMF was removed under high vacuum, and the residue was passed through a silica gel pad, eluting with ethyl acetate:methanol (1:1). The yellow fractions were pooled, and removal of solvent in vacuo left a brown solid (0.12 g): ¹H NMR (DMSO-d₆): δ 3.3 (s, 6H), 7.35 (d, J = 5.0, 1H), 7.80 (bs, 1H), 7.91 (s, 1H), 7.95 (d, J = 5.0, 1H), 10.32 (bs,1H); C₁₀H₁₁N₃O: APES+ MS: *m*/*z* 190 (M+H).

### f) 4-{[(2-Oxo-1,2-dihydro-3H-pyrrolo[2,3-c]pyridin-3-ylidene)methyl]amino}-benzenesulfonamide:

To a 25-ml round-bottom flask was added 0.12 g (0.47 mmol) of 3-dimethylaminomethylene-1,6-diazainden-2(3H)-one, 0.081 g (0.47 mmol) of sulfanilamide, 10 ml of ethanol and two drops of concentrated hydrochloric acid. The reaction mixture was refluxed using an oil bath with stirring for 3 h, then cooled and filtered. The collected solid was dissolved in a minimum volume of hot methanol. Upon cooling, a dark material deposited. The methanol solution was decanted and diluted to twice its volume with ethyl acetate. A light brown solid deposited after standing for 48 h. The solid was isolated by filtration, dried, and redissolved in hot methanol (30 mL). The solution was concentrated to 20 mL and diluted with an equal volume of ethyl acetate. On cooling, a tan solid formed. The solid was isolated by filtration and washed with methanol/ethyl acetate to give 25 mg (17%) of the title compound. ¹H NMR (DMSO-d₆): δ 7.42 (s, 2H), 7.82 (d, J = 8.5 Hz, 2H), 7.90 (d, J = 8.5 Hz, 2H), 8.28 (s, 1H), 8.11 (d, J = 6.0 Hz, 1H), 8.38 (d, J = 6.0 Hz, 1H), 9.35 (d, J = 13.5 Hz, 1H), 11.53 (s, 1H), 11.60 (d, J = 13.5 Hz, 1H), 14.6 (br s, 1H); C₁₄H₁₂N₄O₃S: APCl-MS *m*/*z* 315 (M-H).

### Example 3: 4-{[(2-Oxo-1,2-dihydro-3H-pyrrolo[2,3-b]pyridin-3-ylidene)methyl] amino}benzenesulfonamide

Dimethylformamide di-tert-butyl acetal (180 mg, 0.89 mmol) was added to a solution of 1,2-dihydro-3*H*-pyrrolo[2,3-b]pyridin-2-one (70 mg, 0.52 mmol) in 0.25 ml DMF, and the reaction mixture was slowly warmed to 100 °C. The cooled solution was then diluted with 5 ml of ethanol. Sulfanilamide (172 mg, 1.00 mmol) and methanesulfonic acid (60 mg, 0.63 mmol) were added, and the reaction mixture was stirred at reflux for 2 h. The cooled solution was diluted with 4 ml of water, treated with NaHCO₃ (70 mg, 0.83 mmol) and stirred 10 min. The resulting solid was filtered, washed with water and ethanol, and then suspended in boiling methanol and filtered upon cooling. Inorganics were removed by filtration through a short silica gel column, eluting with DMF. The resulting solution was diluted with an equal volume of ice water, and the suspension was refrigerated overnight. The solid was isolated by filtration and dried to give 36 mg (21%) of the title compound as a yellow solid: ¹H NMR (400 MHz, DMSO-d₆) (4:1 ratio of Z:E isomers): δ (Z) 11.07 (s, 1H), 10.76 (d, J = 12.4 Hz, 1H), 8.67 (d, J = 12.5 Hz, 1H), 7.92 (d, J = 5.1 Hz, 1H), 7.84 (d, J = 7.3 Hz, 1H), 7.77 (d, J = 8.7 Hz, 2H), 7.55 (d, J = 8.6 Hz, 2H), 7.25 (s, 2H), 6.93 (dd, J = 7.3, 5.1 Hz, 1H); (E) 10.79 (s, 1H), 9.70 (d, J = 13.4 Hz, 1H), 8.23 (d, J = 7.3 Hz, 1H). ESI-MS m/z 315 (M-H). Anal. Calcd. for C₁₄H₁₂N₄O₃S • 0.5 H₂O: C, 51.68; H, 4.03; N, 17.03. Found: C, 51.75; H, 3.95; N, 17.26.

### Compounds Prepared Via Solution Phase Library Techniques (Parallel Synthesis)

We now set forth a selected number of synthesis examples that illustrate the solution library techniques that can be used to obtain the compounds of the invention. It is believed that one of ordinary skill in the art will, in view of the synthesis scheme set forth below (Scheme 7), be able to follow this procedure or modify it accordingly without undue experimentation in order to obtain any of the substitutions disclosed above. The following examples are illustrative examples of the solution phase synthesis, not intended to limit the scope of the invention in any way. 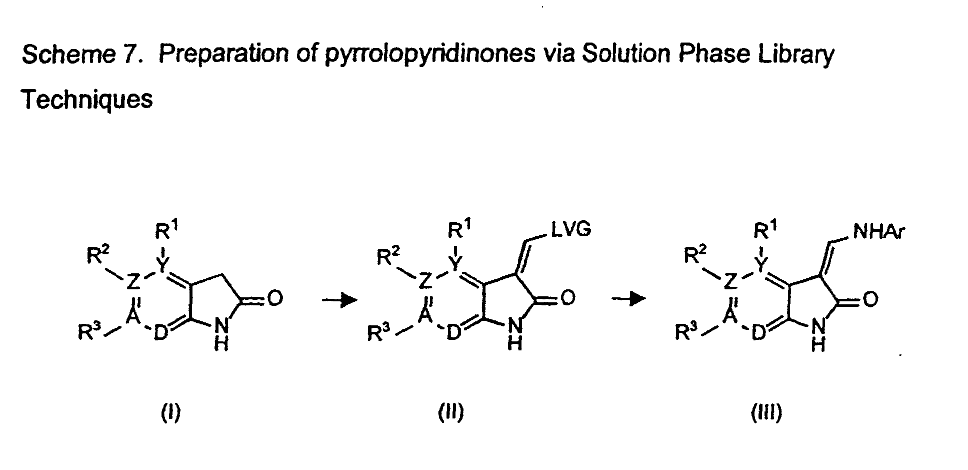 wherein R¹, R², R³, Y, Z, A, D and Ar are as defined hereinabove, and wherein LVG is a leaving group selected from the group including: OCH₃, OCH₂CH₃, OH, N(CH₃)₂.

### Synthesis of Intermediates:

### 5-Bromo-1,3-dihydro-2H-pyrrolo[2,3-b]pyridin-2- one

### a) 3,3,5-Tribromo-1,3-dihydro-2H-pyrrolo[2,3-b]pyridin-2-one

A solution of 3,3-dibromo-1,3-dihydro-2*H*-pyrrolo[2,3-*b*]pyridin-2-one (5.0 g, 13.4 mmol) in tert-BuOH (100 mL) and water (100 mL) was stirred at room temperature and bromine (5.5 g, 34.3 mmol) was added dropwise over 20 min. A saturated aqueous solution of sodium bicarbonate (approx. 15 mL) was added dropwise over 30 min to raise the pH of the solution to 6.5. The yellow solid formed was collected by filtration. The filtrate was condensed to approx. 100 mL and extracted with CHCl₃ (2 x 50 mL). The combined organic extracts were dried over anhydrous magnesium sulfate, and the solvent was evaporated under reduced pressure to leave a yellow solid. The solids were combined and dried under vacuum to give 3,3,5-tribromo-1,3-dihydro-2*H*-pyrrolo[2,3-*b*]pyridin-2-one as a yellow solid, 6.25 g (98%). ¹H NMR (CDCl₃) δ 9.4 (br s, 1H), 8.28 (d, 1H, J = 2 Hz), 7.95 (d, 1H, J = 2 Hz).

### b) 5-Bromo-1,3-dihydro-2H-pyrrolo[2,3-b]pyridin-2-one

A solution of 3,3,5-tribromo-1,3-dihydro-2*H*-pyrrolo[2,3-*b*]pyridin-2-one (5.0 g, 13.4 mmol) in fresh THF (100 mL) was stirred at room temperature, and a saturated aqueous solution of ammonium chloride (100 mL) was added. The flask was placed in a water bath, and activated zinc dust (15.0 g, 230 mmol) was added. The mixture was stirred for 20 min, and the zinc was removed by filtration through a pad of diatomaceous earth. The organic layer was separated, and the aqueous layer was extracted with THF (20 mL). The combined organic layers were washed with saturated brine solution and dried over anhydrous magnesium sulfate. Solvent was removed under reduced pressure. The brown residue was triturated with water (20 mL), and the tan solid was collected by filtration and dried under vacuum to give the title compound as a tan solid, 2.02 g (71%). ¹H NMR (d₆-DMSO)δ 11.13 (s, 1H), 8.15 (s, 1H), 8.76 (s, 1H), 3.57 (s, 2H). MS (AP -ve) 211 (100)(M-H).

### 5-Phenyl-1,3-dihydro-2H-pyrrolo[2,3-b]pyridin-2-one

To a stirred mixture of 5-bromo-1,3-dihydro-2*H*-pyrrolo[2,3-*b*]pyridin-2-one (213 mg, 1 mmol) and phenylboronic acid (183 mg, 1.5 mmol) in toluene (6 ml) and ethanol (6 ml) were added 1 M sodium carbonate solution (2.5 ml, 2.5 mmol), lithium chloride (127 mg, 3 mmol) and dichlorobis(triphenylphosphine)-palladium(II) (35 mg, 0.05 mmol) under N₂ atmosphere. The reaction mixture was heated to reflux at 95 °C for 18 hours. The reaction mixture was diluted with chloroform (50 ml) and washed with brine (20 ml). The aqueous layer was thoroughly extracted with chloroform. The combined organic layers were dried over anhydrous MgSO₄, filtered and evaporated under vacuum to give crude product. Trituation of the crude product with diethyl ether yielded 5-phenyl-1,3-dihydro-2*H*-pyrrolo[2,3-*b*]pyridin-2-one as a yellow solid (108 mg, 51.4%). ¹H NMR (d₆-DMSO): δ 11.04 (s, 1H), 8.32 (s, 1H), 7.83 (s, 1H), 7.60 (d, 2H, *J* = 7.4 Hz), 7.44 (t, 2H, *J* = 7.4 Hz), 7.32 (t, 1H, *J* = 7.4 Hz), 3.58 (s, 2H). MS (-ve APCI): 210 (48, M⁺), 209 (100, M-H).

### 5-(2-Furyl)-1,3-dihydro-2H-pyrrolo[2,3-b]pyridin-2-one

5-Bromo-1,3-dihydro-2*H*-pyrrolo[2,3-*b*]pyridin-2-one (0.75 g, 3.52 mmol), 2-tributyltinfuran (1.26 g, 3.52 mmol), tetraethylammonium chloride hydrate (1.94 g, 10.6 mmol) were combined and dissolved in acetonitrile (10 mL) at room temperature under an atmosphere of nitrogen. Bistriphenylphosphine dichloropalladium (II) (0.25 g, 0.35 mmol) was added, and the reaction was warmed to 85 °C for 16 h. The reaction was cooled to room temperature and diluted with aqueous KF (10%, 60 mL). This was stirred for 20 minutes and then diluted with EtOAc (60 mL). The biphasic system was passed through celite, the layers separated, and the volatiles removed in vacuo. The resulting residue was triturated with diethyl ether, and the solids were collected by filtration to afford the title compound as a light yelow solid (0.28 g, 36% yield). ¹H NMR 300 MHz (DMSO-d₆): δ 11.18 (bs, 1H); 8.45 (s, 1H); 7.92 (s, 1H); 7.79 (s, 1H); 6.95 (d, 1H); 6.60 (d, 1H); 3.64 (s, 2H). APCl m/z 201 (M+1).

### 5-(3-thienyl)-1,3-dihydro-2H-pyrrolo[2,3-b]pyridin-2-one

5-Bromo-1,3-dihydro-2H-pyrrolo[2,3-b]pyridin-2-one (0.20 g, 0.94 mmol), 3-tributyltinthiophene (0.42 g, 1.12 mmol), tetraethylammonium chloride hydrate (0.16 g, 0.94 mmol) were combined and dissolved in acetonitrile (10 mL) at room temperature under nitrogen. Bistriphenylphosphine dichloropalladium (II) (0.033 g, 0.047 mmol) was added, and the reaction was warmed to 85 °C for 20 h. Fresh catalyst (bistriphenylphosphine dichloropalladium (II), 0.033 g, 0.047 mmol) was added to the reaction mixture, and the reaction was stirred at 85 °C for 24 h. The reaction was cooled to room temperature and diluted with water (20 mL) and EtOAc (20 ml). The biphasic system was passed through celite, and the layers were separated. The organic layer was washed with brine (10 mL) and dried over sodium sulfate. The volatiles were removed in vacuo. The resulting residue was triturated with diethyl ether and collected by filtration to provide the title compound (0.16 g, 80% yield). ¹H NMR 400 MHz (DMSO-d₆) δ 11.03 (bs, 1H); 8.43 (s, 1H); 7.92 (s, 1H); 7.84 (s, 1H); 7.60 (m, 1H); 7.53 (d, 1H); 3.58 (s, 2H).

### Ethyl 2-oxo-2,3-dihydro-1H-pyrrolo[2,3-b]pyridine-5-carboxylate

To a mixture of 5-bromo-1,3-dihydro-2*H*-pyrrolo[2,3-*b*]pyridin-2-one (213 mg, 1 mmol) in dimethylsulfoxide (1 ml) and ethanol (5 ml) in Parr bomb were added triethylamine (0.31 ml, 2.25 mmol), palladium acetate (33.7 mg, 0.15 mmol), and 1,4-(bisdiphenylphosphino)propane (61.9 mg, 0.15 mmol). Carbon monoxide gas (40 atm) was applied and the reaction mixture was heated at 95 °C for 18 hours with vigorous stirring. The reaction mixture was diluted with diethyl ether (50 ml) and washed with water (10 ml). The aqueous layer was thoroughly extracted with diethyl ether. The combined organic layers were dried over anhydrous MgSO₄, filtered and evaporated under vacuum to give crude product. Trituration of the crude product with methanol provided the title compound as a tan solid (53 mg, 26 %). ¹H NMR (d₆-DMSO): δ 11.39 (s, 1H), 8.62 (s, 1H), 7.95 (s, 1H), 4.27 (q, 2H, *J* = 7 Hz), 3.59 (s, 2H), 1.28 (t, 3H, *J =* 7 Hz). MS (-ve APCI): 205 (4, M-H).

### 1,3-Dihydro-2H-pyrrolo[2,3-b]pyridin-2-one

### a) 3,3-dibromo-1,3-dihydro-2H-pyrrolo[2,3-b]pyridin-2-one

A solution of pyrrolo[2,3-b]pyridine (4.0g, 34 mmol) in tert-BuOH (200 mL) was stirred at room temperature and pyridinium perbromide (32.5g, 0.1 mol) was added in portions over 30 min, and the reaction mixture was stirred for 3 h. Pyridinium perbromide (10.8 g, 33 mmol) was added, and the mixture was stirred for a further 2 h. The tert-BuOH was evaporated under reduced pressure, and the residue was partitioned between water (300 mL) and EtOAc (300 mL). The organic layer was separated, and the aqueous layer was extracted with EtOAc. The combined organic layers were washed with water (2 x 50 mL) and brine. The organic layer was dried over anhydrous MgSO₄ and filtered, and the solvent was evaporated. Trituration of the residue with CH₂Cl₂ gave a white solid which was collected by filtration and dried under vacuum to provide 8.35 g of the title compound. ¹H NMR (d₆-DMSO) δ 11.99 (s, 1H), 8.21 (dd, 1H, J = 5.1, 1.5 Hz), 8.00 (dd, 1H, J = 7.5, 1.5 Hz), 7.17 (dd, 1H, J = 7.5, 5.1 Hz). MS (+ve ES) 293 (28), (M+H) 147 (100).

### b) 1,3-Dihydro-2H-pyrrolo[2,3-b]pyridin-2-one

A solution of 3,3-dibromo-1,3-dihydro-2*H*-pyrrolo[2,3-*b*]pyridin-2-one (2.0 g, 7.2 mmol) in THF (50 mL) was stirred at room temperature, and a saturated aqueous solution of NH₄Cl was added. Activated zinc powder was added, and the reaction mixture was stirred for 2 h. The zinc was removed by filtration through a pad of diatomaceous earth, and the organic layer was separated. The aqueous layer was extracted with THF (10 mL), and the combined organic layers were dried over anhydrous MgSO₄, filtered and evaporated. The residue was slurried in 10:1 CHCl₃:MeOH (15 mL) and filtered through a pad of silica gel, and the filtrate was evaporated. The residue was triturated with water, and the solid was collected by filtration and dried under vacuum to give the title compound, 0.668g (70%). ¹H NMR (d₆-DMSO) δ 10.94 (s, 1H), 8.02 (d, 1H, J = 5.2 Hz), 7.52 (d, 1H, J = 6.8 Hz), 6.90 (dd, 1H, J = 6.8, 5.2 Hz), 3.53 (s, 2H). MS (AP-ve) 133 (100) (M-H)

### 1,3-Dihydro-2H-pyrrolo[3,2-b]pyridin-2-one

### a) Diethyl (3-nitropyridin-2-yl)-malonate

Sodium hydride (60% dispersion in oil, 5.57 g, 0.14 mol) was carefully washed with hexanes under nitrogen before the addition of DMSO (115 mL). Diethyl malonate (22.3 g, 0.14 mol) was added dropwise over 20 min, and the mixture was stirred for an additional 30 min at room temperature. 2-Chloro-3-nitropyridine (10 g, 0.06 mol) was added to the reaction, and the reaction was placed in a pre-heated oil bath set to 100°C for 15 min. The reaction was cooled to room temperature and poured into aqueous ammonium chloride (saturated solution, 150 mL). The aqueous solution was extracted with EtOAc:hexanes (1:1) four times (200 mL each), and the organic layers were combined. The organics were concentrated to afford a solid that was recrystallized from a minimal amount of EtOAc:hexanes (1:1) to provide the title compound (12.5 g, 70% yield). APCI MS *m*/*z* 281 (M-H).

### b) Ethyl 2-(3-nitro-pyridin-2-yl)-acetate

Diethyl (3-nitropyridin-2-yl)-malonate (12.5 g, 0.044 mol) was dissolved in DMSO (150 mL), and water (0.79 mL, 0.044 mol) and lithium chloride (4.65 g, 0.11 mol) were added at room temperature under nitrogen. The reaction was warmed to 100°C for 12 h, and more lithium chloride (1 g) was added to the reaction. The reaction was heated for another 5 hours and cooled to room temperature. Brine (150 mL) was added, and the reaction mixture was extracted with EtOAc (3 x 275 mL). The extracts were combined, dried over sodium sulfate and concentrated in vacuo. The resulting residue was triturated with diethyl ether and collected by filtration to yield the title compound (8.6 g, 92 % yield). ¹H NMR 400 MHz (DMSO-d₆): δ 8.83 (m, 1H); 8.53 (m, 1H); 7.65 (m, 1H); 4.23 (s, 2H); 4.07 (m, 2H); 1.16 (m, 3H).

### c) Ethyl 2-(3-amino-pyridin-2-yl)-acetate

Under an atmosphere of nitrogen, Pd/C (10%, 1.36 g) was added to a round bottom flask. Ethyl 2-(3-nitro-pyridin-2-yl)-acetate (8.6 g, 0.41 mol) was dissolved in ethanol (200 mL) and added to the reaction vessel. The reaction was placed under an atmosphere of hydrogen and stirred at room temperature for 30 min. The reaction was filtered through celite, and the filtrate was concentrated in vacuo to afford the title compound as a tan solid (6.94g, 94% yield).

### d) 1,3-Dihydro-2H-pyrrolo[3,2-b]pyridin-2-one

Ethyl 2-(3-amino-pyridin-2-yl)-acetate (6.94 g, 0.038 mol) was dissolved in diethyl ether (100 mL) at room temperature. Hydrochloric acid (2M, 35 mL) was added, and the reaction was stirred for 30 minutes. The volatiles were removed to afford a brown solid that was recrystallized from ethanol and diethyl ether to provide the title compound (4.0 g, 62% yield). ¹H NMR 400 MHz (DMSO-d₆): δ 12.35 (s, 1H); 8.12 (m, 1H); 7.90 (m, 1H); 7.14 (m, 1H); 5.75 (s, 2H). Electrospray MS *m*/*z* 135 (M+H).

### 6-Chloro-1,3-dihydro-2H-pyrrolo[2,3-b]pyridin-2-one

### a) 3,3-Dibromo-6-chloro-1,3-dihydro-2H-pyrrolo[2,3-b]pyridin-2-one

To a stirred solution of 1.32 g (8.7 mmol) of 6-chloro-1*H*-pyrrolo[2,3-*b*]pyridine (Minakata et al., Synthesis **1992**, 661-663) in tert-butanol (80 mL) was added 9.9 g (28 mmol) of 90% pyridine hydrobromide perbromide, resulting in immediate formation of a thick yellow precipitate. The reaction was concentrated, and the crude residue was chromatographed on silica gel, eluting with a hexane to 90% hexane/10% EtOAc gradient, to give 2.36 g of the title compound as a white solid [¹H NMR (CDCl₃): δ 7.16 (d, J = 8.0 Hz, 1H), 7.81 (d, J = 8.0 Hz, 1H), 9.0 (bs, 1H)] containing about 30% of 3,3,5-tribromo-6-chloro-1,3-dihydro-2*H*-pyrrolo[2,3-*b*]pyridin-2-one as an inseparable impurity [¹H NMR δ 8.05 (s, 1H), 9.0 (bs, 1H)].

### b) 6-Chloro-1,3-dihydro-2H-pyrrolo[2,3-b]pyridin-2-one

A solution of 2.36 g (7.26 mmol) of the mixture of 3,3-dibromo-6-chloro-1,3-dihydro-2*H*-pyrrolo[2,3-*b*]pyridin-2-one and 3,3,5-tribromo-6-chloro-1,3-dihydro-2*H*-pyrrolo[2,3-*b*]pyridin-2-one in THF (70 mL) and saturated ammonium chloride solution (70 mL) was treated with 6 g (92 mmol) of powdered zinc. The mixture was stirred for 2 h, and another 6 g (92 mmol) portion of zinc was added. Stirring was continued another 2 h. The zinc was filtered off and washed with ether. The ether phase was separated, and the aqueous phase was extracted twice with a 1:1 mixture of THF/ether. The combined organic extracts were dried over magnesium sulfate, filtered and concentrated. The crude residue was loaded onto 7.5 g of silica gel and chromatographed on silica gel, eluting with a 90% hexane/10% ethyl acetate to 66% hexane/ 33% ethyl acetate gradient to give 0.647 g of the title compound. ¹H NMR (DMSO-d₆): δ 3.57 (s, 2H), 7.04 (d, J = 7.6 Hz, 1H), 7.61 (d, J = 7.6 Hz, 1H), 11.2 (bs, 1H).

### Synthesis of Monomers:

### Ethyl 3-[(Z)-ethoxymethylidene]-2-oxo-1,2-dihydro-3H-pyrrolo[2,3-b]pyridine-5-carboxylate

### (Procedure A)

Ethyl 2-oxo-2,3-dihydro-1*H*-pyrrolo[2,3-*b*]pyridine-5-carboxylate (0.040 g, 0.19 mmol) and diethoxymethyl acetate (0.16 mL, 0.97 mmol) were combined and dissolved in acetic acid (1 mL). The reaction was warmed to 110 °C and stirred at this temperature for 1 h. The reaction was cooled to room temperature, and diethyl ether was added to precipitate the title compound as a beige solid that was collected by filtration (35 mg, 69% yield). ¹H NMR 400 MHz (DMSO-d₆): δ 11.30 (s, 1H); 8.58 (s, 1H); 8.05 (s, 1H); 7.93 (s, 1H); 4.44 (m, 2H); 4.28 (m, 2H); 1.35 (m, 3H); 1.28 (m, 3H).

### 3-[Ethoxymethylidene]-5-phenyl-1H-pyrrolo[2,3-b]pyridin-2-one

This compound was synthesized from 5-phenyl-1,3-dihydro-2*H*-pyrrolo[2,3-*b*]pyridin-2-one and diethoxymethyl acetate according to Procedure A. ¹H NMR 400 MHz (DMSO-d₆): δ 10.93 (s, 1H); 8.24 (s, 1H); 7.86-7.82 (m, 2H); 7.62-7.56 (m, 2H); 7.44 (m, 2H); 7.34 (m, 1H); 4.40 (m, 2H); 1.35 (m, 3H).

### 3-[Ethoxymethylidene]-5-(2-furyl)-1H-pyrrolo[2,3-b]pyridin-2-one

This compound was synthesized from 5-(2-furyl)-1,3-dihydro-2*H*-pyrrolo[2,3-*b*]pyridin-2-one and diethoxymethyl acetate according to Procedure A. ¹H NMR 400 MHz (DMSO-d₆): δ 10.96 (s, 1H); 8.36 (s, 1H); 7.87-7.84 (m, 2H); 7.72 (s, 1H); 6.87 (d, 1H); 6.56 (m, 1H); 4.42 (m, 2H); 1.36 (m, 3H).

### 3-[Ethoxymethylidene]-5-(3-thienyl)-1H-pyrrolo[2,3-b]pyridin-2-one

This compound was synthesized from 5-(3-thienyl)-1*H*-pyrrolo[2,3-*b*]pyridin-2-one and diethoxymethyl acetate according to Procedure A. ¹H NMR 400 MHz (DMSO-d₆): δ 10.89 (s, 1H); 8.33 (s, 1H); 7.88 (s, 1H); 7.84 (s, 1H); 7.79 (s, 1H); 7.62 (m, 1H); 7.49 (d, 1H); 4.40 (m, 2H); 1.36 (m, 3H).

### 5-Bromo-3-[ethoxymethylidene]-1H-pyrrolo[2,3-b]pyridin-2-one

This compound was synthesized from 5-bromo-1*H*-pyrrolo[2,3-*b*]pyridin-2-one and and diethoxymethyl acetate according to Procedure A. ¹H NMR 400 MHz (DMSO-d₆): δ 11.02 (s, 1H); 8.07 (s, 1H); 7.88 (s, 1H); 7.71 (s, 1H); 4.40 (m, 2H); 1.34 (m, 3H).

### 6-Chloro-3-[ethoxymethylidene]-1H-pyrrolo[2,3-b]pyridin-2-one

This compound was synthesized from 6-chloro-1*H*-pyrrolo[2,3-*b*]pyridin-2-one and diethoxymethyl acetate according to Procedure A. ¹H NMR 400 MHz (DMSO-d₆): δ 11.06 (s, 1H); 7.84 (s, 1H); 7.63 (d, 1H); 6.98 (d, 1H); 4.39 (m, 2H); 1.32 (m, 3H).

### 3-[(Dimethyamino)methylidene]-1H-pyrrolo[2,3-b]pyridin-2-one

This compound was prepared in situ (during library synthesis) from 1,3-dihydro-2*H*-pyrrolo[2,3-*b*]pyridin-2-one and dimethylformamide di-t-butylacetal in DMF.

### 3-[(Dimethylamino)methylidene]-1H-pyrrolo[3,2-b]pyridin-2-one

This monomer was generated in situ (during library synthesis) from 1,3-dihydro-2*H*-pyrrolo[3,2-*b*]pyridin-2-one and dimethylformamide di-t-butylacetal in DMF.

### Solution Phase Library Synthesis:

The compounds described here were prepared as part of a larger library of related compounds using the following procedure. Stock solutions (0.037M in ethanol) were prepared for each set of pyrrolopyridinone monomers. For the aniline set (4-aminobenzenesulfonamide, 1*H*-indazol-6-amine, and 6-quinolinamine), a slight excess of stock solution was prepared.

3-[(Dimethylamino)methylidene]-1*H*-pyrrolo[2,3-*b*]pyridin-2-one and 3-[(dimethylamino)methylidene]-1*H*-pyrrolo[3,2-*b*]pyridin-2-one were generated *in situ* by preparing stock solutions of the corresponding pyrrolopyridinone. For example, 20.1 mg of 1,3-dihydro-2*H*-pyrrolo[3,2-*b*]pyridinone was dissolved in 4.0 mL of ethanol. Both of the pyrrolopyridinones were transferred (0.20 ml/well) to a 96-well dry heating block (vwrBRAND Dry Block Heater, cat #13259-066). The ethanol was evaporated off at 50 °C until it was clear that there was no solvent remaining. DMF (0.20 mL) was added followed by the addition of dimethylformamide di-t-butylacetal (0.003 mL), and this remained at room temperature for 1 h.

The ethoxymethylidenepyrrolopyridinones (0.20 mL/well) were transferred to wells in the dry block heater. The aniline set (0.20 mL/well) was transferred to the appropriate wells such that each pyrrolopyridinone was reacted with each aniline. The plates were heated to 70 °C for 4 h, and then the reaction was cooled to 40 °C and heating was continued for another 16 h. Ethanol was added as necessary to keep a constant reaction volume in the wells.

Upon completion of the reaction, methanol (1.0 mL) was added to each well. Using a multi-pipettor, the contents of the reaction wells were transferred to the appropriate wells of a 96-well (Beckmann) plate. The volatiles were removed using a nitrogen flow to substantially reduce the volume of solvent, followed by placing the plates in a vacuum drying oven at 70 °C under 15 mm Hg of pressure. All of the wells were analysed by LC-MS.

### Example 3: 3-[(1H-Indazol-6-ylamino)methylidene]-1H-pyrrolo[3,2-b]pyridin-2 one

High resolution masspec: calc. for C₁₅H₁₁N₅O, 278.1042 (M+H); found, 278.1036.

### Example 4: 3-[(6-Quinolinylamino)methylidene]-1,3-dihydro-2H-pyrrolo[3,2 b]pyridin-2-one

High resolution masspec: calc. for C₁₇H₁₂N₄O, 289.1089 (M+H); found, 289.1085

### Example 5: 4-{[(2-Oxo-1,2-dihydro-3H-pyrrolo[2,3-b]pyridin-3-ylidene)methyl]amino} benzenesulfonamide

Low resolution mass spec: 317 (M+H).

### Example 6: 3-[(1H-Indazol-6-ylamino)methylidene]-1H-pyrrolo[2,3-b]pyridin-2 one

High resolution masspec: calc. for C₁₅H₁₁N₅O, 278.1042 (M+H); found 278.1033.

### Example 7: 3-[(6-Quinolinylamino)methylidene]-1,3-dihydro-2H-pyrrolo[2,3 b]pyridin-2-one

High resolution masspec: calc. for C₁₇H₁₂N₄O, 289.1089 (M+H); found, 289.1079

### Example 8: 4-{[(2-Oxo-5-phenyl-1,2-dihydro-3H-pyrrolo[2,3-b]pyridin-3 ylidene)methyl]amino}benzenesulfonamide

High resolution masspec: calc. for C₂₀H₁₆N₄O₃S, 393.1021 (M+H); found, 393.1003.

### Example 9: 3-[(1H-Indazol-6-ylamino)methylidene]-5-phenyl-1H-pyrrolo[2,3-b]pyridin-2-one

High resolution masspec: calc. for C₂₁H₁₅N₅O, 354.1355 (M+H), found, 354.1336.

### Example 10: 5-Phenyl-3-{(6-qulnolinylamino)methylidene]-1,3-dihydro-2H-pyrrolo[2,3-b]pyridin-2-one

High resolution masspec: calc. for C₂₃H₁₆N₄O, 365.1402 (M+H); found, 365.1396.

### Example 11: 4-({[5-(2-Furyl)-2-oxo-1,2-dihydro-3H-pyrrolo[2,3-b]pyridin-3-ylidene]methyl}amino)benzenesulfonamide

High resolution masspec: calc. for C₁₈H₁₄N₄O₄S, 383.0814 (M+H); found, 383.0800.

### Example 12: 5-(2-Furyl)-3-[(1H-indazol-6-ylamino)methylidene]-1H-pyrrolo[2,3-b]pyridin-2-one

Low resolution masspec: 344 (M+H).

### Example 13: 5-(2-Furyl)-3-[(6-quinolinylamino)methylidene]-1,3-dihydro-2H-pyrrolo[2,3-b]pyridin-2-one

High resolution masspec: calc. for C₂₁H₁₄N₄O₂, 355.1195 (M+H); found, 355.1182.

### Example 14: 4-({[2-Oxo-5-(3-thienyl)-1,2-dihydro-3H-pyrrolo[2,3-b]pyridin-3-ylidene]methyl}amino)benzenesulfonamide

Low resolution masspec: 399 (M+H).

### Example 15: 3-[(1H-Indazol-6-ylamino)methylidene]-5-(3-thienyl)-1,3-dihydro-2H-pyrrolo[2,3-b]pyridin-2-one

High resolution masspec: calc. for C₁₉H₁₃N₅OS, 360.0919 (M+H); found, 360.0903.

### Example 16: 3-[(6-Quinolinylamino)methylidene]-5-(3-thienyl)-1,3-dihydro-2H-pyrrolo[2,3-b]pyridin-2-one

High resolution masspec: calc. for C₂₁H₁₄N₄OS, 371.0966 (M+H); found, 371.0956.

### Example 17: 4-{[(5-Bromo-2-oxo-1,2-dihydro-3H-pyrrolo[2,3-b]pyridin-3-ylidene)methyl]amino}benzenesulfonamide

High resolution masspec: calc. for C₁₄H₁₁BrN₄O₃S, 394.9813 (M+H); found, 394.9792.

### Example 18: 5-Bromo-3-[(1H-indazol-6-ylamino)methylidene]-1,3-dihydro-2H-pyrrolo[2,3-b]pyridin-2-one

High resolution masspec: calc. for C₁₅H₁₀BrN₅O, 356.0146 (M+H); found, 356.0135.

### Example 19: 5-Bromo-3-[(6-quinolinylamino)methylidene]-1,3-dihydro-2H-pyrrolo[2,3-b]pyridin-2-one

High resolution masspec: calc. for C₁₇H₁₁BrN₄O, 367.0194 (M+H); found, 367.0177.

### Example 20: 4-{[(6-Chloro-2-oxo-1,2-dihydro-3H-pyrrolo[2,3-b]pyridin-3-ylidene)methyl]amino}benzenesulfonamide

High resolution masspec: calc. for C₁₄H₁₁ClN₄O₃S, 351.0318 (M+H); found, 351.0315.

### Example 21: 6-Chloro-3-[(1H-indazol-6-ylamino)methylidene]-1,3-dihydro-2H-pyrrolo[2,3-b]pyridin-2-one

High resolution masspec: calc. for C₁₅H₁₀ClN₅O, 312.0652 (M+H); found, 5 312.0628.

### Example 22: 6-Chloro-3-[(6-quinolinylamino)methylidene]-1,3-dihydro-2H-pyrrolo[2,3-b]pyridin-2-one

High resolution masspec: calc. for C₁₇H₁₁ClN₄O, 323.0699 (M+H); found, 323.0697.

### Example 23: Ethyl 3-{[4-(aminosulfonyl)anilino]methylidene}-2-oxo-1,2-dihydro-3H-pyrrolo[2,3-b]pyridine-5-carboxylate

High resolution masspec: calc. for C₁₇H₁₆N₄O₅S, 389.0919 (M+H); found, 389.0914.

### Example 24: Ethyl 3-[(1H-indazol-6-ylamino)methylidene]-2-oxo-1,2-dihydro-3H-pyrrolo[2,3-b]pyridine-5-carboxylate

High resolution masspec: calc. for C₁₈H₁₅N₅O₃, 350.1253 (M+H); found, 350.1241.

### Example 25: Ethyl 2-oxo-3-[(6-quinolinylamino)methylidene]-2,3-dihydro-1H-pyrrolo[2,3-b]pyridine-5-carboxylate

High resolution masspec: calc. for C₂₀H₁₆N₄O₃, 361.1300 (M+H); found, 361.1299.

### PHARMACEUTICAL FORMULATION AND DOSES

The compounds of the present invention can be administered in such oral (including buccal and sublingual) dosage forms as tablets, capsules (each including timed release and sustained release formulations), pills, powders, granules, elixirs, tinctures, suspensions, syrups and emulsions. Likewise, they may also be administered in nasal, ophthalmic, otic, rectal, topical, intravenous (both bolus and infusion), intraperitoneal, intraarticular, subcutaneous or intramuscular inhalation or insufflation form, all using forms well known to those of ordinary skill in the pharmaceutical arts.

The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter or arrest the progress of the condition.

Oral dosages of the present invention, when used for the indicated effects, will range between about 0.1 to 100 mg/kg of body weight per day, and particularly 1 to 10 mg/kg of body weight per day. Oral dosage units will generally be administered in the range of from 1 to about 250 mg and more preferably from about 25 to 250 mg. The daily dosage for a 70 kg mammal will generally be in the range of about 70 mg to 7 grams of a compound of formula I or II.

While the dosage to be administered is based on the usual conditions such as the physical condition of the patient, age, body weight, past medical history, route of administrations, severity of the conditions and the like, it is generally preferred for oral administration to administer to a human. In some cases, a lower dose is sufficient and, in some cases, a higher dose or more doses may be necessary. Topical application similarly may be once or more than once per day depending upon the usual medical considerations. Advantageously, compounds of the present invention may be administered in a single daily dose, or the total daily dosage may be administered in divided doses of two, three or four times daily. The compounds of the invention can be prepared in a range of concentrations for topical use of 0.1 to 5mg/ml of suitable solvent. A preferred volume for application to the scalp is 2 to 20 ml, resulting in an effective dosage delivered to the patient of 0.2 to 100mg. For treatment of chemotherapy-induced alopecia, administration 1 to 2 times prior to chemotherapy administration would be preferred, with additional applications administered as needed. A similar regimen can be pursued for treatment of alopecia induced by radiation therapy. Furthermore, preferred compounds for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

In the methods of the present invention, the compounds herein described in detail can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders indude starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A dear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or saccharin, and the like can also be added.

Where appropriate, dosage unit formulations for oral administration can be microencapsulated. The formulation can also be prepared to prolong or sustain the release as for example by coating or embedding particulate material in polymers, wax or the like.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monodonal antibodies as individual carriers to which the compound molecules are coupled. The compounds of the present invention may also be coupled with soluble polymers as targetable drug carriers. Such 5 polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

The present invention includes pharmaceutical compositions containing 0.01 to 99.5%, more particularly, 0.5 to 90% of a compound of the formula (II) in combination with a pharmaceutically acceptable carrier.

Parenteral administration can be effected by utilizing liquid dosage unit forms such as sterile solutions and suspensions intended for subcutaneous, intramuscular or intravenous injection. These are prepared by suspending or dissolving a measured amount of the compound in a non-toxic liquid vehicle suitable for injection such as aqueous oleaginous medium and sterilizing the suspension or solution.

Altematively, a measured amount of the compound is placed in a vial and the vial and its contents are sterilized and sealed. An accompanying vial or vehicle can be provided for mixing prior to administration. Non-toxic salts and salt solutions can be added to render the injection isotonic. Stabilizers, preservations and emulsifiers can also be added.

Rectal administration can be effected utilizing suppositories in which the compound is admixed with low-melting water-soluble or insoluble solids such as polyethylene glycol, cocoa butter, higher ester as for example flavored aqueous solution, while elixirs are prepared through myristyl palmitate or mixtures thereof.

Topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams. The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

For administration by inhalation the compounds according to the invention are conveniently delivered in the form of an aerosol spray presentation from pressurized packs or a nebulizer, with the use of a suitable propellant, e.g. dichlorodifluoromethane, trichlorofluoromethane, dichlorotetrafluoroethane, tetrafluoroethane, heptafluoropropane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

The preferred pharmaceutical compositions are those in a form suitable for oral administration, such as tablets and liquids and the like and topical formulations.

### BIOLOGICAL DATA

The compounds of the present invention have valuable pharmacologic properties. Different compounds from this class are particularly effective at inhibiting the CDK1 and CDK2 enzymes at concentrations which range from 0.01 to 3 µM and additionally show specificity relative to other kinases. Substrate phosphorylation assays were carried out as follows:

### CDK2

Cyclin dependent protein kinase assays utilized the peptides Biotin-aminohexyl-AAKAKKTPKKAKK and Biotin-aminohexyl-ARRPMSPKKKA-NH₂ as phosphoryl group acceptors. CDK2 was expressed utilizing a baculovirus expression system and was partially purified to comprise 20-80% of total protein, with no detectable competing reactions present. Typically, assays were performed by incubating enzyme (0.2-10 nM), with and without inhibitor, one of the two peptide substrates (1-10 nM), [γ-³²P]ATP (1-20 nM), and 10-20 mM Mg²⁺ for periods of time generally within the range 10-120 min. Reactions were terminated with 0.2-2 volumes of either 20% acetic acid or 50-100 mM EDTA buffered to pH 7 (substrate consumption <20%). The buffer employed in enzyme assays was either 30 mM HEPES 7.4 containing 0.15 M NaCI and 5% DMSO, the buffer 50 mM MOPS 7.0 containing 0.15 M NaCI and 5% DMSO, or the buffer 100 mM HEPES pH 7.5 containing 0.1 mg/mL BSA and 5% DMSO. Inhibitors were diluted in 100% DMSO prior to addition into the assay. Detection of peptide phosphorylation was accomplished by scintillation counting following either collection of peptide onto phosphocellulose filters (for reactions stopped with acetic acid), collection of peptide in wells of 96 well plates coated with Streptavidin (Pierce) (reactions were stopped with EDTA), or addition of Avidin coated Scintillant impregnated beads (Scintillation Proximity Assays from Amersham; reactions were stopped with EDTA). Counts detected by any of these methodologies minus the appropriate background (assays with additional 40 mM EDTA or lacking peptide substrate) were assumed to be proportional to the reaction initial rates, and IC50s were determined by a least squares fit to the equation CPM = Vₘₐₓ*(1-([I]/(K+[I])))+nsb, or pIC50s were determined by a fit to the equation CPM = nsb+(Vₘₐₓ-nsb)/(1+(x/10^{x}-plC50)), where nsb are the background counts.

### VEGFR-2

The peptide substrate used in the VEGFR-2 assay was biotin-aminohexyl-EEEEYFELVAKKKK-NH₂. The kinase domain of the enzyme was purified to homogeneity from a baculovirus expression system. The enzyme was preactivated on ice for 15 min in the presence of 100 µM ATP and 20 mM MgCl₂, and stored at -80°C until needed for assay. The activated enzyme was diluted to 0.4 nM into a 60 µl reaction containing 100 mM HEPES, pH 7.5, 5 µM ATP, 10 mM MgCl₂, 5 µM peptide, 0.1 mM DTT, 0.05 mg/ml BSA, and an inhibitor at varying concentrations. The controls were reactions in the presence (negative controls) or absence (positive controls) of 50 mM EDTA. Reactions were incubated for 30 min at room temperature, and then quenched by the addition of EDTA to 60 mM in 210 µl. The quenched samples (190 µl) were transferred to a neutravidin-coated plate (Pierce) and incubated at room temperature for 40 min to allow biotinylated peptide to bind to the neutravidin. The unbound components of the reaction were removed by washing with a plate washer, then 200 µl HRP-PY20 anti-phosphotyrosine antibody conjugate was added to each well. After incubation for 40 min, the plate was washed to remove any unbound anitbody. A HRP substrate, K-blue (Neogen) was added and the reaction was quenched with Red Stop (Neogen) after 20 min. The absorbance of the wells was read at A₆₅₀ in a plate reader. IC₅₀ values were obtained by fitting raw data to A₆₅₀ = V_{MAX} *(1-[I]/IC₅₀ + [I]))) + b, where b is background.

### c-fms

c-fms protein kinase assays utilized the peptide substrate, biotin-EAIYAAPFAKKK-NH_{2,} as the phosphoryl group acceptor. The c-fms intracellular domain was expressed from a baculovirus expression system, as an amino-terminal GST fusion protein, and purified to homogeneity using Glutathione agarose from Sigma Chemical Co.. Maximum activation of the enzyme was achieved by preactivation at room temperature for 120 min in the presence of 100 µM ATP and 15 mM MgCl₂, This enzyme stock was diluted to 150 nM prior to using in the assay. Typically assays were performed in white, opaque, 96-well plates in a 45ul assay volume including 15ul 6% DMSO, with or without compounds , 15ul of the preactivated, diluted enzyme, and 15ul of a substrate mixture. Reactions contained 50 mM HEPES, pH 7.5, 1.7 µM ATP, 15 mM MgCl₂, 3 µM peptide, 2.5 mM DTT, 50mM NaCI and 0.15uCi/assay [□□³²P]ATP. The controls were reactions in the presence (negative controls) or absence (positive controls) of 50 mM EDTA. Reactions were allowed to proceed for 90 min at room temperature. The reaction products were quantified using Scintillation Proximity technolgy. The reactions were quenched by the addition of 200ul of a solution containing 0.3mg streptavidin SPA beads from Amersham, 50 mM EDTA, 0.1% TX-100, 50uM ATP, in PBS, pH7.2 (phosphate buffered solution). Plates were sealed and counted in a Packard Topcount scintillation counter. IC50 values were obtained by fitting raw data to the equation y=Vmax*(1-(x/(k+x))).

The results shown in Table 3 summarise representative data: Table 3 illustrates the inhibitory activity of compounds of the present invention against three different kinases (CDK2, c-fms, and VEGFR2).

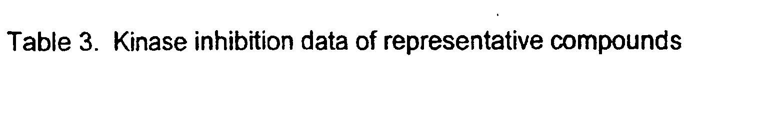

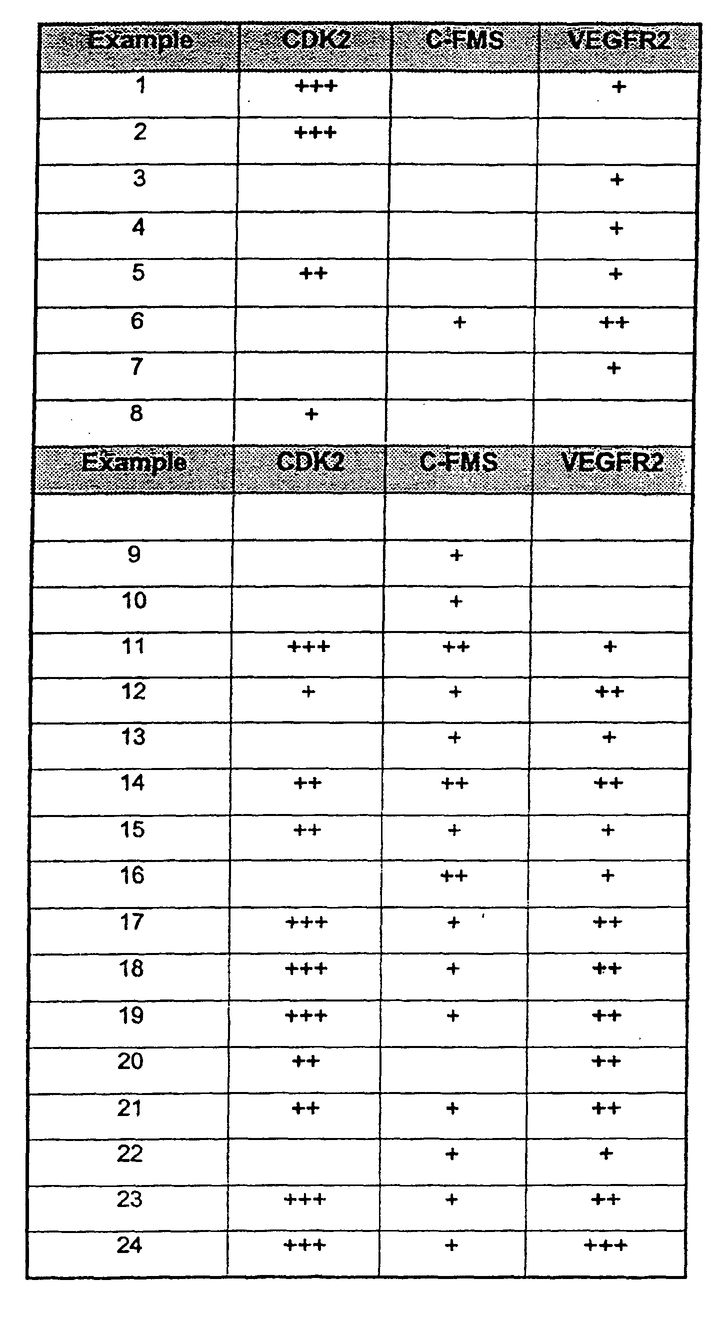

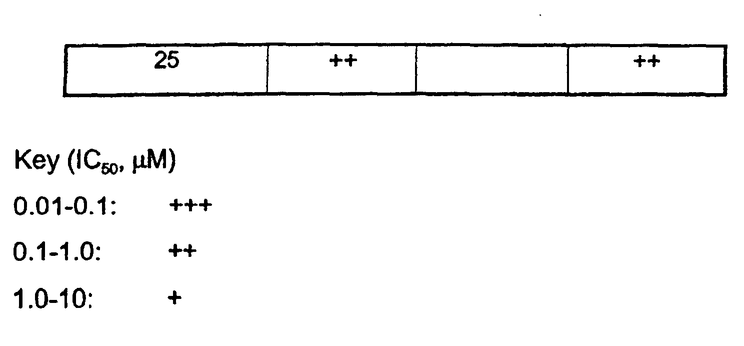

### UTILITY OF INVENTION

Inhibitors of members of the CDK family of kinases find utility as agents in the treatment of a wide variety of disorders which have a proliferative component or which involve regulation of cyclin dependent kinase function. These include cancers, restenosis, psoriasis, and actinic keratosis.

The present invention demonstrates methodologies by which the onset of cell death in normal proliferating cells induced by chemotherapeutic drugs may be prevented by the prior treatment with inhibitors of cyclin dependent kinases. This may be useful to decrease the severity of chemotherapy -induced side effects due to killing of normal cells. These side effects may include, but are not limited to alopecia, mucocitis (nausea and vomiting, diahrea, oral lesions), neutropenia and thrombocytopenia. Inhibitors of cyclin dependent kinases CDK2 and CDK4 prevent the progression of normal cells into both S-phase (DNA synthesis) or M-phase (mitosis), reducing their susceptibility to incur damage by certain chemotherapeutic drugs which act in those phases of the cell cycle. When the compounds of the present invention are used in conjunction with chemotherapeutic agents, they may reduce the severity of chemotherapy-induced side effects.

The compounds of the present invention may also be used in combination with radiation treatment to show similar protection of normal cells from the effects of radiation and may be used as radiosensitizers to increase the tumour killing by radiation therapy.

The compounds of the present invention which are inhibitory for CDK4 or CDK6 activity will selectively inhibit cell cycle progression in cells which retain a functional retinoblastoma protein. Thus, it will be expected that inhibition of CDK4 will systemically protect normal dividing cells, including the GI and oral mucosa, hematopoietic cells and cells in the hair follicle, but be unable to protect tumour cells with loss of RB function, either by deletion or mutation. This implies that compounds that inhibit CDK4 will be useful as systemically administered cytoprotectant drugs in patients with tumours which have lost Rb, with no protective effect on the tumour itself. Such compounds could be expected to allow for increased dosing frequency and dose escalation of the cytotoxic regimens in these patients, improving the outcome of the patient.

The compounds of the present invention may also be used for the treament of other conditions mentioned in connection with modulators of CDK activity. In particular for the treatment of diseases that respond to inhibition of CDK activity, including protection of cells from infection by other viruses and treatment of Alzheimers. Furthermore, these compounds will have utility in the specific inhibition of non-human CDK activities, such as the *Aspergillus fumigatus* cdc2 homologue and will therefore be useful in the treatment of fungal or other eukaryotic infections.

While the invention has been described and illustrated with reference to certain preferred embodiments thereof, those skilled in the art will appreciate that various changes, modifications and substitutions can be made therein without departing from the spirit and scope of the invention. For example, effective dosages other than the preferred dosages as set forth herein above may be applicable as a consequence of variations in the responsiveness of the mammal being treated for cancer conditions, or for other indications for the compounds of the invention as indicated above. Likewise, the specific pharmacologic responses observed may vary according to and depending upon the particular active compound selected or whether there are present certain pharmaceutical carriers, as well as the type of formulation and mode of administration employed, and such expected variations or differences in the results are contemplated in accordance with the objects and practices of the present invenion. It is intended, therefore, that the invention be limited only by the scope of the claims that follow and that such claims be interpreted as broadly as is reasonable.

The application of which this description and claim(s) forms part may be used as a basis for priority in respect of any subsequent application. The claims of such subsequent application may be directed to any feature or combination of features described herein. They may take the form of product, formulation, process or use claims and may include, by way of example and without limitation, one or more of the following claim(s):

## Claims

1. A compound of the formula: 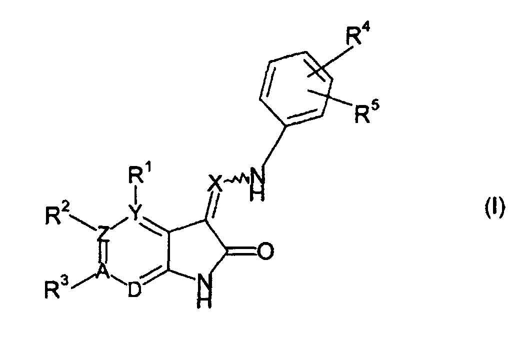 wherein:
X is selected from the group consisting of: N, CH, CCF₃, and C(C₁₋₁₂aliphatic);
Y is C or N, with the proviso that when Y is N, R¹ is absent, Z and A are each C, and
D is CH;
Z is C or N, with the proviso that when Z is N, R² is absent, Y and A are each C, and
D is CH;
A is C or N, with the proviso that when A is N, R³ is absent, Y and Z are each C, and
D is CH;
D is CH or N, with the proviso that when D is N, then Y, Z and A are each C;
with the further proviso that Y, Z and A, and D do not all simultaneously represent C and CH respectively;
R¹ is selected from the group consisting of: hydrogen, C₁₋₁₂ aliphatic, thiol, hydroxy, hydroxy-C₁₋₁₂ aliphatic, Aryl, Aryl-C₁₋₁₂ aliphatic, R⁶-Aryl-C₁₋₁₂ aliphatic, Cyc, Cyc-C₁₋₆ aliphatic, Het, Het-C₁₋₁₂ aliphatic, C₁₋₁₂ alkoxy, Aryloxy, amino, C₁₋₁₂ aliphatic amino, di-C₁₋₁₂ aliphatic amino, di-C₁₋₁₂ aliphatic aminocarbonyl, di-C₁₋₁₂ aliphatic aminosulfonyl, C₁₋₁₂ alkoxycarbonyl, halogen, cyano, sulfonamide and nitro, where R⁶, Aryl, Cyc and Het are as defined below;
R² is selected from the group consisting of: hydrogen, C₁₋₁₂ aliphatic, N-hydroxyimino-C₁₋₁₂ aliphatic, C₁₋₁₂ alkoxy, hydroxy-C₁₋₁₂ aliphatic, C₁₋₁₂ alkoxycarbonyl, carboxyl C₁₋₁₂ aliphatic, Aryl, R⁶-Aryl-oxycarbonyl, R⁶-oxycarbonyl-Aryl, Het, aminocarbonyl, C₁₋₁₂ aliphatic-aminocarbonyl, Aryl-C₁₋₁₂ aliphatic-aminocarbonyl, R⁶-Aryl-C₁₋₁₂ aliphatic-aminocarbonyl, Het-C₁₋₁₂ aliphatic-aminocarbonyl, hydroxy-C₁₋₁₂ aliphatic-aminocarbonyl, C₁₋₁₂-alkoxy-C₁₋₁₂ aliphatic-aminocarbonyl, C₁₋₁₂ alkoxy-C₁₋₁₂ aliphatic-amino, di-C₁₋₁₂ aliphatic amino, di-C₁₋₁₂ aliphatic aminocarbonyl, di-C₁₋₁₂ aliphatic aminosulfonyl, halogen, hydroxy, nitro, C₁₋₁₂ aliphatic-sulfonyl, aminosulfonyl and C₁₋₁₂ aliphatic-aminosulfonyl, where R⁶ Aryl and Het are as defined below;
R¹ and R² are optionally joined to form a fused ring selected from the group as defined for Het below, and said fused ring is optionally substituted by one or more substituents selected from the group consisting of: C₁₋₁₂ aliphatic, halogen, nitro, cyano, C₁₋₁₂ alkoxy, carbonyl-C₁₋₁₂ alkoxy and oxo;
R³ is selected from the group consisting of: hydrogen, C₁₋₁₂ aliphatic, hydroxy, hydroxy C₁₋₁₂ aliphatic, di-C₁₋₁₂ aliphatic amino, di-C₁₋₁₂ aliphatic aminocarbonyl, di-C₁₋₁₂ aliphatic aminosulfonyl, C₁₋₁₂ alkoxy, Aryl, Aryloxy, hydroxy-Aryl, Het, hydroxy-Het, Het-oxy and halogen, where Aryl and Het are as defined below;
R² and R³ are optionally joined to form a fused ring selected from the group as defined for Het below, and said fused ring is optionally substituted by C₁₋₆ aliphatic and/or C₁₋₆ aliphatic-carbonyl;
R⁴ is selected from the group consisting of: sulfonic acid, C₁₋₁₂ aliphatic-sulfonyl, sulfonyl-C₁₋₁₂ aliphatic, C₁₋₁₂ aliphatic-sulfonyl-C₁₋₆ aliphatic, C₁₋₆ aliphatic-amino, R⁷-sulfonyl, R⁷-sulfonyl-C₁₋₁₂ aliphatic, R⁷-aminosulfonyl, R⁷-aminosulfonyl-C₁₋₁₂ aliphatic, R⁷-sulfonylamino, R⁷-sulfonylamino-C₁₋₁₂ aliphatic, aminosulfonylamino, di-C₁₋₁₂ aliphatic amino, di-C₁₋₁₂ aliphatic aminocarbonyl, di-C₁₋₁₂ aliphatic aminosulfonyl, di-C₁₋₁₂ aliphatic aminosulfonyl-C₁₋₁₂ aliphatic, (R⁸)₁₋₃-Arylamino, (R⁸)₁₋₃-Arylsulfonyl, (R⁸)₁₋₃-Aryl-aminosulfonyl, (R⁸)₁₋₃-Aryl-sufonylamino, Het-amino, Het-sulfonyl, Het-aminosulfonyl, aminoiminoamino and aminoiminoaminosulfonyl, where R⁷, R⁸, Aryl and Het are as defined below;
R⁵ is hydrogen or R⁴ and R⁵ are optionally joined to form a fused ring selected from the group as defined for Het below, and said fused ring is optionally substituted by one or more substituents selected from the group consisting of: C ₁₋₁₂ aliphatic, oxo and dioxo;
R⁶ is selected from the group consisting of: C₁₋₁₂ aliphatic, hydroxy, C₁₋₁₂ alkoxy and halogen;
R⁷ is selected from the group consisting of: hydrogen, C₁₋₁₂ aliphatic, C₁₋₁₂ alkoxy, hydroxy-C₁₋₁₂ alkoxy, hydroxy-C₁₋₁₂ aliphatic, carboxylic acid, C₁₋₁₂ aliphatic-carbonyl, Het, Het-C₁₋₁₂-aliphatic, Het-C₁₋₁₂-alkoxy, di-Het-C₁₋₁₂-alkoxy Aryl, Aryl-C₁₋₁₂-aliphatic, Aryl-C₁₋₁₂-alkoxy, Aryl-carbonyl, C₁₋₁₈ alkoxyalkoxyalkoxyalkoxyaliphatic and hydroxyl, where Het and Aryl are as defined below;
R⁸ is selected from the group consisting of: hydrogen, nitro, cyano, C₁₋₁₂ alkoxy, halo, carbonyl-C₁₋₁₂ alkoxy and halo-C₁₋₁₂ aliphatic;
Aryl is selected from the group consisting of: phenyl, naphthyl, phenanthryl and anthracenyl;
Cyc is selected from the group consisting of: cyclopropyl, cyclobutyl, cyclopentyl, cyclohexyl, cycloheptyl and cyclooctyl, and optionally has one or more degrees of unsaturation;
Het is a saturated or unsaturated heteroatom ring system selected from the group consisting of: benzimidazole, dihydrothiophene, dioxin, dioxane, dioxolane, dithiane, dithiazine, dithiazole, dithiolane, furan, imidazole, isoquinoline, morpholine, oxazole, oxadiazole, oxathiazole, oxathiazolidine, oxazine, oxadiazine, piperazine, piperadine, pyran, pyrazine, pyrazole, pyridine, pyrimidine, pyrrole, pyrrolidine, quinoline, tetrahydrofuran, tetrazine, thidiazine, thiadiazole, thiatriazole, thiazine, thiazole, thiomorpholine, thiophene, thiopyran, triazine and triazole;
and the salts and solvates thereof.

2. The compound of claim 1 wherein X is selected from the group consisting of: N, CH and CCH₃.

3. The compound of claim 1 where X is CH.

4. The compound of claim 1 where X is CCH₃.

5. The compound of claim 1 where X is N.

6. The compound of claim 1 where Y is C.

7. The compound of claim 1 where Y is N, Z and A are each C, and D is CH.

8. The compound of claim 1 where Z is C.

9. The compound of claim 1 where Z is N, Y and A are each C, and D is CH.

10. The compound of claim 1 where A is C.

11. The compound of claim 1 where A is N, Y and Z are each C, and D is CH.

12. The compound of claim 1 where D is CH.

13. The compound of claim 1 where D is N and Y, Z and A are each C.

14. The compound of claim 1 where R¹ is selected from the group consisting of: hydrogen, halogen, amide, nitro, C₁₋₆alkyl, hydroxy, hydroxyalkyl, pyrimidineC₁₋₆ alkyl, C₁₋₆alkoxycarbonyl, cyclic C₃₋₆alkyl, hydroxyphenylC₁₋₆ alkyl, phenoxy, alkoxy and pyrazole.

15. The compound of claim 1 where R¹ is hydrogen or methyl.

16. The compound of claim 1 where R¹ is hydrogen.

17. The compound of claim 1 where R¹ is fused with R² to form a fused ring selected from the group consisting of: thiazole, pyrazole, triazole, halogen-substituted diazole, acyl substituted pyrrole and pyridine.

18. The compound of claim 1 where R¹ is fused with R² to form fused thiazole or fused pyridine.

19. The compound of claim 1 where R² is selected from the group consisting of:
hydrogen, halogen, sulfate, amine, quaternary amine, amide, ester, phenyl, alkoxy, aminosulfonyl, C₁₋₆alkyl sulfonyl, furanyl C₁₋₆alkyl amide, pyridinyl C₁₋₆alkyl amide, alkoxy-substituted phenyl C₁₋₆alkyl amide, morpholino C₁₋₆alkyl amide, imidazolyl C₁₋₆alkyl amide, hydroxy C₁₋₆alkyl amide, alkoxy C₁₋₆alkyl amide, C₁₋₆alkyl amide, C₁₋₆alkyl sulfonamide, C₁₋₆alkyl hydroxy substituted amino, nitro, halogen-substituted phenoxycarbonyl and triazole and oxazole rings.

20. The compound of claim 1 where R³ is selected from the group consisting of:
hydrogen, C₁₋₆alkyl, hydroxyC₁₋₆alkyl, halogen, phenoxy and alkoxy.

21. The compound of claim 1 where R² is selected from the group consisting of:
hydrogen, phenyl, 2-furanyl, 3-thiophenyl, bromo and carbethoxy.

22. The compound of claim 1 where R² is fused with R³ to form a fused ring selected from the group consisting of: oxazole, pyrrole, and dioxolane, which fused ring is optionally substituted by C₁₋₆alkyl or C₁₋₆alkyl carbonyl, and which fused ring is optionally a hetero ring having nitrogen as the heteroatom and forming a quaternary ammonium salt ionically bonded with a halogen atom.

23. The compound of claim 1 where R³ is hydrogen or chloro.

24. The compound of claim 1 where R³ is hydrogen.

25. The compound of claim 1 where R⁴ is selected from the group consisting of:
sulfonylamino, sulfonylaminoamino, C₁₋₆alkyl sulfonylamino, C₁₋₆alkylsulfonyl C₁₋₆alkyl, alkoxysulfonylamino, phenylcarbonylsulfonylamino, phenoxysulfonyl, hydroxy C₁₋₆alkylsulfonylamino, hydroxy C₁₋₆alkylsulfonylamino C₁₋₆alkyl, alkyl, phenylsulfonylamino (optionally substituted by halogen-substituted C₁₋₆alkyl), aminoiminosulfonylamino, alkylsulfonylaminoalkyl, pyridinyl C₁₋₆alkyl sulfonylamino, benzamideazolesulfonylamino, pyridylsulfonylamino, pyrimidinylsulfonylamino, thiadiazolylsulfonylamino (optionally substituted by C₁₋₆alkyl), thiazolesulfonylamino, hydroxyalkoxyalkylsulfonylamino and 4'-SO₂NH[(CH₂)₂O]₄CH₃.

26. The compound of claim 1 where R⁴ is selected from the group consisting of: 2-pyridine sulfonylamino, 4-pyridine sulfonylamino, hydroxy n-butyl sulfonylamino, methylsulfonylaminomethylene, sulfonyldimethylamino, fused 1,2-pyrazole and sulfonylamino.

27. The compound of claim 1 where R⁴ is sulfonylamino or fused 1,2-pyrazole.

28. The compound of claim 1 where R⁴ is fused with R⁵ to form a fused ring selected from the group consisting of imidazole, triazole, cyclic sulfonylamino and thiophene, where said fused ring is optionally disubstituted on the sulfur heteroatom by oxo.

29. The compound of claim 1 where R⁵ is hydrogen.

30. The compound of claim 1 where R⁶ is selected from the group consisting of:
hydrogen, C₁₋₆ alipatic, hydroxy, C₁₋₆ alkoxy and halogen.

31. The compound of claim 1 where R⁶ is selected from the group consisting of:
hydroxy, C₁₋₆ alkoxy and halogen.

32. The compound of claim 1 where R⁸ is hydrogen or halo C₁₋₆ aliphatic.

33. The compound of claim 1 where R⁸ is trifluoromethyl.

34. A compound of formula (I) as claimed in claim 1
wherein
X is selected from the group consisting of: N, CH and C(C₁₋₆ aliphatic);
Y is C or N, with the proviso that when Y is N, R¹ is absent, Z and A are each C, and D is CH;
Z is C or N, with the proviso that when Z is N, R² is absent, Y and A are each C, and D is CH;
A is C or N, with the proviso that when A is N, R³ is absent, Y and Z are each C, and D is CH;
D is CH or N, with the proviso that when D is N, then Y, Z and A are C;
with the further proviso that Y, Z and A, and D do not all simultaneously represent C and CH respectively;
R¹ is selected from the group consisting of: hydrogen, C₁₋₆ aliphatic, hydroxy-C₁₋₆ aliphatic, Aryl-C₁₋₆ aliphatic, R⁶-Aryl-C₁₋₆ aliphatic, Cyc-C₁₋₆ aliphatic, Het-C₁₋₆ aliphatic, C₁₋₆ alkoxy, Aryloxy, aminocarbonyl, di-C₁₋₆ aliphatic amino, di-C₁₋₆ aliphatic aminocarbonyl, di-C₁₋₆ aliphatic aminosulfonyl, C₁₋₆ alkoxycarbonyl, halogen and nitro, where R⁶, Aryl, Cyc and Het are as defined below;
R² is selected from the group consisting of: hydrogen, C₁₋₆ aliphatic, R⁷-C₁₋₆ aliphatic, C₁₋₆alkoxy, hydroxy-C₁₋₆ aliphatic, C₁₋₆ alkoxycarbonyl, carboxyl C₁₋₆ aliphatic, Aryl, R⁶-Aryl-oxycarbonyl, R⁶-oxycarbonyl-Aryl, Het, aminocarbonyl, C₁₋₆ aliphatic-aminocarbonyl, Aryl-C₁₋₆ aliphatic-aminocarbonyl, R⁶-Aryl-C₁₋₆ aliphatic-aminocarbonyl, Het-C₁₋₆ aliphatic-aminocarbonyl, hydroxy-C₁₋₆ aliphatic-aminocarbonyl, C₁₋₆-alkoxy-C₁₋₆ aliphatic-aminocarbonyl, C₁₋₆ alkoxy-C₁₋₆ aliphatic-amino, di-C₁₋₆ aliphatic amino, di-C₁₋₆ aliphatic aminocarbonyl, di-C₁₋₆ aliphatic aminosulfonyl, halogen, hydroxy, nitro, sulfo, C₁₋₆ aliphatic-sulfonyl, aminosulfonyl, C₁₋₆ aliphatic-aminosulfonyl and quaternary ammonium, where R⁶, R⁷, Aryl and Het are as defined below;
R¹ and R² are optionally joined to form a fused ring selected from the group as defined for Het below, and said fused ring is optionally substituted by halogen and/or oxo;
R³ is selected from the group consisting of: hydrogen, C₁₋₆ aliphatic, hydroxy, hydroxy C₁₋₆ aliphatic, di-C₁₋₆ aliphatic amino, di-C₁₋₆ aliphatic aminocarbonyl, di-C₁₋₆ aliphatic aminosulfonyl, C₁₋₆ alkoxy, Aryl, Aryloxy, hydroxy-Aryl, Het, hydroxy-Het, Het-oxy and halogen, where Aryl and Het are as defined below;
R² and R³ are optionally joined to form a fused ring selected from the group as defined for Het below, and said fused ring is optionally substituted by C₁₋₆ aliphatic or C₁₋₆ aliphatic-carbonyl;
R⁴ is selected from the group consisting of: sulfonic acid, C₁₋₁₂ aliphatic-sulfonyl, sulfonyl-C₁₋₁₂ aliphatic, C₁₋₁₂ aliphatic-sulfonyl-C₁₋₆ aliphatic, C₁₋₆ aliphatic-amino, R⁷-sulfonyl, R⁷-sulfonyl-C₁₋₁₂ aliphatic, R⁷-aminosulfonyl, R⁷-aminosulfonyl-C₁₋₁₂ aliphatic, R⁷-sulfonylamino, R⁷-sulfonylamino-C₁₋₁₂ aliphatic, aminosulfonylamino, di-C₁₋₁₂ aliphatic amino, di-C₁₋₁₂ aliphatic aminocarbonyl, di-C₁₋₁₂ aliphatic aminosulfonyl, di-C₁₋₁₂ aliphatic aminosulfonyl-C₁₋₁₂ aliphatic, (R⁸)₁₋₃-Arylamino, (R⁸)₁₋₃-Arylsulfonyl, (R⁸)₁₋₃-Aryl-aminosulfonyl, (R⁸)₁₋₃-Aryl-sulfonylamino, Het-amino, Het-sulfonyl, Het-aminosulfonyl, aminoiminoamino and aminoiminoaminosulfonyl, where R⁷, R⁸, Aryl and Het are as defined below;
R⁵ is hydrogen;
R⁴ and R⁵ are optionally joined to form a fused ring selected from the group as defined for Het below, and said fused ring is optionally substituted by oxo or dioxo;
R⁶ is selected from the group consisting of: hydrogen, C₁₋₆ aliphatic, hydroxy, C₁₋₆ alkoxy and halogen;
R⁷ is selected from the group consisting of: hydrogen, C₁₋₁₂ aliphatic, C₁₋₁₂ alkoxy, hydroxy-C₁₋₁₂ alkoxy, hydroxy-C₁₋₁₂ aliphatic, carboxylic acid, C₁₋₁₂ aliphatic-carbonyl, Het, Het-C₁₋₁₂-aliphatic, Het-C₁₋₁₂-alkoxy, di-Het-C₁₋₁₂-alkoxy Aryl, Aryl-C₁₋₁₂-aliphatic, Aryl-C₁₋₁₂-alkoxy, Aryl-carbonyl, C₁₋₁₈ alkoxyalkoxyalkoxyalkoxyaliphatic and hydroxyl, where Het and Aryl are as defined below;
R⁸ is hydrogen and/or halo-C₁₋₆ aliphatic;
Aryl is phenyl or naphthyl;
Cyc is selected from the group consisting of: cyclopropyl, cyclobutyl, cyclopentyl, cyclohexyl, cycloheptyl and cyclooctyl, and optionally has one or more degrees of unsaturation;
Het is a saturated or unsaturated heteroatom ring system selected from the group consisting of: benzimidazole, dihydrothiophene, dioxin, dioxane, dioxolane, dithiane, dithiazine, dithiazole, dithiolane, furan, imidazole, morpholine, oxazole, oxadiazole, oxathiazole, oxathiazolidine, oxazine, oxadiazine, piperazine, piperadine, pyran, pyrazine, pyrazole, pyridine, pyrimidine, pyrrole, pyrrolidine, tetrahydrofuran, tetrazine, thiadiazine, thiadiazole, thiatriazole, thiazine, thiazole, thiomorpholine, thiophene, thiopyran, triazine and triazole;
and the salts and solvates thereof.

35. A compound of formula (I) as claimed in claim 1
wherein
X is selected from the group consisting of: N, CH and CCH_{3;}
Y is C or N, with the proviso that when Y is N, R¹ is absent, Z and A are each C, and D is CH;
Z is C or N, with the proviso that when Z is N, R² is absent, Y and A are each C, and D is CH;
A is C or N, with the proviso that when A is N, R³ is absent, X and Y are each C, and D is CH;
D is CH or N, with the proviso that when D is N, then Y, Z and A are each C;
with the further proviso that Y, Z and A, and D do not all simultaneously represent C and CH respectively;
R¹ is selected from the group consisting of: hydrogen, C₁₋₆ aliphatic, hydroxy-C₁₋₆ aliphatic, di-C₁₋₆ aliphatic amino, di-C₁₋₆ aliphatic aminocarbonyl, di-C₁₋₆ aliphatic aminosulfonyl, Aryl-C₁₋₆ aliphatic, R⁶Aryl-C₁₋₆ aliphatic, Cyc-C₁₋₆ aliphatic, Het-C₁₋₆ aliphatic, C₁₋₆ alkoxy, Aryloxy, aminocarbonyl, C₁₋₆ alkoxycarbonyl, halogen and nitro, where R⁶, Aryl, Cyc and Het are as defined below;
R² is selected from the group consisting of: hydrogen, C₁₋₆ aliphatic, N-hydroxyimino-C₁₋₆ aliphatic, C₁₋₆alkoxy, C₁₋₆ alkoxycarbonyl, Aryl, R⁶-Aryloxycarbonyl, Het, aminocarbonyl, C₁₋₆ aliphatic aminocarbonyl, Aryl-C₁₋₆ aliphatic aminocarbonyl, R⁶-Aryl-C₁₋₆ aliphatic aminocarbonyl, Het-C₁₋₆ aliphatic aminocarbonyl, di-C₁₋₆ aliphatic amino, di-C₁₋₆ aliphatic aminocarbonyl, di-C₁₋₆ aliphatic aminosulfonyl, hydroxy-C₁₋₆ aliphatic aminocarbonyl, C₁₋₆-alkoxy-C₁₋₆ aliphatic aminocarbonyl, C₁₋₆ alkoxy-C₁₋₆ aliphatic amino, halogen, hydroxy, nitro, C₁₋₆ aliphatic sulfonyl, aminosulfonyl and C₁₋₆ aliphatic aminosulfonyl, where R⁶, Aryl and Het are as defined below;
R¹ and R² are optionally joined to form a fused ring selected from the group as defined for Het below, and said fused ring is optionally substituted by halogen and/or oxo;
R³ is selected from the group consisting of: hydrogen, C₁₋₆ aliphatic, hydroxy, hydroxy C₁₋₆ aliphatic, di-C₁₋₆ aliphatic amino, di-C₁₋₆ aliphatic aminocarbonyl, di-C₁₋₆ aliphatic aminosulfonyl C₁₋₆ alkoxy, Aryloxy, Het and halogen, where Aryl and Het are as defined below;
R² and R³ are optionally joined to form a fused ring selected from the group as defined for Het below, and said fused ring is optionally substituted by C₁₋₆ alkyl and/or C₁₋₆ alkylcarbonyl;
R⁴ is selected from the group consisting of: R⁷-sulfonyl, R⁷-sulfonyl C₁₋₆-aliphatic, C₁₋₆ aliphatic sulfonyl-C₁₋₆ aliphatic, R⁷-aminosulfonyl, di-C₁₋₆ aliphatic amino, di-C₁₋₆ aliphatic aminocarbonyl, di-C₁₋₆ aliphatic aminosulfonyl, di-C₁₋₆ aliphatic aminosulfonyl-C₁₋₆ aliphatic, R⁷-aminosulfonyl C₁₋₆ aliphatic, aminosulfonylamino, R⁷-C₁₋₆ aliphatic aminosulfonyl-C₁₋₆ aliphatic, Aryl, Het, R⁸-Aryl-aminosulfonyl, Het-aminosulfonyl and aminoiminoaminosulfonyl, where R⁷, R⁸, Aryl and Het are as defined below;
R⁵ is hydrogen;
R⁴ and R⁵ are optionally joined to form a fused ring selected from the group as defined for Het below, and said fused ring is optionally substituted by oxo or dioxo;
R⁶ is selected from the group consisting of: hydroxy, C₁₋₆ alkoxy and halogen;
R⁷ is selected from the group consisting of: hydrogen, C₁₋₆ aliphatic, hydroxy C₁₋₆-alkoxy, hydroxy-C₁₋₆ aliphatic, C₁₋₆ aliphatic carbonyl, Aryl-carbonyl, C₁₋₁₂ alkoxyalkoxyalkoxyalkoxyalkyl, hydroxyl, Aryl, Aryl-C₁₋₆-alkoxy, Aryl-C₁₋₆-aliphatic,
Het, Het-C₁₋₆-alkoxy, di-Het-C₁₋₆-alkoxyl Het-C₁₋₆-aliphatic and di-Het-C₁₋₆-aliphatic;
R⁸ is trifluoromethyl;
Aryl is phenyl;
Cyc is cyclobutyl;
Het is a saturated or unsaturated heteroatom ring system selected from the group consisting of: benzimidazole, dihydrothiophene, dioxolane, furan, imidazole, morpholine, oxazole, pyridine, pyrrole, pyrrolidine, thiadiazole, thiazole, thiophene,
and triazole;
and the salts and solvates thereof.

36. A compound as claimed in any one of claims 1 to 35 in the form of a substantially pure E geometric isomer.

37. A compound as claimed in any one of claims 1 to 35 in the form of a substantially pure Z geometric isomer.

38. A compound as claimed in any one of claims 1 to 35 in the form of a mixture of E geometric isomer and Z geometric isomer in any proportions of said geometric isomers.

39. A compound as claimed in claim 1, in E, Z, or E and Z, form, selected from the group consisting of:
4-{[(2-Oxo-1,2-dihydro-3H-pyrrolo[3,2-c]pyridin-3-ylidene)methyl]amino}benzenesulfonamide;
4-{[(2-Oxo-1,2-dihydro-3H-pyrrolo[2,3-c]pyridin-3-ylidene)methyl]amino}benzenesulfonamide;
3-[(1H-Indazol-6-ylamino)methylidene]-1H-pyrrolo[3,2-b]pyridin-2-one;
3-[(6-Quinolinylamino)methylidene]-1,3-dihydro-2H-pyrrolo[3,2-b]pyridin-2-one;
4-{[(2-Oxo-1,2-dihydro-3H-pyrrolo[2,3-b]pyridin-3-ylidene)methyl]amino} benzenesulfonamide;
3-[(1H-Indazol-6-ylamino)methylidene]-1H-pyrrolo[2,3-b]pyridin-2-one;
3-[(6-Quinolinylamino)methylidene]-1,3-dihydro-2H-pyrrolo[2,3-b]pyridin-2-one;
4-{[(2-Oxo-5-phenyl-1,2-dihydro-3H-pyrrolo[2, 3-b]pyridin-3-ylidene)methyl]amino} benzenesulfonamide;
3-[(1H-Indazol-6-ylamino)methylidene]-5-phenyl-1H-pyrrolo[2,3-b]pyridin-2-one;
5-Phenyl-3-[(6-quinolinylamino)methylidene]-1,3-dihydro-2H-pyrrolo[2,3-b]pyridin-2-one;
4-({[5-(2-Furyl)-2-oxo-1,2-d ihydro-3H-pyrrolo[2,3-b]pyridin-3-ylidene]methyl}amino)benzenesulfonamide;
5-(2-Furyl)-3-[(1H-indazol-6-ylamino)methylidene]-1H-pyrrolo[2,3-b]pyridin-2-one;
5-(2-Furyl)-3-[(6-quinolinylamino)methylidene]-1,3-dihydro-2H-pyrrolo[2,3-b]pyridin-2-one;
4-({[2-Oxo-5-(3-thienyl)-1,2-dihydro-3H-pyrrolo[2,3-b]pyridin-3-ylidene]methyl}amino)benzenesulfonamide;
3-[(1H-lndazol-6-ylamino)methylidene]-5-(3-thienyl)-1,3-dihydro-2H-pyrrolo[2,3-b]pyridin-2-one;
3-[(6-Quinolinylamino)methylidene]-5-(3-thienyl)-1,3-dihydro-2H-pyrrolo[2,3-b]pyridin-2-one;
4-{[(5-Bromo-2-oxo-1,2-dihydro-3H-pyrrolo[2,3-b]pyridin-3-ylidene)methyl]amino}benzenesulfonamide;
5-Bromo-3-[(1H-indazol-6-ylamino)methylidene]-1,3-dihydro-2H-pyrrolo[2,3-b]pyridin-2-one;
5-Bromo-3-[(6-quinolinylamino)methylidene]-1,3-dihydro-2H-pyrrolo[2,3-b]pyridin-2-one;
4-{[(6-Chloro-2-oxo-1,2-dihydro-3H-pyrrolo[2,3-b]pyridin-3-ylidene)methyl]amino}benzenesulfonamide;
6-Chloro-3-[(1H-indazol-6-ylamino)methylidene]-1,3-dihydro-2H-pyrrolo[2,3-b]pyridin-2-one;
6-Chloro-3-[(6-quinolinylamino)methylidene]-1,3-dihydro-2H-pyrrolo[2,3-b]pyridin-2-one;
Ethyl-3-{[4-(aminosulfonyl)anilino]methylidene}-2-oxo-1,2-dihydro-3H-pyrrolo[2,3-b]pyridine-5-carboxylate;
Ethyl-3-[(1H-indazol-6-ylamino)methylidene]-2-oxo-1,2-dihydro-3H-pyrrolo[2,3-b]pyridine-5-carboxylate; and
Ethyl-2-oxo-3-[(6-quinolinylamino)methylidene]-2,3-dihydro-1H-pyrrolo[2,3-b]pyridine-5-carboxylate;
and the pharmaceutically acceptable salts and solvates thereof.

40. A pharmaceutical composition comprising a compound as claimed in any one of claims 1 to 39 and one or more pharmaceutically acceptable carriers, diluents and/or excipients.

41. A compound as claimed in any one of claims 1 to 39 for use in therapy.

42. The use of a compound as claimed in any one of claims 1 to 39 in the preparation of a medicament for the treatment of a disease or disorder in an animal wherein the disease or disorder comprises at least one condition selected from the group consisting of: organ transplant rejection, tumor growth, chemotherapy-induced alopecia, chemotherapy-induced thrombocytopenia, chemotherapy-induced leukopenia, mucocitis, plantar-palmar syndrome, restenosis, atherosclerosis, rheumatoid arthritis, angiogenesis, hepatic cirrhosis, glomerulonephritis, diabetic nephropathy, malignant nephrosclerosis, thrombotic microangiopathy, glomerulopathy, psoriasis, diabetes mellitus, inflammation, neurodegenerative disease, macular degeneration, actinic keratosis and hyperproliferative disorders.

43. The use of a compound as claimed in any one of claims 1 to 39 in the preparation of a medicament for the treatment of a disease or disorder in an animal wherein the disease or disorder comprises a viral or eukaryotic infection.

44. The use of a compound as claimed in any one of claims 1 to 39 in the preparation of a medicament for the treatment of a disease or disorder in an animal wherein the disease or disorder comprises alopecia.

45. The use of a compound as claimed in any one of claims 1 to 39 in the preparation of a medicament for the treatment of a disease or disorder in an animal wherein the disease or disorder comprises at least one condition mediated by a kinase.

46. The use of claim 45 wherein the kinase is a mitogen activated protein kinase.

47. The use of claim 45 wherein the kinase is selected from the group consisting of: abl, ARaf, ATK, ATM, bcr-abl, Blk, BRaf, Brk, Btk, CDK1, CDK2, CDK3, CDK4, CDK5, CDK6, CDK7, CDK8, CDK9, c-fms, c-kit, c-met, cRaf1, CSF1R, CSK, c-src, EGFR, ErbB2, ErbB3, ErbB4, ERK, ERK1, ERK2, Fak, fes, FGFR1, FGFR2, FGFR3, FGFR4, FGFR5, Fgr, FLK-4, Fps, Frk, Fyn, GSK, gsk3a, gsk3b, Hck, IGF-1R, IKK, IKK1, IKK2, IKK3, INS-R, Integrin-linked kinase, JAK, JAK1, JAK2, JAK3, JNK, Lck, Lyn, MEK, MEK1, MEK2, p38, PDGFR, PIK, PKB1, PKB2, PKB3, PKC, PKCa, PKCb, PKCd, PKCe, PKCg, PKCI, PKCm, PKCz, PLK1, Polo-like kinase, PYK2, tie₁, tie₂, TrkA, TrkB, TrkC, UL13, UL97, VEGF-R1, VEGF-R2, Yes and Zap70.

48. The use of claim 45 wherein the kinase is selected from the group consisting of: VEGF-R2, CDK1, CDK2, CDK3, Zap70, Lck and c-fms.

49. The use of claim 45 wherein the kinase is VEGF-R2.

50. The use according to any one of claims 42 to 49 wherein the compound or physiologically acceptable salt thereof is administered in an amount of from about 0.1 to about 100 mg/kg of body weight per day.

51. The use according to claim 50 wherein the compound or physiologically acceptable salt thereof is administered in an amount of from about 0.1 to about 10 mg/kg of body weight per day.

52. The use according to any one of claims 42 to 51 wherein the animal is a human.

53. The use according to any one of claims 42 to 52 wherein the compound is coadministered with one or more anti-cancer agents and/or treatments.

## Patentansprüche

1. Verbindung der Formel: 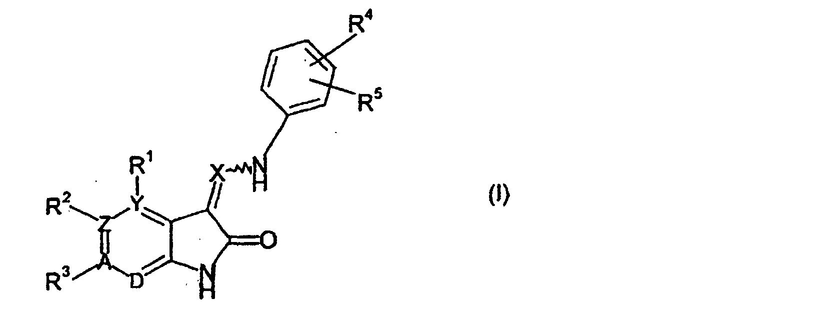 wobei:
X aus einem N-Atom und den Resten CH, CCF₃ und C(aliphatisches-C₁₋₁₂) ausgewählt ist;
Y ein C- oder N-Atom ist, mit der Maßgabe, dass, falls Y ein N-Atom ist, R¹ abwesend ist, Z und A jeweils ein C-Atom sind und D eine CH-Gruppe ist;
Z ein C- oder N-Atom ist, mit der Maßgabe, dass, falls Z ein N-Atom ist, R² abwesend ist, Y und A jeweils ein C-Atom sind und D eine CH-Gruppe ist;
A ein C- oder N-Atom ist, mit der Maßgabe, dass, falls A ein N-Atom ist, R³ abwesend ist, Y und Z jeweils ein C-Atom sind und D eine CH-Gruppe ist;
D eine CH-Gruppe oder ein N-Atom ist, mit der Maßgabe, dass, falls D ein N-Atom ist,
Y, Z und A dann jeweils ein C-Atom sind;
mit der weiteren Maßgabe, dass Y, Z, und A, und D nicht alle gleichzeitig ein C-Atom beziehungsweise eine CH-Gruppe bedeuten;
R¹ aus einem Wasserstoffatom, einem aliphatischen-C₁₋₁₂-Rest, einer Thiol- oder Hydroxygruppe, den Resten Hydroxy-aliphatisches-C₁₋₁₂, Aryl, Aryl-aliphatisches-C₁₋₁₂,
R⁶-Aryl-aliphatisches-C₁₋₁₂, Cyc, Cyc-aliphatisches-C₁₋₆, Het, Het-aliphatisches-C₁₋₁₂, einem C₁₋₁₂-Alkoxy- oder Aryloxyrest, einer Aminogruppe, den Resten aliphatisches-C₁₋₁₂-amino, Di-aliphatisches-C₁₋₁₂-amino, Di-aliphatisches-C₁₋₁₂-aminocarbonyl, Dialiphatisches-C₁₋₁₂-aminosulfonyl, einem C₁₋₁₂-Alkoxycarbonylrest, einem Halogenatom, einer Cyano-, Sulfonamid- und Nitrogruppe ausgewählt ist, wobei R⁶ ein
Rest Aryl, Cyc und Het wie nachstehend definiert ist;
R² aus einem Wasserstoffatom, den Resten aliphatisches-C₁₋₁₂, N-Hydroxyiminoaliphatisches-C₁₋₁₂, C₁₋₁₂-Alkoxy, Hydroxy-aliphatisches-C₁₋₁₂, C₁₋₁₂-Alkoxycarbonyl, Carboxyl-aliphatisches-C₁₋₁₂, Aryl, R⁶-Aryl-oxycarbonyl, R⁶-Oxycarbonyl-aryl, Het, Aminocarbonyl, aliphatisches-C₁₋₁₂-Aminocarbonyl, Aryl-aliphatisches-C₁₋₁₂aminocarbonyl, R⁶-Aryl-aliphatisches-C₁-₁₂-aminocarbonyl, Het-aliphatisches-C₁₋₁₂aminocarbonyl, Hydroxy-aliphatisches-C₁₋₁₂-aminocarbonyl, C₁₋₁₂-Alkoxyaliphatisches-C₁₋₁₂-aminocarbonyl, C₁₋₁₂-Alkoxy-aliphatisches-C₁₋₁₂-amino, Dialiphatisches-C₁₋₁₂-amino, Di-aliphatisches-C₁-₁₂-aminocarbonyl, Di-aliphatisches-C₁₋₁₂-aminosulfonyl, einem Halogenatom, einer Hydroxy- oder Nitrogruppe, den Resten aliphatisches-C₁₋₁₂-Sulfonyl, Aminosulfonyl und aliphatisches-C₁₋₁₂-Aminosulfonyl ausgewählt ist, wobei R⁶ Aryl und Het wie nachstehend definiert sind;
R¹ und R² gegebenenfalls verbunden sind, um einen kondensierten Ring, ausgewählt aus den Resten wie nachstehend für Het definiert, zu bilden, und der kondensierte Ring gegebenenfalls durch einen oder mehrere Substituenten, ausgewählt aus einem aliphatischen-C₁₋₁₂-Rest, einem Halogenatom, einer Nitro- oder Cyanogruppe, den Resten C₁₋₁₂-Alkoxy, Carbonyl-C₁₋₁₂-alkoxy und Oxo, substituiert ist;
R³ ausgewählt ist aus: einem Wasserstoffatom, einem aliphatischen C₁₋₁₂-Rest, einer Hydroxygruppe, den Resten Hydroxy-aliphatisches-C₁₋₁₂, Di-aliphatisches-C₁₋₁₂-amino, Di-aliphatisches-C₁₋₁₂-aminocarbonyl, Di-aliphatisches-C₁₋₁₂-aminosulfonyl, C₁₋₁₂-Alkoxy, Aryl, Aryloxy, Hydroxy-aryl, Het, Hydroxy-Het, Het-oxy und einem Halogenatom, wobei Aryl und Het wie nachstehend definiert sind;
R² und R³ gegebenenfalls verbunden sind, um einen kondensierten Ring, ausgewählt aus den Resten wie nachstehend für Het definiert, zu bilden, und der kondensierte Ring gegebenenfalls durch einen aliphatischen-C₁₋₆-und/oder aliphatischen-C₁₋₆-Carbonylrest substituiert ist;
R⁴ ausgewählt ist aus: einer Sulfonsäuregruppe, den Resten aliphatisches-C₁₋₁₂-Sulfonyl, Sulfonyl-aliphatisches-C₁₋₁₂, aliphatisches-C₁₋₁₂-Sulfonyl-aliphatisches-C₁₋₆, aliphatisches-C₁₋₆-amino, R⁷-Sulfonyl, R⁷-Sulfonyl-aliphatisches-C₁₋₁₂, R⁷-Aminosulfonyl, R⁷-Aminosulfonyl-aliphatisches-C₁₋₁₂, R⁷-Sulfonylamino, R⁷-Sulfonylamino-aliphatisches-C₁₋₁₂, Aminosulfonylamino, Di-aliphatisches-C₁₋₁₂-amino, Di-aliphatisches-C₁₋₁₂-aminocarbonyl, Di-aliphatisches-C₁₋₁₂-aminosulfonyl, Dialiphatisches-C₁₋₁₂-aminosulfonyl-aliphatisches-C₁₋₁₂, (R⁸)₁₋₃-Arylamino, (R⁸)₁₋₃-Arylsulfonyl, (R⁸)₁₋₃-Aryl-aminosulfonyl, (R⁸)₁₋₃-Aryl-sulfonylamino, Het-amino, Het-sulfonyl, Het-aminosulfonyl, Aminoiminoamino und Aminoiminoaminosulfonyl, wobei
R⁷, R⁸, Aryl und Het wie nachstehend definiert sind;
R⁵ ein Wasserstoffatom ist oder R⁴ und R⁵ gegebenenfalls verbunden sind, um einen kondensierten Ring, ausgewählt aus einem Rest wie nachstehend für Het definiert, zu bilden, und der kondensierte Ring gegebenenfalls durch einen oder mehrere Substituenten, ausgewählt aus den Resten aliphatisches-C₁₋₁₂, Oxo und Dioxo, substituiert ist;
R⁶ ausgewählt ist aus einem aliphatischen C₁₋₁₂-Rest, einer Hydroxygruppe, einem C₁₋₁₂-Alkoxyrest und einem Halogenatom;
R⁷ ausgewählt ist aus: einem Wasserstoffatom, den Resten aliphatisches-C₁₋₁₂, C₁₋₁₂-Alkoxy, Hydroxy-C₁₋₁₂-alkoxy, Hydroxy-aliphatisches-C₁₋₁₂, einer Carbonsäuregruppe, den Resten aliphatisches-C₁₋₁₂-Carbonyl, Het, Het-aliphatisches-C₁₋₁₂, Het-C₁₋₁₂-alkoxy,
Di-Het-C₁₋₁₂-alkoxy-aryl, Aryl-aliphatisches-C₁₋₁₂, Aryl-C₁₋₁₂-alkoxy, Aryl-carbonyl, aliphatisches C₁₋₁₈-Alkoxyalkoxyalkoxyalkoxy und einer Hydroxylgruppe, wobei Het und Aryl wie nachstehend definiert sind;
R⁸ ausgewählt ist aus: einem Wasserstoffatom, einer Nitro- oder Cyanogruppe, einem C₁₋₁₂-Alkoxyrest, einem Halogenatom, einem Carbonyl-C₁₋₁₂-alkoxyrest und einem Halogen-aliphatisches-C₁₋₁₂-Rest;
Aryl ausgewählt ist aus: einem Phenyl-, Naphthyl-, Phenanthryl- und Anthracenylrest; Cyc ausgewählt ist aus: einem Cyclopropyl-, Cyclobutyl-, Cyclopentyl-, Cyclohexyl-, Cycloheptyl- und Cyclooctylrest, und gegebenenfalls eine oder mehrere ungesättigte Stellen aufweist;
Het ein gesättigtes oder ungesättigtes Heteroatomringsystem ist, ausgewählt aus: einem Benzimidazol-, Dihydrothiophen-, Dioxin-, Dioxan-, Dioxolan-, Dithian-, Dithiazin-, Dithiazol-, Dithiolan-, Furan-, Imidazol-, Isochinolin-, Morpholin-, Oxazol-, Oxadiazol-, Oxathiazol-, Oxathiazolidin-, Oxazin-, Oxadiazin-, Piperazin-, Piperadin-, Pyran-, Pyrazine-, Pyrazol-, Pyridin-, Pyrimidin-, Pyrrol-, Pyrrolidin-, Chinolin-, Tetrahydrofuran-, Tetrazin-, Thidiazin-, Thiadiazol-, Thiatriazol-, Thiazin-, Thiazol-, Thiomorpholin-, Thiophen-, Thiopyran-, Triazin- und Triazolerest;
und Salzen und Solvaten davon.

2. Verbindung gemäß Anspruch 1, wobei X aus einem N-Atom und einer CH- und CCH₃-Gruppe ausgewählt ist.

3. Verbindung gemäß Anspruch 1, wobei X eine CH-Gruppe ist.

4. Verbindung gemäß Anspruch 1, wobei X eine CCH₃-Gruppe ist.

5. Verbindung gemäß Anspruch 1, wobei X ein N-Atom ist.

6. Verbindung gemäß Anspruch 1, wobei Y ein C-Atom ist.

7. Verbindung gemäß Anspruch 1, wobei Y ein N-Atom ist, Z und A jeweils ein C-Atom sind und D eine CH-Gruppe ist.

8. Verbindung gemäß Anspruch 1, wobei Z ein C-Atom ist.

9. Verbindung gemäß Anspruch 1, wobei Z ein N-Atom ist, Y und A jeweils ein C-Atom sind und D eine CH-Gruppe ist.

10. Verbindung gemäß Anspruch 1, wobei A ein C-Atom ist.

11. Verbindung gemäß Anspruch 1, wobei A ein N-Atom ist, Y und Z jeweils ein C-Atom sind und D eine CH-Gruppe ist.

12. Verbindung gemäß Anspruch 1, wobei D eine CH-Gruppe ist.

13. Verbindung gemäß Anspruch 1, wobei D ein N-Atom ist und Y, Z und A jeweils ein C-Atom sind.

14. Verbindung gemäß Anspruch 1, wobei R¹ ausgewählt ist aus: einem Wasserstoff- oder Halogenatom, einem Amidrest, einer Nitrogruppe, einem C₁₋₆-Alkylrest, einer Hydroxygruppe, einem Hydroxyalkyl-, Pyrimidin-C₁₋₆-alkyl-, C₁₋₆-Alkoxycarbonyl-, cyclischen C₃₋₆-Alkyl-, Hydroxyphenyl-C₁₋₆-alkyl-, Phenoxy-, Alkoxy- und Pyrazolrest.

15. Verbindung gemäß Anspruch 1, wobei R¹ ein Wasserstoffatom oder eine Methylgruppe ist.

16. Verbindung gemäß Anspruch 1, wobei R¹ ein Wasserstoffatom ist.

17. Verbindung gemäß Anspruch 1, wobei R¹ mit R² kondensiert ist, um einen kondensierten Ring zu bilden, der ausgewählt ist aus: einem Thiazol-, Pyrazol-, Triazol-, Halogen-substituierten Diazol-, Acyl-substituierten Pyrrol- und Pyridinrest.

18. Verbindung gemäß Anspruch 1, wobei R¹ mit R² kondensiert ist, um einen kondensierten Thiazol- oder kondensierten Pyridinrest zu bilden.

19. Verbindung gemäß Anspruch 1, wobei R² ausgewählt ist aus: einem Wasserstoff- oder Halogenatom, einem Sulfat-, Amin-, quartären Amin-, Amid-, Ester-, Phenyl-, Alkoxy-, Aminosulfonyl-, C₁₋₆-Alkylsulfonyl-, Furanyl-C₁₋₆-alkylamid-, Pyridinyl-C₁₋₆alkylamid-, Alkoxy-substituierten Phenyl-C₁₋₆-alkylamid-, Morpholino-C₁₋₆-alkylamid-, Imidazolyl-C₁₋₆-alkylamid-, Hydroxy-C₁₋₆-alkylamid-, Alkoxy-C₁₋₆-alkylamid-, C₁₋₆-Alkylamid-, C₁₋₆-Alkylsulfonamid-, und C₁₋₆-Alkyl-hydroxy-substituierten-amino-Rest, einer Nitrogruppe, einem Halogen-substituierten Phenoxycarbonylrest und Triazol- und Oxazolringen.

20. Verbindung gemäß Anspruch 1, wobei R³ ausgewählt ist aus: einem Wasserstoffatom, einem C₁₋₆-Alkyl-, und Hydroxy-C₁₋₆-alkylrest, einem Halogenatom, einem Phenoxyund Alkoxyrest.

21. Verbindung gemäß Anspruch 1, wobei R² ausgewählt ist aus: einem Wasserstoffatom, einem Phenyl-, 2-Furanyl-, 3-Thiophenylrest, einem Bromatom und einem Carbethoxyrest.

22. Verbindung gemäß Anspruch 1, wobei R² mit R³ kondensiert ist, um einen kondensierten Ring zu bilden, der ausgewählt ist aus: einem Oxazol-, Pyrrol- und Dioxolanrest, wobei der kondensierte Ring gegebenenfalls durch einen C₁₋₆-Alkyl- oder C₁₋₆-Alkylcarbonylrest substituiert ist, und wobei der kondensierte Ring gegebenenfalls ein Heteroring ist, welcher ein Stickstoffatom als Heteroatom aufweist und ein quartäres Ammoniumsalz mit einem ionisch gebundenen Halogenatom bildet.

23. Verbindung gemäß Anspruch 1, wobei R³ ein Wasserstoff- oder Chloratom ist.

24. Verbindung gemäß Anspruch 1, wobei R³ ein Wasserstoffatom ist.

25. Verbindung gemäß Anspruch 1, wobei R⁴ ausgewählt ist aus: einem Sulfonylamino-, Sulfonylaminoamino-, C₁₋₆-Alkylsulfonylamino-, C₁₋₆-Alkylsulfonyl-C₁₋₆-alkyl-, Alkoxysulfonylamino-, Phenylcarbonylsulfonylamino-, Phenoxysulfonyl-, Hydroxy-C₁₋₆-alkylsulfonylamino-, Hydroxy-C₁₋₆-alkylsulfonylamino-C₁₋₆-alkyl-, Alkyl-, Phenylsulfonylamino- (gegebenenfalls durch einen Halogen-substituierten C₁₋₆-Alkylrest substituiert), Aminoiminosulfonylamino-, Alkylsulfonylaminoalkyl-, Pyridinyl-C₁₋₆-alkylsulfonylamino-, Benzamidazolsulfonylamino-, Pyridylsulfonylamino-, Pyrimidinylsulfonylamino-, Thiadiazolylsulfonylamino(gegebenenfalls durch einen C₁₋₆-Alkylrest substituiert), Thiazolsulfonylamino-, und einem Hydroxyalkoxyalkylsulfonylaminorest und einer Gruppe 4'-SO₂NH[(CH₂)₂O]₄CH₃.

26. Verbindung gemäß Anspruch 1, wobei R⁴ ausgewählt ist aus: einem 2-Pyridinsulfonylamino-, 4-Pyridinsulfonylamino-, Hydroxy-n-butylsulfonylamino-, Methylsulfonylaminomethylen-, Sulfonyldimethylamino-, kondensierten 1,2-Pyrazolund Sulfonylaminorest.

27. Verbindung gemäß Anspruch 1, wobei R⁴ ein Sulfonylamino- oder kondensierter 1,2-Pyrazolrest ist.

28. Verbindung gemäß Anspruch 1, wobei R⁴ mit R⁵ kondensiert ist, um einen kondensierten Ring zu bilden, der ausgewählt ist aus einen Imidazol, Triazol, cyclischen Sulfonylamino- und Thiophenrest, wobei der kondensierte Ring gegebenenfalls an dem Schwefelheteroatom durch Oxoreste disubstituiert ist.

29. Verbindung gemäß Anspruch 1, wobei R⁵ ein Wasserstoffatom ist.

30. Verbindung gemäß Anspruch 1, wobei R⁶ ausgewählt ist aus: einem Wasserstoffatom, einem aliphatischen C₁₋₆-Rest, einer Hydroxygruppe, einem C₁₋₆-Alkoxyrest und einem Halogenatom.

31. Verbindung gemäß Anspruch 1, wobei R⁶ ausgewählt ist aus: einer Hydroxygruppe, einem C₁₋₆-Alkoxyrest und einem Halogenatom.

32. Verbindung gemäß Anspruch 1, wobei R⁸ ein Wasserstoffatom oder ein Halogenaliphatischer-C₁₋₆-Rest ist.

33. Verbindung gemäß Anspruch 1, wobei R⁸ eine Trifluormethylgruppe ist.

34. Verbindung der Formel (I) gemäß Anspruch 1,
wobei
X ausgewählt ist aus: einem N-Atom und den Resten CH und C(aliphatisches-C₁₋₆);
Y ein C- oder N-Atom ist, mit der Maßgabe, dass, falls Y ein N-Atom ist, R¹ abwesend ist, Z und A jeweils ein C-Atom sind und D eine CH-Gruppe ist;
Z ein C- oder N-Atom ist, mit der Maßgabe, dass, falls Z ein N-Atom ist, R² abwesend ist, Y und A jeweils ein C-Atom sind und D eine CH-Gruppe ist;
A ein C- oder N-Atom ist, mit der Maßgabe, dass, falls A ein N-Atom ist, R³ abwesend ist, Y und Z jeweils ein C-Atom sind und D eine CH-Gruppe ist;
D eine CH-Gruppe oder ein N-Atom ist, mit der Maßgabe, dass, falls D ein N-Atom ist, Y, Z und A dann ein C-Atom sind;
mit der weiteren Maßgabe, dass Y, Z und A und D nicht alle gleichzeitig ein C-Atom beziehungsweise eine CH-Gruppe bedeuten;
R¹ ausgewählt ist aus: einem Wasserstoffatom, den Resten aliphatisches-C₁₋₆, Hydroxyaliphatisches-C₁₋₆, Aryl-aliphatisches-C₁₋₆, R⁶-Aryl-aliphatisches-C₁₋₆, Cycaliphatisches-C₁₋₆, Het-aliphatisches-C₁₋₆, C₁₋₆-Alkoxy, Aryloxy, Aminocarbonyl, Dialiphatisches-C₁₋₆-amino, Di-aliphatisches-C₁₋₆-aminocarbonyl, Di-aliphatisches-C₁₋₆aminosulfonyl, C₁₋₆-Alkoxycarbonyl, einem Halogenatom und einer Nitrogruppe, wobei R⁶, Aryl, Cyc und Het wie nachstehend definiert sind;
R² ausgewählt ist aus: einem Wasserstoffatom, den Resten aliphatisches-C₁₋₆, R⁷aliphatisches-C₁₋₆, C₁₋₆-Alkoxy, Hydroxy-aliphatisches-C₁₋₆, C₁₋₆-Alkoxycarbonyl, Carboxyl-aliphatisches-C₁₋₆, Aryl, R⁶-Aryl-oxycarbonyl, R⁶-Oxycarbonyl-aryl, Het, Aminocarbonyl, aliphatisches-C₁₋₆-Aminocarbonyl, Aryl-aliphatisches-C₁₋₆aminocarbonyl, R⁶-Aryl-aliphatisches-C₁₋₆-aminocarbonyl, Het-aliphatisches-C₁₋₆aminocarbonyl, Hydroxy-aliphatisches-C₁₋₆-aminocarbonyl, C₁₋₆-Alkoxy-aliphatisches-C₁₋₆-aminocarbonyl, C₁₋₆-Alkoxy-aliphatisches-C₁₋₆-amino, Di-aliphatisches-C₁₋₆-amino, Di-aliphatisches-C₁₋₆-aminocarbonyl, Di-aliphatisches-C₁₋₆-aminosulfonyl, einem Halogenatom, einer Hydroxy- oder Nitrogruppe, den Resten Sulfo, aliphatisches-C₁₋₆-Sulfonyl, Aminosulfonyl, aliphatisches-C₁₋₆-Aminosulfonyl und quartäres Ammonium,
wobei R⁶, R⁷, Aryl und Het wie nachstehend definiert sind;
R¹ und R² gegebenenfalls verbunden sind, um einen kondensierten Ring, ausgewählt aus den Resten wie nachstehend für Het definiert, zu bilden, und der kondensierte Ring gegebenenfalls durch ein Halogenatom und/oder einen Oxorest substituiert ist;
R³ ausgewählt ist aus: einem Wasserstoffatom, einem aliphatischen C₁₋₆-Rest, einer Hydroxygruppe, den Resten Hydroxy-aliphatisches-C₁₋₆, Di-aliphatisches-C₁₋₆-amino, Di-aliphatisches-C₁₋₆-aminocarbonyl, Di-aliphatisches-C₁₋₆-aminosulfonyl, C₁₋₆-Alkoxy, Aryl, Aryloxy, Hydroxy-Aryl, Het, Hydroxy-Het, Het-oxy und einem Halogenatom,
wobei Aryl und Het wie nachstehend definiert sind;
R² und R³ gegebenenfalls verbunden sind, um einen kondensierten Ring, ausgewählt aus den Resten wie nachstehend für Het definiert, zu bilden, und der kondensierte Ring gegebenenfalls durch einen aliphatischen-C₁₋₆- oder aliphatischen-C₁₋₆-carbonylrest subsituiert ist;
R⁴ ausgewählt ist aus: einer Sulfonsäuregruppe, den Resten aliphatisches-C₁₋₁₂-Sulfonyl, Sulfonyl-aliphatisches-C₁₋₁₂, aliphatisches-C₁₋₁₂-Sulfonyl-aliphatisches-C₁₋₆, aliphatisches-C₁₋₆-Amino, R⁷-Sulfonyl, R⁷-Sulfonyl-aliphatisches-C₁₋₁₂, R⁷-Aminosulfonyl, R⁷-Aminosulfonyl-aliphatisches-C₁₋₁₂, R⁷-Sulfonylamino, R⁷-Sulfonylamino-aliphatisches-C₁₋₁₂, Aminosulfonylamino, Di-aliphatisches-C₁₋₁₂-amino, Di-aliphatisches-C₁₋₁₂-aminocarbonyl, Di-aliphatisches-C₁₋₁₂-aminosulfonyl, Dialiphatisches-C₁₋₁₂-aminosulfonyl-aliphatisches-C₁₋₁₂, (R⁸)₁₋₃-Arylamino, (R⁸)₁₋₃-Arylsulfonyl, (R⁸)₁₋₃-Aryl-aminosulfonyl, (R⁸)₁₋₃-Aryl-sulfonylamino, Het-amino, Het-sulfonyl, Het-aminosulfonyl, Aminoiminoamino und Aminoiminoaminosulfonyl, wobei
R⁷, R⁸, Aryl und Het wie nachstehend definiert sind;
R⁵ ein Wasserstoffatom ist;
R⁴ und R⁵ gegebenenfalls verbunden sind, um einen kondensierten Ring, ausgewählt aus den Resten wie nachstehend für Het definiert, zu bilden, und der kondensierte Ring gegebenenfalls durch die Reste Oxo oder Dioxo substituiert ist;
R⁶ ausgewählt ist aus: einem Wasserstoffatom, einem aliphatischen C₁₋₆-Rest, einer Hydroxygruppe, einem C₁₋₆-Alkoxyrest und einem Halogenatom;
R⁷ ausgewählt ist aus: einem Wasserstoffatom, den Resten aliphatisches-C₁₋₁₂, C₁₋₁₂-Alkoxy, Hydroxy-C₁₋₁₂-alkoxy, Hydroxy-aliphatisches-C₁₋₁₂, einer Carbonsäuregruppe, den Resten aliphatisches-C₁₋₁₂-Carbonyl, Het, Het-aliphatisches-C₁₋₁₂, Het-C₁₋₁₂-alkoxy, Di-Het-C₁₋₁₂-alkoxy-aryl, Aryl-aliphatisches-C₁₋₁₂, Aryl-C₁₋₁₂-alkoxy, Aryl-carbonyl, aliphatisches-C₁₋₁₈-Alkoxyalkoxyalkoxyalkoxy und einer Hydroxylgruppe, wobei Het und Aryl wie nachstehend definiert sind;
R⁸ ein Wasserstoffatom und/oder ein Rest Halogen-aliphatisches-C₁₋₆ ist;
Aryl ein Phenyl- oder Naphthylrest ist;
Cyc ausgewählt ist aus: einem Cyclopropyl-, Cyclobutyl-, Cyclopentyl-, Cyclohexyl-, Cycloheptyl- und Cyclooctylrest, und gegebenenfalls eine oder mehrere ungesättigte Stellen aufweist;
Het ein gesättigtes oder ungesättigtes Heteroatomringsystem ist, ausgewählt aus: einem Benzimidazol-, Dihydrothiophen-, Dioxin-, Dioxan-, Dioxolan-, Dithian-, Dithiazin-, Dithiazol-, Dithiolan-, Furan-, Imidazol-, Morpholin-, Oxazol-, Oxadiazol-, Oxathiazol-, Oxathiazolidin-, Oxazin-, Oxadiazin-, Piperazin-, Piperadin-, Pyran-, Pyrazin-, Pyrazol-, Pyridin-, Pyrimidin-, Pyrrol-, Pyrrolidin-, Tetrahydrofuran- Tetrazin-, Thiadiazin-, Thiadiazol-, Thiatriazol-, Thiazin-, Thiazol-, Thiomorpholin-, Thiophen-, Thiopyran-Triazin- und Triazolrest;
und Salzen und Solvaten davon.

35. Verbindung der Formel (I) gemäß Anspruch 1,
wobei
X ausgewählt ist aus: einem N-Atom, einer CH- und CCH₃-Gruppe;
Y ein C- oder N-Atom ist, mit der Maßgabe, dass, falls Y ein N-Atom ist, R¹ abwesend ist, Z und A jeweils ein C-Atom sind und D eine CH-Gruppe ist;
Z ein C- oder N-Atom ist, mit der Maßgabe, dass, falls Z ein N-Atom ist, R² abwesend ist, Y und A jeweils ein C-Atom sind und D eine CH-Gruppe ist;
A ein C- oder N-Atom ist, mit der Maßgabe, dass, falls A ein N-Atom ist, R³ abwesend ist, X und Y jeweils ein C-Atom sind und D eine CH-Gruppe ist;
D eine CH-Gruppe oder ein N-Atom ist, mit der Maßgabe, dass, falls D ein N-Atom ist, Y, Z und A dann jeweils ein C-Atom sind;
mit der weiteren Maßgabe, dass Y, Z und A und D nicht alle gleichzeitig ein C-Atom beziehungsweise eine CH-Gruppe bedeuten;
R¹ ausgewählt ist aus: einem Wasserstoffatom, den Resten aliphatisches-C₁₋₆, Hydroxyaliphatisches-C₁₋₆, Di-aliphatisches-C₁₋₆-amino, Di-aliphatisches-C₁₋₆-aminocarbonyl, Di-aliphatisches-C₁₋₆-aminosulfonyl, Aryl-aliphatisches-C₁₋₆, R⁶-Aryl-aliphatisches-C₁₋₆, Cyc-aliphatisches-C₁₋₆, Het-aliphatisches-C₁₋₆, C₁₋₆-Alkoxy, Aryloxy, Aminocarbonyl, C₁₋₆-Alkoxycarbonyl, einem Halogenatom und einer Nitrogruppe, wobei R⁶, Aryl, Cyc und Het wie nachstehend definiert sind;
R² ausgewählt ist aus: einem Wasserstoffatom, den Resten aliphatisches-C₁₋₆, N-Hydroxyimino-aliphatisches-C₁₋₆, C₁₋₆-Alkoxy, C₁₋₆-Alkoxycarbonyl, Aryl, R⁶-Aryloxycarbonyl, Het, Aminocarbonyl, aliphatisches-C₁₋₆-Aminocarbonyl, Arylaliphatisches-C₁₋₆-aminocarbonyl, R⁶-Aryl-aliphatisches-C₁₋₆-aminocarbonyl, Hetaliphatisches-C₁₋₆-aminocarbonyl, Di-aliphatisches-C₁₋₆-amino, Di-aliphatisches-C₁₋₆aminocarbonyl, Di-aliphatisches-C₁₋₆-aminosulfonyl, Hydroxy-aliphatisches-C₁₋₆aminocarbonyl, C₁₋₆-Alkoxy-aliphatisches-C₁₋₆-aminocarbonyl, C₁₋₆-Alkoxyaliphatisches-C₁₋₆-amino, einem Halogenatom, einer Hydroxy- oder Nitrogruppe, den Resten aliphatisches-C₁₋₆-Sulfonyl, Aminosulfonyl und aliphatisches-C₁₋₆-Aminosulfonyl, wobei R⁶, Aryl und Het wie nachstehend definiert sind;
R¹ und R² gegebenenfalls verbunden sind, um kondensierten Ring, ausgewählt aus den Resten wie nachstehend für Het definiert, zu bilden, und der kondensierte Ring gegebenenfalls durch ein Halogenatom und/oder einen Oxorest substituiert ist;
R³ ausgewählt ist aus: einem Wasserstoffatom, einem aliphatischen C₁₋₆-Rest, einer Hydroxygruppe, den Resten Hydroxy-aliphatisches-C₁₋₆, Di-aliphatisches-C₁₋₆-amino, Di-aliphatisches-C₁₋₆-aminocarbonyl, Di-aliphatisches-C₁₋₆-aminosulfonyl-C₁₋₆-alkoxy, Aryloxy, Het und einem Halogenatom, wobei Aryl und Het wie nachstehend definiert sind;
R² und R³ gegebenenfalls verbunden sind, um einen kondensierten Ring, ausgewählt aus den Resten wie nachstehend für Het definiert, zu bilden, und der kondensierte Ring gegebenenfalls durch einen C₁₋₆-Alkyl- und/oder C₁₋₆-Alkylcarbonylrest substituiert ist;
R⁴ ausgewählt ist aus: den Resten R⁷-Sulfonyl, R⁷-Sulfonyl-aliphatisches-C₁₋₆, aliphatisches-C₁₋₆-Sulfonyl-aliphatisches-C₁₋₆, R⁷-Aminosulfonyl, Di-aliphatisches-C₁₋₆amino, Di-aliphatisches-C₁₋₆-aminocarbonyl, Di-aliphatisches-C₁₋₆-aminosulfonyl, Dialiphatisches-C₁₋₆-aminosulfonyl-aliphatisches-C₁₋₆, R⁷-Aminosulfonyl-aliphatisches-C₁₋₆, Aminosulfonylamino, R⁷-aliphatisches-C₁₋₆-Aminosulfonyl-aliphatisches-C₁₋₆, Aryl, Het, R⁸-Aryl-aminosulfonyl, Het-aminosulfonyl und Aminoiminoaminosulfonyl,
wobei R⁷, R⁸, Aryl und Het wie nachstehend definiert sind;
R⁵ ein Wasserstoffatom ist;
R⁴ und R⁵ gegebenenfalls verbunden sind, um einen kondensierten Ring, ausgewählt aus den Resten wie nachstehend für Het definiert, zu bilden, und der kondensierte Ring gegebenenfalls durch die Reste Oxo oder Dioxo substituiert ist;
R⁶ ausgewählt ist aus: einer Hydroxygruppe, einem C₁₋₆-Alkoxyrest und einem Halogenatom;
R⁷ ausgewählt ist aus: einem Wasserstoffatom, den Resten aliphatisches-C₁₋₆, Hydroxy-C₁₋₆-alkoxy, Hydroxy-aliphatisches-C₁₋₆, aliphatisches-C₁₋₆-Carbonyl, Aryl-carbonyl,
C₁₋₁₂-Alkoxyalkoxyalkoxyalkoxyalkyl, einer Hydroxylgruppe, den Resten Aryl, Aryl-C₁₋₆-alkoxy, Aryl-aliphatisches-C₁₋₆, Het, Het-C₁₋₆-alkoxy, Di-Het-C₁₋₆-alkoxy, Hetaliphatisches-C₁₋₆ und Di-Het-aliphatisches-C₁₋₆;
R⁸ eine Trifluormethylgruppe ist;
Aryl eine Phenylgruppe ist;
Cyc eine Cyclobutylgruppe ist;
Het ein gesättigtes oder ungesättigtes Heteroatomringsystem ist, ausgewählt aus: einem Benzimidazol-, Dihydrothiophen-, Dioxolan-, Furan-, Imidazol-, Morpholin-, Oxazol-, Pyridin-, Pyrrol-, Pyrrolidin-, Thiadiazol-, Thiazol-, Thiophen-, und Triazolrest;
und Salzen und Solvaten davon.

36. Verbindung gemäß einem der Ansprüche 1 bis 35 in Form eines im wesentlichen reinen geometrischen E-Isomeren.

37. Verbindung gemäß einem der Ansprüche 1 bis 35 in Form eines im wesentlichen reinen geometrischen Z-Isomeren.

38. Verbindung gemäß einem der Ansprüche 1 bis 35 in Form eines Gemisches aus geometrischen E-Isomeren und geometrischen Z-Isomeren in beliebigen Verhältnissen der geometrischen Isomeren.

39. Verbindung gemäß Anspruch 1 in E-, Z- oder E- und Z-Form, ausgewählt aus:
4-{[(2-Oxo-1,2-dihydro-3H-pyrrolo[3,2-c]pyridin-3-yliden)methyl]amino}benzolsulfonamid;
4-{[(2-Oxo-1,2-dihydro-3H-pyrrolo[2,3-c]pyridin-3-yliden)methyl]amino}benzolsulfonamid;
3-[(1H-Indazol-6-ylamino)methyliden]-1H-pyrrolo[3,2-b]pyridin-2-on;
3-[(6-Chinolinylamino)methyliden]-1,3-dihydro-2H-pyrrolo [3,2-b] pyridin-2-on;
4-{[(2-Oxo-1,2-dihydro-3H-pyrrolo[2,3-b]pyridin-3-yliden)methyl]amino}benzolsulfonamid;
3-[(1H-Indazol-6-ylamino)methyliden]-1H-pyrrolo[2,3-b]pyridin-2-on;
3-[(6-Chinolinylamino)methyliden]-1,3-dihydro-2H-pyrrolo[2,3-b]pyridin-2-on;
4-{[(2-Oxo-5-phenyl-1,2-dihydro-3H-pyrrolo[2,3-b]pyridin-3-yliden)methyl]amino}benzolsulfonamid;
3-[(1H-Indazol-6-ylamino)methyliden]-5-phenyl-1H-pyrrolo[2,3-b]pyridin-2-on;
5-Phenyl-3-[(6-chinolinylamino)methyliden]-1,3-dihydro-2H-pyrrolo[2,3-b]pyridin-2-on;
4-({[5-(2-Furyl)-2-oxo-1,2-dihydro-3H-pyrrolo[2,3-b]pyridin-3-yliden]methyl}amino)benzolsulfonamid;
5-(2-Furyl)-3-[(1H-indazol-6-ylamino)methyliden]-1H-pyrrolo[2,3-b]pyridin-2-on;
5-(2-Furyl)-3-[(6-chinolinylamino)methyliden]-1,3-dihydro-2H-pyrrolo[2,3-b]pyridin-2-on;
4-({[2-Oxo-5-(3-thienyl)-1,2-dihydro-3H-pyrrolo[2,3-b]pyridin-3-yliden]methyl}amino)benzolsulfonamid;
3-[(lH-Indazol-6-ylamino)methyliden]-5-(3-thienyl)-1,3-dihydro-2H-pyrrolo[2,3-b]pyridin-2-on;
3-[(6-Chinolinylamino)methyliden]-5-(3-thienyl)-1,3-dihydro-2H-pyrrolo[2,3-b]pyridin-2-on;
4-{[(5-Brom-2-oxo-1,2-dihydro-3H-pyrrolo[2,3-b]pyridin-3-yliden)methyl]amino}benzolsulfonamid;
5-Brom-3-[(1H-indazol-6-ylamino)methyliden]-1,3-dihydro-2H-pyrrolo[2,3-b]pyridin-2-on;
5-Brom-3-[(6-chinolinylamino)methyliden]-1,3-dihydro-2H-pyrrolo[2,3-b]pyridin-2-on; 4-{[(6-Chlor-2-oxo-1,2-dihydro-3H-pyrrolo[2,3-b]pyridin-3-yliden)methyl]amino}benzolsulfonamid;
6-Chlor-3-[(1H-indazol-6-ylamino)methyliden]-1,3-dihydro-2H-pyrrolo[2,3-b]pyridin-2-on;
6-Chlor-3-[(6-chinolinylamino)methyliden]-1,3-dihydro-2H-pyrrolo[2,3-b]pyridin-2-on; Ethyl-3-{[4-(aminosulfonyl)anilino]methyliden}-2-oxo-1,2-dihydro-3H-pyrrolo[2,3-b]pyridin-5-carboxylat;
Ethyl-3-[(1H-indazol-6-ylamino)methyliden]-2-oxo-1,2-dihydro-3H-pyrrolo[2,3-b]pyridin-5-carboxylat; und
Ethyl-2-oxo-3-[(6-chinolinylamino)methyliden]-2,3-dihydro-1H-pyrrolo[2,3-b]pyridin-5-carboxylat;
und pharmazeutisch verträglichen Salzen und Solvaten davon.

40. Arzneimittel, umfassend eine Verbindung nach einem der Ansprüche 1 bis 39 und einen oder mehrere pharmazeutisch verträgliche Träger, Verdünnungsmittel und/oder Excipienten.

41. Verbindung nach einem der Ansprüche 1 bis 39 zur Verwendung in der Therapie.

42. Verwendung einer Verbindung nach einem der Ansprüche 1 bis 39 zur Herstellung eines Medikaments zur Behandlung einer Erkrankung oder Störung bei einem Tier, wobei die Erkrankung oder Störung mindestens einen Zustand, ausgewählt aus Abstoßung eines transplantierten Organs, Tumorwachstum, Chemotherapie-induzierter Alopezie, Chemotherapie-induzierter Thrombozytopenie, Chemotherapie-induzierter Leukopenie, Mukositis, Plantar-Palmar-Syndrom, Restenose, Arteriosklerose, rheumatische Arthritis, Angiogenese, Leberzirrhose, Glomerulonephritis, diabetische Nephrophatie, bösartige Nephrosklerose, thrombotische Mikroangiopathie, Glomerulopathie, Psoriasis, Diabetes mellitus, Entzündung, neurodegenerative Erkrankung, Makuladegeneration, aktinische Keratose und hyperproliferative Störungen, umfasst.

43. Verwendung einer Verbindung nach einem der Ansprüche 1 bis 39 zur Herstellung eines Medikaments zur Behandlung einer Erkrankung oder Störung bei einem Tier, wobei die Erkrankung oder Störung eine Virusinfektion oder eukaryontische Infektion umfasst.

44. Verwendung einer Verbindung nach einem der Ansprüche 1 bis 39 zur Herstellung eines Medikaments zur Behandlung einer Erkrankung oder Störung bei einem Tier, wobei die Erkrankung oder Störung Alopezie umfasst.

45. Verwendung einer Verbindung nach einem der Ansprüche 1 bis 39 zur Herstellung eines Medikaments zur Behandlung einer Erkrankung oder Störung bei einem Tier, wobei die Erkrankung oder Störung mindestens einen durch eine Kinase vermittelten Zustand umfasst.

46. Verwendung gemäß Anspruch 45, wobei die Kinase eine mitogenetisch aktivierte Proteinkinase ist.

47. Verwendung gemäß Anspruch 45, wobei die Kinase ausgewählt ist aus: abl, ARaf, ATK, ATM, bcr-abl, Blk, BRaf, Brk, Btk, CDK1, CDK2, CDK3, CDK4, CDK5, CDK6, CDK7, CDK8, CDK9, c-fms, c-kit, c-met, cRafl, CSF1R, CSK, c-src, EGFR, ErbB2, ErbB3, ErbB4, ERK, ERK1, ERK2, Fak, fes, FGFR1, FGFR2, FGFR3, FGFR4, FGFR5, Fgr, FLK-4, Fps, Frk, Fyn, GSK, gsk3a, gsk3b, Hck, IGF-1R, IKK, IKK1, IKK2, IKK3, INS-R, Integrin-verbundene Kinase, JAK, JAK1, JAK2, JAK3, JNK, Lck, Lyn, MEK, MEK1, MEK2, p38, PDGFR, PIK, PKB1, PKB2, PKB3, PKC, PKCa, PKCb, PKCd, PKCe, PKCg, PKCl, PKCm, PKCz, PLK1, Polo-artige Kinase, PYK2, tie₁, tie₂, TrkA, TrkB, TrkC, UL13, UL97, VEGF-R1, VEGF-R2, Yes und Zap70.

48. Verwendung gemäß Anspruch 45, wobei die Kinase ausgewählt ist aus: VEGF-R2, CDK1, CDK2, CDK3, Zap70, Lck und c-fms.

49. Verwendung gemäß Anspruch 45, wobei die Kinase VEGF-R2 ist.

50. Verwendung gemäß einem der Ansprüche 42 bis 49, wobei die Verbindung oder ein physiologisch verträgliches Salz davon in einer Menge von etwa 0,1 bis etwa 100 mg/kg Körpergewicht pro Tag verabreicht wird.

51. Verwendung gemäß Anspruch 50, wobei die Verbindung oder ein physiologisch verträgliches Salz davon in einer Menge von etwa 0,1 bis etwa 10 mg/kg Körpergewicht pro Tag verabreicht wird.

52. Verwendung gemäß einem der Ansprüche 42 bis 51, wobei das Tier ein Mensch ist.

53. Verwendung gemäß einem der Ansprüche 42 bis 52, wobei die Verbindung zusammen mit einem oder mehreren Mitteln und/oder Behandlungen gegen Krebs verabreicht wird.

## Revendications

1. Composé de formule : 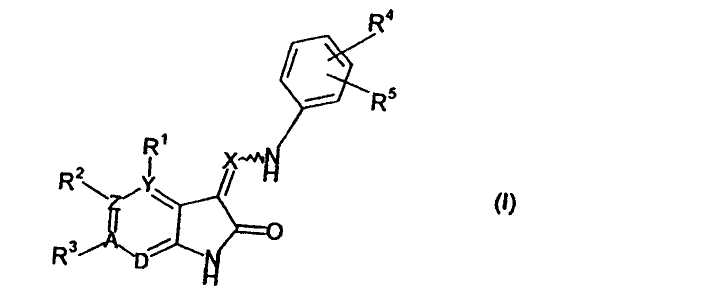 dans laquelle :
X est choisi dans le groupe consistant en N, CH, CCF₃ et C(radical aliphatique en C₁ à C₁₂) ;
Y représente C ou N, sous réserve que, lorsque Y représente N, R¹ soit absent, Z et A représentent chacun C, et D représente un groupe CH ;
Z représente C ou N, sous réserve que, lorsque Z représente N, R² soit absent, Y et A représentent chacun C, et D représente un groupe CH ;
A représente C ou N, sous réserve que, lorsque A représente N, R³ soit absent, Y et Z représentent chacun C, et D représente un groupe CH ;
D représente CH ou N, sous réserve que, lorsque D représente N, alors Y, Z et A représentent chacun C ;
sous réserve en outre que Y, Z et A et D ne représentent pas tous simultanément respectivement C et un groupe CH ;
R¹ est choisi dans le groupe consistant en : un atome d'hydrogène, des groupes aliphatiques en C₁ à C₁₂, thiol, hydroxy, hydroxy(aliphatique en C₁ à C₁₂), aryle, aryle(aliphatique en C₁ à C₁₂), R⁶-aryle(aliphatique en C₁ à C₁₂), Cyc, Cyc(aliphatique en C₁ à C₆), Het, Het(aliphatique en C₁ à C₁₂), alkoxy en C₁ à C₁₂, aryloxy, amino, (aliphatique en C₁ à C₁₂)amino, di(aliphatique en C₁ à C₁₂) amino, di(aliphatique en C₁ à C₁₂)aminocarbonyle, di(aliphatique en C₁ à C₁₂)aminosulfonyle, (alkoxy en C₁ à C₁₂)carbonyle, halogéno, cyano, sulfonamide et nitro, dans lesquels R⁶, aryle, Cyc et Het répondent aux définitions ci-dessous ;
R² est choisi dans le groupe consistant en : un atome d'hydrogène, des groupes aliphatiques en C₁ à C₁₂, N-hydroxyimino(aliphatique en C₁ à C₁₂), alkoxy en C₁ à C₁₂, hydroxy(aliphatique en C₁ à C₁₂), (alkoxy en C₁ à C₁₂)carbonyle, carboxyle(aliphatique en C₁ à C₁₂), aryle, R⁶-aryloxycarbonyle, R⁶-oxycarbonylaryle, Het, aminocarbonyle, (aliphatique en C₁ à C₁₂)aminocarbonyle, aryle(aliphatique en C₁ à C₁₂)aminocarbonyle, R⁶-aryle(aliphatique en C₁ à C₁₂)aminocarbonyle, Het-(àliphatique en C₁ à C₁₂)aminocarbonyle, hydroxy(aliphatique en C₁ à C₁₂)aminocarbonyle, (alkoxy en C₁ à C₁₂) (aliphatique en C₁ à C₁₂)aminocarbonyle, (alkoxy en C₁ à C₁₂) (aliphatique en C₁ à C₁₂)amino, di(aliphatique en C₁ à C₁₂)amino, di(aliphatique en C₁ à C₁₂)aminocarbonyle, di(aliphatique en C₁ à C₁₂)aminosulfonyle, halogéno, hydroxy, nitro, (aliphatique en C₁ à C₁₂) sulfonyle, aminosulfonyle et (aliphatique en C₁ à C₁₂)aminosulfonyle, dans lesquels R⁶, aryle et Het répondent aux définitions ci-dessous ;
R¹ et R² sont facultativement joints pour former un noyau condensé choisi dans le groupe répondant à la définition mentionnée pour Het ci-dessous, et ledit noyau condensé est facultativement substitué avec un ou plusieurs substituants choisis dans le groupe consistant en les substituants : aliphatique en C₁ à C₁₂, halogéno, nitro, cyano, alkoxy en C₁ à C₁₂, carbonyle(alkoxy en C₁ à C₁₂) et oxo ;
R³ est choisi dans le groupe consistant en : un atome d'hydrogène, des groupes aliphatiques en C₁ à C₁₂, hydroxy, hydroxy(aliphatique en C₁ à C₁₂), di(aliphatique en C₁ à C₁₂)amino, di(aliphatique en C₁ à C₁₂)aminocarbonyle, di(aliphatique en C₁ à C₁₂)aminosulfonyle, alkoxy en C₁ à C₁₂, aryle, aryloxy, hydroxyaryle, Het, hydroxy-Het, Het-oxy et halogéno, dans lesquels les termes aryle et Het répondent aux définitions ci-dessous ;
R² et R³ sont facultativement joints pour former un noyau condensé choisi dans le groupe répondant à la définition pour Het ci-dessous, et ledit noyau,condensé est facultativement substitué avec un substituant aliphatique en C₁ à C₆ et/ou (aliphatique en C₁ à C₆)carbonyle ;
R⁴ est choisi dans le groupe consistant en des groupes : 'acide sulfonique, (aliphatique en C₁ à C₁₂)-sulfonyle, sulfonyle(aliphatique en C₁ à C₁₂), (aliphatique en C₁ à C₁₂)sulfonyle(aliphatique en C₁ à C₆), (aliphatique en C₁ à C₆) amino, R⁷-sulfonyle, R⁷-sulfonyle(aliphatique en C₁ à C₁₂), R⁷-aminosulfonyle, R⁷-aminosulfonyle(aliphatique en C₁ à C₁₂), R⁷-sulfonylamino, R⁷-sulfonylamino(aliphatique en C₁ à C₁₂), aminosulfonylamino, di(aliphatique en C₁ à C₁₂)amino, di(aliphatique en C₁ à C₁₂)aminocarbonyle, di(aliphatique en C₁ à C₁₂)aminosulfonyle, di(aliphatique en C₁ à C₁₂)aminosulfonyle(aliphatique en C₁ à C₁₂), (R⁸)₁₋₃arylamino, (R⁸)₁₋₃arylsulfonyle, (R⁸)₁₋₃-arylaminosulfonyle, (R⁸)₁₋₃-arylsulfonylamino, Het-amino, Het-sulfonyle, Het-aminosulfonyle, amino-iminoamino et amino-iminoaminosulfonyle, dans lesquels R⁷, R⁸, aryle et Het répondent aux définitions ci-dessous ;
R⁵ représente un atome d'hydrogène, ou bien R⁴ et R⁵ sont facultativement joints pour former un noyau condensé choisi dans le groupe répondant à la définition mentionnée pour Het ci-dessous, et ledit noyau condensé est facultativement substitué avec un ou plusieurs substituants choisis dans le groupe consistant en les substituants : aliphatique en C₁ à C₁₂, oxo et dioxo ;
R⁶ est choisi dans le groupe consistant en des groupes : aliphatique en C₁ à C₁₂, hydroxy, alkoxy en C₁ à C₁₂ et halogéno ;
R⁷ est choisi dans le groupe consistant en : un atome d'hydrogène, des groupes aliphatiques en C₁ à C₁₂, alkoxy en C₁ à C₁₂, hydroxyalkoxy en C₁ à C₁₂, hydroxy(aliphatique en C₁ à C₁₂), acide carboxylique, (aliphatique en C₁ à C₁₂)carbonyle, Het, Het(aliphatique en C₁ à C₁₂), Het(alkoxy en C₁ à C₁₂), di-Het(alkoxy en C₁ à C₁₂)aryle, aryle(aliphatique en C₁ à C₁₂), aryle(alkoxy en C₁ à C₁₂), arylcarbonyle, (alkoxy en C₁ à C₁₈)oxyalkoxyalkoxyaliphatique et hydroxyle, dans lesquels Het et aryle répondent aux définitions mentionnées ci-dessous ;
R⁸ est choisi dans le groupe consistant en : un atome d'hydrogène, des groupes nitro, cyano, alkoxy en C₁ à C₁₂, halo, carbonyle(alkoxy en C₁ à C₁₂) et halogéno-aliphatique en C₁ à C₁₂ ;
le groupe aryle est choisi dans le groupe consistant en les groupes : phényle, naphtyle, phénanthryle et anthracényle ;
Cyc est choisi dans le groupe consistant en les groupes : cyclopropyle, cyclobutyle, cyclopentyle, cyclohexyle, cycloheptyle et cyclooctyle, et possède facultativement un ou plusieurs degrés d'insaturation ;
Het représente un système de noyau hétérocyclique saturé ou insaturé choisi dans le groupe consistant en : benzimidazole, dihydrothiophène, dioxine, dioxanne, dioxolanne, dithiane, dithiazine, dithiazole, dithiolane, furanne, imidazole, isoquinoléine, morpholine, oxazole, oxadiazole, oxathiazole, oxathiazolidine, oxazine, oxadiazine, pipérazine, pipéradine, pyranne, pyrazine, pyrazole, pyridine, pyrimidine, pyrrole, pyrrolidine, quinoléine, tétrahydrofuranne, tétrazine, thidiazine, thiadiazole, thiatriazole, thiazine, thiazole, thiomorpholine, thiophène, thiopyranne, triazine et triazole ;
et ses sels et produits de solvatation.

2. Composé suivant la revendication 1, dans lequel X est choisi dans le groupe consistant en : N, CH et CCH₃.

3. Composé suivant la revendication 1, dans lequel X représente un groupe CH.

4. Composé suivant la revendication 1, dans lequel X représente en groupe CCH₃.

5. Composé suivant la revendication 1, dans lequel X réprésente N.

6. Composé suivant la revendication 1, dans lequel Y représente C.

7. Composé suivant la revendication 1, dans lequel Y représente N, Z et A représentent chacun C, et D représente un groupe CH.

8. Composé suivant la revendication 1, dans lequel Z représente C.

9. Composé suivant la revendication 1, dans lequel Z représente N, Y et A représentent chacun C, et D représente un groupe CH.

10. Composé suivant la revendication 1, dans lequel A représente C.

11. Composé suivant la revendication 1, dans lequel A représente N, Y et Z représentent chacun C, et D représente un groupe CH.

12. Composé suivant la revendication 1, dans lequel D représente un groupe CH.

13. Composé suivant la revendication 1, dans lequel D représente N et Y, Z et A représentent chacun C.

14. Composé suivant la revendication 1, dans lequel R¹ est choisi dans le groupe consistant en : un atome d'hydrogène, des groupes halogéno, amide, nitro, alkyle en C₁ à C₆, hydroxy, hydroxyalkyle, pyrimidine(alkyle en C₁ à C₆), (alkoxy en C₁ à C₆)carbonyle, cycloalkyle en C₃ à C₆, hydroxyphényle(alkyle en C₁ à C₆), phénoxy, alkoxy et pyrazole.

15. Composé suivant la revendication 1, dans lequel R¹ représente un groupe d'hydrogène ou méthyle.

16. Composé suivant la revendication 1, dans lequel R¹ représente un atome d'hydrogène.

17. Composé suivant la revendication 1, dans lequel R¹ est condensé avec R² pour former un noyau condensé choisi dans le groupe consistant en : thiazole, pyrazole, triazole, diazole à substituant halogéno, pyrrole à substituant acyle et pyridine.

18. Composé suivant la revendication 1, dans lequel R¹ est condensé avec R² pour former un groupe thiazole condensé ou pyridine condensé.

19. Composé suivant la revendication 1, dans lequel R² est choisi dans le groupe consistant en : un atome d'hydrogène, des groupes halogéno, sulfate, amine, amine quaternaire, amide, ester, phényle, alkoxy, aminosulfonyle, alkylsulfonyle en C₁ à C₆, furannyle(alkyle en C₁ à C₆)amide, pyridinyle(alkyle en C₁ à C₆)amide, phényle(alkyle en C₁ à C₆)amide à substituant alkoxy, morpholino(alkyle en C₁ à C₆)amide, imidazolyle(alkyle en C₁ à C₆)amide, hydroxy(alkyle en C₁ à C₆)amide, alkoxy(alkyle en C₁ à C₆)amide, (alkyle en C₁ à C₆)amide, (alkyle en C₁ à C₆)sulfonamide, amino à substituant (alkyle en C₁ à C₆)-hydroxy, nitro, phénoxycarbonyle à substituant halogéno et des noyaux triazole et oxazole.

20. Composé suivant la revendication 1, dans lequel R³ est choisi dans le groupe consistant en : un atome d'hydrogène, des groupes alkyle en C₁ à C₆, hydroxyalkyle en C₁ à C₆, halogéno, phénoxy et alkoxy.

21. Composé suivant la revendication 1, dans lequel R² est choisi dans le groupe consistant en : un atome d'hydrogène, les groupes phényle, 2-furannyle, 3-thiophényle, bromo et carbéthoxy.

22. Composé suivant la revendication 1, dans lequel R² est condensé avec R³ pour former un noyau condensé choisi dans le groupe consistant en : oxazole, pyrrole et dioxolanne, noyau condensé qui est facultativement substitué avec un substituant alkyle en C₁ à C₆ ou (alkyle en C₁ à C₆)carbonyle, et noyau condensé qui est facultativement un noyau hétérocyclique comprenant de l'azote comme hétéroatome et formant un sel d'ammonium quaternaire ioniquement lié avec un atome d'halogène.

23. Composé suivant la revendication 1, dans lequel R³ représente un groupe hydrogène ou un atome chloro.

24. Composé suivant la revendication 1, dans lequel R³ représente un atome d'hydrogène.

25. Composé suivant la revendication 1, dans lequel R⁴ est choisi dans le groupe consistant en des groupes : sulfonylamino, sulfonylaminoamino, (alkyle en C₁ à C₆)sulfonylamino, (alkyle en C₁ à C₆)sulfonyle(alkyle en C₁ à C₆), alkoxysulfonylamino, phénylcarbonylsulfonylamino, phénoxysulfonyle, hydroxy(alkyle en C₁ à C₆)sulfonylamino, hydroxy(alkyle en C₁ à C₆)sulfonylamino(alkyle en C₁ à C₆), alkyle, phénylsulfonylamino (facultativement substitué avec un groupe alkyle en C₁ à C₆ à substituant halogéno), aminoiminosulfonylamino, alkylsulfonylaminoalkyle, pyridinyle(alkyle en C₁ à C₆)sulfonylamino, benzamideazolesulfonylamino, pyridylsulfonylamino, pyrimidinylsulfonylamino, thiadiazolylsulfonylamino (facultativement substitué avec un substituant alkyle en C₁ à C₆), thiazolesulfonylamino, hydroxyalkoxyalkylsulfonylamino et 4'-SO₂NH[(CH₂)₂O]₄CH₃.

26. Composé suivant la revendication 1, dans lequel R⁴ est choisi dans le groupe consistant en des groupes : 2-pyridinesulfonylamino, 4-pyridinesulfonylamino, hydroxy-n-butylsulfonylamino, méthylsulfonylaminoéthylène, sulfonyldiméthylamino, 1,2-pyrazole condensé et sulfonylamino.

27. Composé suivant la revendication 1, dans lequel R⁴ représente un groupe sulfonylamino ou 1,2-pyrazole condensé.

28. Composé suivant la revendication 1, dans lequel R⁴ est condensé avec R⁵ pour former un noyau condensé choisi dans le groupe consistant en imidazole, triazole, sulfonylamino cyclique et thiophène, ledit noyau condensé étant facultativement disubstitué sur l'hétéroatome de soufre avec un groupe oxo.

29. Composé suivant la revendication 1, dans lequel R⁵ représente un atome d'hydrogène.

30. Composé suivant la revendication 1, dans lequel R⁶ est choisi dans le groupe consistant en : un atome d'hydrogène, des groupes aliphatiques en C₁ à C₆, hydroxy, alkoxy en C₁ à C₆ et halogéno.

31. Composé suivant la revendication 1, dans lequel R⁶ est choisi dans le groupe consistant en des groupes : hydroxy, alkoxy en C₁ à C₆ et halogéno.

32. Composé suivant la revendication 1, dans lequel R⁸ représente un atome d'hydrogène ou un groupe halogéno-aliphatique en C₁ à C₆.

33. Composé suivant la revendication 1, dans lequel R⁸ représente un groupe trifluorométhyle.

34. Composé de formule (I) suivant la revendication 1,
dans lequel
X est choisi dans le groupe consistant en : N, CH et C(aliphatique en C₁ à C₆) ;
Y représente C ou N, sous réserve que, lorsque Y représente N, R¹ soit absent, Z et A représentent chacun C, et D représente un groupe CH ;
Z représente C ou N, sous réserve que, lorsque Z représente N, R² soit absent, Y et A représentent chacun C et D représente un groupe CH ;
A représente C ou N, sous réserve que, lorsque A représente N, R³ soit absent, Y et Z représentent chacun C et D représente un groupe CH ;
D représente CH ou N, sous réserve que, lorsque D représente N, alors Y, Z et A représentent C ;
sous réserve en outre que Y, Z et A, et D ne représentent pas tous simultanément, respectivement, C et un groupe CH ;
R¹ est choisi dans le groupe consistant en : un atome d'hydrogène, des groupes aliphatique en C₁ à C₆, hydroxyaliphatique en C₁ à C₆, aryle(aliphatique en C₁ à C₆), R⁶-aryle(aliphatique en C₁ à C₆), Cyc(aliphatique en C₁ à C₆), Het(aliphatique en C₁ à C₆), alkoxy en C₁ à C₆, aryloxy, aminocarbonyle, di(aliphatique en C₁ à C₆)amino, di(aliphatique en C₁ à C₆) aminocarbonyle, di(aliphatique en C₁ à C₆)aminosulfonyle, (alkoxy en C₁ à C₆)carbonyle, halogéno et nitro, dans lesquels R⁶, aryle, Cyc et Het répondent aux définitions ci-dessous ;
R² est choisi dans le groupe consistant en : un atome d'hydrogène, des groupes aliphatiques en C₁ à C₆, R⁷(aliphatique en C₁ à C₆), alkoxy en C₁ à C₆, hydroxyaliphatique en C₁ à C₆, (alkoxy en C₁ à C₆)carbonyle, carboxyle(aliphatique en C₁ à C₆), aryle, R⁶-aryloxycarbonyle, R⁶-oxycarbonylaryle, Het, aminocarbonyle, (aliphatique en C₁ à C₆)aminocarbonyle, aryle(aliphatique en C₁ à C₆)aminocarbonyle, R⁶-aryle(aliphatique en C₁ à C₆)aminocarbonyle, Het(aliphatique en C₁ à C₆)aminocarbonyle, hydroxy(aliphatique en C₁ à C₆)aminocarbonyle, (alkoxy en C₁ à C₆)(aliphatique en C₁ à C₆)aminocarbonyle, (alkoxy en C₁ à C₆) (aliphatique en C₁ à C₆)amino, di(aliphatique en C₁ à C₆)amino, di(aliphatique en C₁ à C₆)aminocarbonyle, di(aliphatique en C₁ à C₆)aminosulfonyle, halogéno, hydroxy, nitro, sulfo, (aliphatique en C₁ à C₆)sulfonyle, aminosulfonyle, (aliphatique en C₁ à C₆)aminosulfonyle et ammonium quaternaire, dans lesquels R⁶, R⁷, aryle et Het répondent aux définitions ci-dessous ;
R¹ et R² sont facultativement joints pour former un noyau condensé choisi dans le groupe tel que défini pour Het ci-dessous, et ledit noyau condensé est facultativement substitué avec un substituant halogéno et/ou oxo ;
R³ est choisi dans le groupe consistant en : un atome d'hydrogène, des groupes aliphatiques en C₁ à C₆, hydroxy, hydroxyaliphatique en C₁ à C₆, di(aliphatique en C₁ à C₆)amino, di(aliphatique en C₁ à C₆)aminocarbonyle, di(aliphatique en C₁ à C₆)aminosulfonyle, alkoxy en C₁ à C₆, aryle, aryloxy, hydroxyaryle, Het, hydroxy-Het, Het-oxy et halogéno, dans lesquels aryle et Het répondent aux définitions ci-dessous ;
R² et R³ sont facultativement joints pour former un noyau condensé choisi dans le groupe tel que défini pour Het ci-dessous, et ledit noyau condensé est facultativement substitué avec un substituant aliphatique en C₁ à C₆ ou (aliphatique en C₁ à C₆)carbonyle ;
R⁴ est choisi dans le groupe consistant en des groupes : acide sulfonique, (aliphatique en C₁ à C₁₂)sulfonyle, sulfonyle(aliphatique en C₁ à C₁₂), (aliphatique en C₁ à C₁₂)sulfonyle(aliphatique en C₁ à C₆), (aliphatique en C₁ à C₆)amino, R⁷-sulfonyle, R⁷-sulfonyle(aliphatique en C₁ à C₁₂), R⁷-aminosulfonyle, R⁷-aminosulfonyle (aliphatique en C₁ à C₁₂), R⁷-sulfonylamino, R⁷-sulfonylamino(aliphatique en C₁ à C₁₂), aminosulfonylamino, di(aliphatique en C₁ à C₁₂)amino, di(aliphatique en C₁ à C₁₂)aminocarbonyle, di(aliphatique en C₁ à C₁₂)aminosulfonyle, di(aliphatique en C₁ à C₁₂)aminosulfonyle(aliphatique en C₁ à C₁₂), (R⁸)₁₋₃-arylamino, (R⁸)₁₋₃-arylsulfonyle, (R⁸)₁₋₃-arylaminosulfonyle, (R⁸)₁₋₃-arylsulfonylamino, Het-amino, Het-sulfonyle, Het-aminosulfonyle, amino-iminoamino et amino-iminoaminosulfonyle, dans lesquels R⁷, R⁸, aryle et Het répondent aux définitions ci-dessous ;
R⁵ représente un atome d'hydrogène ;
R⁴ et R⁵ sont facultativement joints pour former un noyau condensé choisi dans le groupe tel que défini pour Het ci-dessous, et ledit noyau condensé est facultativement substitué avec un substituant oxo ou dioxo ;
R⁶ est choisi dans le groupe consistant en : un atome d'hydrogène, des groupes aliphatiques en C₁ à C₆, hydroxy, alkoxy en C₁ à C₆ et halogéno ;
R⁷ est choisi dans le groupe consistant en : un atome d'hydrogène, des groupes aliphatiques en C₁ à C₁₂, alkoxy en C₁ à C₁₂, hydroxyalkoxy en C₁ à C₁₂, hydroxyaliphatique en C₁ à C₁₂), acide carboxylique, (aliphatique en C₁ à C₁₂)carbonyle, Het, Het(aliphatique en C₁ à C₁₂), Het(alkoxy en C₁ à C₁₂), di-Het(alkoxy en C₁ à C₁₂)aryle, aryle(aliphatique en C₁ à C₁₂), aryle(alkoxy en C₁ à C₁₂), arylcarbonyle, (alkoxy en C₁ à C₁₈)alkoxyalkoxyalkoxyaliphatique et hydroxyle, dans lesquels Het et aryle répondent aux définitions ci-dessous ;
R⁸ représente un atome d'hydrogène et/ou un groupe halogéno(aliphatique en C₁ à C₆) ;
aryle représente un groupe phényle ou naphtyle ;
Cyc est choisi dans le groupe consistant en les groupes : cyclopropyle, cyclobutyle, cyclopentyle, cyclohexyle, cycloheptyle et cyclooctyle, et possède facultativement un ou plusieurs degrés d'insaturation ;
Het représente un système hétérocyclique saturé ou insaturé choisi dans le groupe consistant en : benzimidazole, dihydrothiophène, dioxine, dioxanne, dioxolanne, dithiane, dithiazine, dithiazole, dithiolane, furanne, imidazole, morpholine, oxazole, oxadiazole, oxathiazole, oxathiozolidine, oxazine, oxadiazine, pipérazine, pipéradine, pyranne, pyrazine, pyrazole, pyridine, pyrimidine, pyrrole, pyrrolidine, tétrahydrofuranne, tétrazine, thiadiazine, thiadiazole, thiatriazole, thiazine, thiazole, thiomorpholine, thiophène, thiopyranne, triazine et triazole ;
et ses sels et produits de solvatation.

35. Composé de formule (I) suivant la revendication 1
dans lequel
X est choisi dans le groupe consistant en : N, CH et CCH₃ ;
Y représente C ou N, sous réserve que, lorsque Y représente N, R¹ soit absent, Z et A représentent chacun C et D représente un groupe CH ;
Z représente C ou N, sous réserve que, lorsque Z représente N, R² soit absent, Y et A représentent chacun C et D représente un groupe CH ;
A représente C ou N, sous réserve que, lorsque A représente N, R³ soit absent, X et Y représentent chacun C et D représente un groupe CH ;
D représente un groupe CH ou N, sous réserve que, lorsque D représente N, alors Y, Z et A représentent chacun C ;
sous réserve en outre que Y, Z, A et D, ne représentent pas tous simultanément, respectivement, C et un groupe CH ;
R¹ est choisi dans le groupe consistant en : un atome d'hydrogène, des groupes aliphatiques en C₁ à C₆, hydroxyaliphatique en C₁ à C₆, di(aliphatique en C₁ à C₆)amino, di(aliphatique en C₁ à C₆)aminocarbonyle, di(aliphatique en C₁ à C₆)aminosulfonyle, aryle(aliphatique en C₁ à C₆), R⁶-aryle(aliphatique en C₁ à C₆), Cyc(aliphatique en C₁ à C₆), Het(aliphatique en C₁ à C₆), alkoxy en C₁ à C₆, aryloxy, aminocarbonyle, (alkoxy en C₁ à C₆)carbonyle, halogéno et nitro, dans lesquels R⁶, aryle, Cyc et Het répondent aux définitions ci-dessous ;
R² est choisi dans le groupe consistant en : un atome d'hydrogène, des groupes aliphatiques en C₁ à C₆, N-hydroxyimino(aliphatique en C₁ à C₆), alkoxy en C₁ à C₆, (alkoxy en C₁ à C₆)carbonyle, aryle, R⁶-aryloxycarbonyle, Het, aminocarbonyle, (aliphatique en C₁ à C₆)aminocarbonyle, aryle(aliphatique en C₁ à C₆)aminocarbonyle, R⁶-aryle(aliphatique en C₁ à C₆)aminocarbonyle, Het(aliphatique en C₁ à C₆)aminocarbonyle, di(aliphatique en C₁ à C₆)amino, di(aliphatique en C₁ à C₆)aminocarbonyle, di(aliphatique en C₁ à C₆)aminosulfonyle, hydroxy(aliphatique en C₁ à C₆)aminocarbonyle, (alkoxy en C₁ à C₆) (aliphatique en C₁ à C₆)aminocarbonyle, (alkoxy en C₁ à C₆) (aliphatique en C₁ à C₆)amino, halogéno, hydroxy, nitro, (aliphatique en C₁ à C₆)sulfonyle, aminosulfonyle et (aliphatique en C₁ à C₆)aminosulfonyle, dans lesquels R⁶, aryle et Het répondent aux définitions ci-dessous ;
R¹ et R² sont facultativement joints pour former un noyau condensé choisi dans le groupe tel que défini pour Het ci-dessous, et ledit noyau condensé est facultativement substitué avec un substituant halogéno et/ou oxo ;
R³ est choisi dans le groupe consistant en : un atome d'hydrogène, des groupes aliphatiques en C₁ à C₆, hydroxy, hydroxyaliphatique en C₁ à C₆, di(aliphatique en C₁ à C₆)amino, di(aliphatique en C₁ à C₆)aminocarbonyle, di(aliphatique en C₁ à C₆)aminosulfonyle (alkoxy en C₁ à C₆), aryloxy, Het et halogéno, dans lesquels aryle et Het répondent aux définitions ci-dessous ;
R² et R³ sont facultativement joints pour former un noyau condensé choisi dans le groupe tel que défini pour Het ci-dessous, et ledit noyau condensé est facultativement substitué avec un substituant alkyle en C₁ à C₆ et/ou (alkyle en C₁ à C₆)carbonyle ;
R⁴ est choisi dans le groupe consistant en des groupes : R⁷-sulfonyle, R⁷-sulfonyle(aliphatique en C₁ à C₆), (aliphatique en C₁ à C₆)sulfonyle (aliphatique en C₁ à C₆), R⁷-aminosulfonyle, di(aliphatique en C₁ à C₆) amino, di(aliphatique en C₁ à C₆)aminocarbonyle, di(aliphatique en C₁ à C₆)aminosulfonyle, di(aliphatique en C₁ à C₆)aminosulfonyle (aliphatique en C₁ à C₆), R⁷-aminosulfonyle(aliphatique en C₁ à C₆), aminosulfonylamino, R⁷ (aliphatique en C₁ à C₆)aminosulfonyle(aliphatique en C₁ à C₆), aryle, Het, R⁸-arylaminosulfonyle, Het-aminosulfonyle et amino-iminoaminosulfonyle, dans lesquels R⁷, R⁸, aryle et Het répondent aux définitions ci-dessous ;
R⁵ représente un atome d'hydrogène ;
R⁴ et R⁵ sont facultativement joints pour former un noyau condensé choisi dans le groupe tel que défini pour Het ci-dessous, et ledit noyau condensé est facultativement substitué avec un substituant oxo ou dioxo ;
R⁶ est choisi dans le groupe consistant en des groupes : hydroxy, alkoxy en C₁ à C₆ et halogéno ;
R⁷ est choisi dans le groupe consistant en : un atome d'hydrogène, des groupes aliphatiques en C₁ à C₆, hydroxyalkoxy en C₁ à C₆, hydroxyaliphatique en C₁ à C₆, (aliphatique en C₁ à C₆)carbonyle, arylcarbonyle, (alkoxy en C₁ à C₁₂)alkoxyalkoxyalkoxyalkyle, hydroxyle, aryle, aryle(alkoxy en C₁ à C₆), aryle(aliphatique en C₁ à C₆), Het, Het(alkoxy en C₁ à C₆), di-Het(alkoxy en C₁ à C₆), Het(aliphatique en C₁ à C₆) et di-Het(aliphatique en C₁ à C₆) ;
R⁸ représente un groupe trifluorométhyle ;
le groupe aryle est un groupe phényle ;
Cyc est un groupe cyclobutyle ;
Het représente un système de noyau hétérocyclique saturé ou insaturé choisi dans le groupe consistant en : benzimidazole, dihydrothiophène, dioxolanne, furanne, imidazole, morpholine, oxazole, pyridine, pyrrole, pyrrolidine, thiadiazole, thiazole, thiophène et triazole ;
et ses sels et produits de solvatation.

36. Composé suivant l'une quelconque des revendications 1 à 35, sous forme d'un isomère géométrique E substantiellement pur.

37. Composé suivant l'une quelconque des revendications 1 à 35, sous forme d'un isomère géométrique Z substantiellement pur.

38. Composé suivant l'une quelconque des revendications 1 à 35, sous forme d'un mélange d'un isomère géométrique E et d'un isomère géométrique Z dans n'importe quelles proportions desdits isomères géométriques.

39. Composé suivant la revendication 1, sous forme E, Z ou E et Z, choisi dans le groupe consistant en :
4-{[(2-oxo-1,2-dihydro-3H-pyrrolo[3,2-c]pyridine-3-ylidène)méthyl]amino}benzènesulfonamide ;
4-{[(2-oxo-1,2-dihydro-3H-pyrrolo[2,3-c]pyridine-3-ylidène)méthyl]amino}benzènesulfonamide ;
3-[(1H-indazole-6-ylamino)méthylidène]-1H-pyrrolo[3,2-b]pyridine-2-one ;
3-[(6-quinolinylamino)méthylidène]-1,3-dihydro-2H-pyrrolo[3,2-b]pyridine-2-one ;
4-{[(2-oxo-1,2-dihydro-3H-pyrrolo[2,3-b]pyridine-3-ylidène)méthyl]amino}benzènesulfonamide ;
3-[(1H-indazole-6-ylamino)méthylidène]-1H-pyrrolo[2,3-b]pyridine-2-one ;
3-[(6-quinolinylamino)méthylidène]-1,3-dihydro-2H-pyrrolo[2,3-b]pyridine-2-one ;
4-{[(2-oxo-5-phényl-1,2-dihydro-3H-pyrrolo[2,3-b]pyridine-3-ylidène)méthyl]amino}benzènesulfonamide ;
3-[(1H-indazole-6-ylamino)méthylidène]-5-phényl-1H-pyrrolo[2,3-b]pyridine-2-one ;
5-phényl-3-[(6-quinolinylainino)méthylidène]-1,3-dihydro-2H-pyrrolo[2,3-b]pyridine-2-one ;
4-{[(5-(2(furyl)-2-oxo-1,2-dihydro-3H-pyrrolo[2,3-b]pyridine-3-ylidène]méthyl}amino)benzènesulfonamide ;
5-(2(furyl)-3-[(1H-indazole-6-ylamino)méthylidène]-1H-pyrrolo[2,3-b]pyridine-2-one ;
5- (2-furyl) -3-[(6-quinolinylamino)méthylidène]-1,3-dihydro-2H-pyrrolo[2, 3-b]pyridine-2-one ;
4-({[2-oxo-5-(3-thienyl)-1,2-dihydro-3H-pyrrolo[2,3-b]pyridine-3-ylidène]méthyl}amino)benzènesulfonamide ;
3-[(1H-indazole-6-ylamino)méthylidène]-5-(3-thiényl)-1,3-dihydro-2H-pyrrolo[2,3-b]pyridine-2-one ;
3-[(6-quinolinylamino)méthylidène]-5-(3-thiényl)-1,3-dihydro-2H-pyrrolo[2,3-b]pyridine-2-one ;
4-{[(5-bromo-2-oxo-1,2-dihydro-3H-pyrrolo[2, 3b]pyridine-3-ylidène)méthyl]amino}benzènesulfonamide ;
5-bromo-3-[(1H-indazole-6-ylamino)méthylidène]-1,3-dihydro-2H-pyrrolo[2,3-b]pyridine-2-one ;
5-bromo-3-[(6-quinolinylamino)méthylidène]-1,3-dihydro-2H-pyrrolo[2,3-b]pyridine-2-one ;
4-{[(6-chloro-2-oxo-1,2-dihydro-3H-pyrrolo[2,3-b]pyridine-3-ylidène)méthyl]amino}benzènesulfonamide ;
6-chloro-3-[(1H-indazole-6-ylamino)méthylidène]-1,3-dihydro-2H-pyrrolo[2,3-b]pyridine-2-one ;
6-chloro-3-[(6-quinolinylamino)méthylidène]-1,3-dihydro-2H-pyrrolo[2,3-b]pyridine-2-one ;
3-{[4-(aminosulfonyl)anilino]méthylidène}-2-oxo-1,2-dihydro-3H-pyrrolo[2,3-b]pyridine-5-carboxylate d'éthyle ;
3-[(1H-indazole-6-ylamino)méthylidène]-2-oxo-1,2-dihydro-3H-pyrrolo[2,3-b]pyridine-5-carboxylate d'éthyle ; et
2-oxo-3-[(6-quinolinylamino)méthylidène]-2,3-dihydro-1H-pyrrolo[2,3-b]pyridine-5-carboxylate d'éthyle ;
et ses sels et produits de solvatation pharmaceutiquement acceptables.

40. Composition pharmaceutique comprenant un composé suivant l'une quelconque des revendications 1 à 39 et un ou plusieurs supports, diluants et/ou excipients pharmaceutiquement acceptables.

41. Composé suivant l'une quelconque des revendications 1 à 39 destiné à être utilisé en thérapeutique.

42. Utilisation d'un composé suivant l'une quelconque des revendications 1 à 39 dans la préparation d'un médicament destiné au traitement d'une maladie ou affection chez un animal, dans laquelle la maladie ou l'affection comprend au moins une affection choisie dans le groupe consistant en : un rejet d'organe transplanté, une croissance tumorale, l'alopécie induite par la chimiothérapie, la thrombocytopénie induite par la chimiothérapie, la leucopénie induite par la chimiothérapie, la mucite, le syndrome plantaire-palmaire, une resténose, l'athérosclérose, l'arthrite rhumatoïde, l'angiogénèse, la cirrhose hépatique, la glomérulonéphrite, la néphropathie diabétique, la néphrosclérose maligne, la microangiopathie thrombotique, la glomérulopathie, le psoriasis, le diabète sucré, l'inflammation, une maladie neurodégénérative, la dégénérescence maculaire, la kératose actinique et des troubles hyperprolifératifs.

43. Utilisation d'un composé suivant l'une quelconque des revendications 1 à 39 dans la préparation d'un médicament destiné au traitement d'une maladie ou affection chez un animal, dans laquelle la maladie ou l'affection comprend un infection virale ou eucaryotique.

44. Utilisation d'un composé suivant l'une quelconque des revendications 1 à 39 dans la préparation d'un médicament destiné au traitement d'une maladie ou affection chez un animal, dans laquelle la maladie ou l'affection comprend l'alopécie.

45. Utilisation d'un composé suivant l'une quelconque des revendications 1 à 39 dans la préparation d'un médicament destiné au traitement d'une maladie ou affection chez un animal, dans laquelle la maladie ou l'affection comprend au moins une affection à médiation par une kinase.

46. Utilisation suivant la revendication 45, dans laquelle la kinase est une protéine-kinase activée par un agent mitogène.

47. Utilisation suivant la revendication 45, dans laquelle la kinase est choisie dans le groupe consistant en : abl, ARaf, ATK, ATM, bcr-abi, Blk, BRaf, Brk, Btk, CDK1, CDK2, CDK3, CDK4, CDK5, CDK6, CDK7, CDK8, CDK9, c-fms, c-kit, c-met, cRaf1, CSF1R, CSK, c-src, EGFR, ErbB2, ErbB3, ErbB4, ERK, ERK1, ERK2, Fak, fes, FGFR1, FGFR2, FGFR3, FGFR4, FGFR5, Fgr, FLK-4, Fps, Frk, Fyn, GSK, gsk3a, gsk3b, Hck, IGF-1R, IKK, IKK1, IKK2, IKK3, INS-R, la kinase liée à l'intégrine, JAK, JAK1, JAK2, JAK3, JNK, Lck, Lyn, MEK, MEK1, MEK2, p38, PDGFR, PIK, PKB1, PKB2, PKB3, PKC, PKCa, PKCb, PKCd, PKCe, PKCg, PKCl, PKCm, PKCz, PLK1, la kinase de type Polo, PYK2, tie₁, tie₂, TrkA, TrkB, TrkC, UL13, UL97, VEGF-R1, VEGF-R2, Yes et Zap70.

48. Utilisation suivant la revendication 45, dans laquelle la kinase est choisie dans le groupe consistant en : VEGF-R2, CDK1, CDK2, CDK3, Zap70, Lck et c-fms.

49. Utilisation suivant la revendication 45, dans laquelle la kinase consiste en VEGF-R2.

50. Utilisation suivant l'une quelconque des revendications 42 à 49, dans laquelle le composé ou son sel physiologiquement acceptable est administré en une quantité d'environ 0,1 à environ 100 mg/kg de poids corporel par jour.

51. Utilisation suivant la revendication 50, dans laquelle le composé ou son sel physiologiquement acceptable est administré en une quantité d'environ 0,1 à environ 10 mg/kg de poids corporel par jour.

52. Utilisation suivant l'une quelconque des revendications 42 à 51, dans laquelle l'animal est un patient humain.

53. Utilisation suivant l'une quelconque des revendications 42 à 52, dans laquelle le composé est coadministré avec un ou plusieurs agents anticancéreux et/ou traitements.
